# EUROPEAN PATENT SPECIFICATION

(11) **EP 2 146 997 B1**
(45) Date of publication and mention of the grant of the patent: **10.11.2010**
(21) Application number: 08742647.4
(22) Date of filing: 07.04.2008
(51) Int. Cl.: C07D 491/048, A61K 31/4355, A61P 3/04, A61P 25/18, A61P 25/22, A61P 25/28

(54) **SUBSTITUTED FURO[2,3-B]PYRIDINE DERIVATIVES AS CANNABINOID-1 RECEPTOR MODULATORS**
SUBSTITUIERTE FURO[2,3-B]PYRIDINDERIVATE ALS CANNABINOID-1-REZEPTORMODULATOREN
DÉRIVÉS FURO[2,3-B]PYRIDINE SUBSTITUÉS UTILES EN TANT QUE MODULATEURS DU RÉCEPTEUR DES CANNABINOÏDES 1

(30) Priority: 11.04.2007 US 922851 P
(43) Date of publication of application: 27.01.2010
(73) Proprietor: Merck Sharp & Dohme Corp., Rahway, NJ 07065 (US)
(72) Inventor: CLEMENTS, Matthew, J., Rahway, New Jersey 07065-0907 (US); DEBENHAM, John, S., Rahway, New Jersey 07065-0907 (US); HALE, Jeffrey, J., Rahway, New Jersey 07065-0907 (US); MADSEN-DUGGAN, Christina, B., Rahway, New Jersey 07065-0907 (US); WALSH, Thomas, F., Rahway, New Jersey 07065-0907 (US); PERESYPKIN, Andrey, V., Rahway, New Jersey 07065-0907 (US); HELMY, Roy, Rahway, New Jersey 07065-0907 (US)
(74) Representative: Buchan, Gavin MacNicol
(86) International application number: PCT/US2008/004533
(87) International publication number: WO 2008/127585

(56) References cited:
- WO-A1-2005/115977
- US-B2- 7 091 216

## Description

### BACKGROUND OF THE INVENTION

Marijuana (*Cannabis sativa L.)* and its derivatives have been used for centuries for medicinal and recreational purposes. A major active ingredient in marijuana and hashish has been determined to be Δ⁹-tetrahydrocannabinol (Δ⁹-THC). Detailed research has revealed that the biological action of Δ⁹-THC and other members of the cannabinoid family occurs through two G-protein coupled receptors termed CB1 and CB2. The CB1 receptor is primarily found in the central and peripheral nervous systems and to a lesser extent in several peripheral organs. The CB2 receptor is found primarily in lymphoid tissues and cells. Three endogenous ligands for the cannabinoid receptors derived from arachidonic acid have been identified (anandamide, 2-arachidonoyl glycerol, and 2-arachidonyl glycerol ether). Each is an agonist with activities similar to Δ⁹-THC, including sedation, hypothermia, intestinal immobility, antinociception, analgesia, catalepsy, anti-emesis, and appetite stimulation.

There are at least three CB1 modulators characterized as inverse agonists/antagonists, ACOMPLIA (rimonabant, *N*-(1-piperidinyl)-5-(4-chlorophenyl)-1-(2,4-dichlorophenyl)-4-methylpyrazole-3-carboxamide, SR141716A), and 3-(4-chlorophenyl-*N'*-(4-chlorophenyl)sulfonyl-*N-*methyl-4-phenyl-4,5-dihydro-1*H*-pyrazole-1-carboxamide (SLV-319), and taranabant, *N*-[(1*S*,2*S*)-3-(4-Chlorophenyl)-2-(3-cyanophenyl)-1-methylpropyl]-2-methyl-2-[[5-(trifluoromethyl)-2-pyridinyl]oxy]propanamide, in clinical development for treatment of eating disorders and/or smoking cessation at this time. There still remains a need for potent low molecular weight CB1 modulators that have pharmacokinetic and pharmacodynamic properties suitable for use as human pharmaceuticals.

Furopyridine compounds are disclosed in US 2006/0046977, EP 0 737 685, JP 2006-45 220, JP 7-76586, WO 2004/014375, WO 2004/096130, WO 2005/009389, WO 2005/061476, WO 2005/067900, WO 2006/013095, and WO 2006/030031. Furopyridine CB1 antagonists/inverse agonists are disclosed in WO 04/012671 and US 7,091,216.

### SUMMARY OF THE INVENTION

The present invention is concerned with novel furo pyridines of structural Formula I: 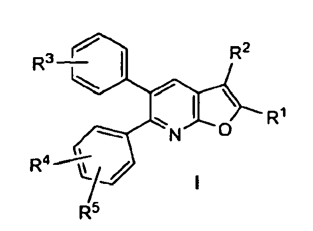 and pharmaceutically acceptable salts thereof which are modulators of and, in particular, antagonists and/or inverse agonists of the Cannabinoid-1 (CB1) receptor and are useful in the treatment, prevention or suppression of diseases mediated by the Cannabinoid-1 (CB1) receptor. In one aspect, the invention is concerned with the use of these novel compounds to selectively antagonize the Cannabinoid-1 (CB1) receptor. As such, compounds of the present invention are useful as centrally acting drugs in the treatment of psychosis, memory deficits, cognitive disorders, Alzheimer's disease, migraine, neuropathy, neuro-inflammatory disorders including multiple sclerosis and Guillain-Barre syndrome and the inflammatory sequelae of viral encephalitis, cerebral vascular accidents, and head trauma, anxiety disorders, stress, epilepsy, Parkinson's disease, movement disorders, and schizophrenia. The compounds are also useful for the treatment of substance abuse disorders, the treatment of obesity or eating disorders, and complications associated therewith, including left ventricular hypertrophy, as well as the treatment of asthma, constipation, chronic intestinal pseudo-obstruction, and cirrhosis of the liver.

The present invention is also concerned with treatment of these conditions, and the use of compounds of the present invention for manufacture of a medicament useful in treating these conditions. The present invention is also concerned with treatment of these conditions through a combination of compounds of formula I and other currently available pharmaceuticals.

The invention is also concerned with pharmaceutical formulations comprising one of the compounds as an active ingredient, as well as processes for preparing the compounds of this invention.

### BRIEF DESCRIPTION OF THE DRAWINGS

FIG. 1 is the X-ray diffraction (XRPD) pattern for the anhydrous free base crystalline 1:1 ethanolate polymorphic Form I of Example 3.
FIG. 2 is the Thermogravimetry analysis (TGA) curve for the anhydrous free base crystalline 1:1 ethanolate polymorphic Form I of Example 3.

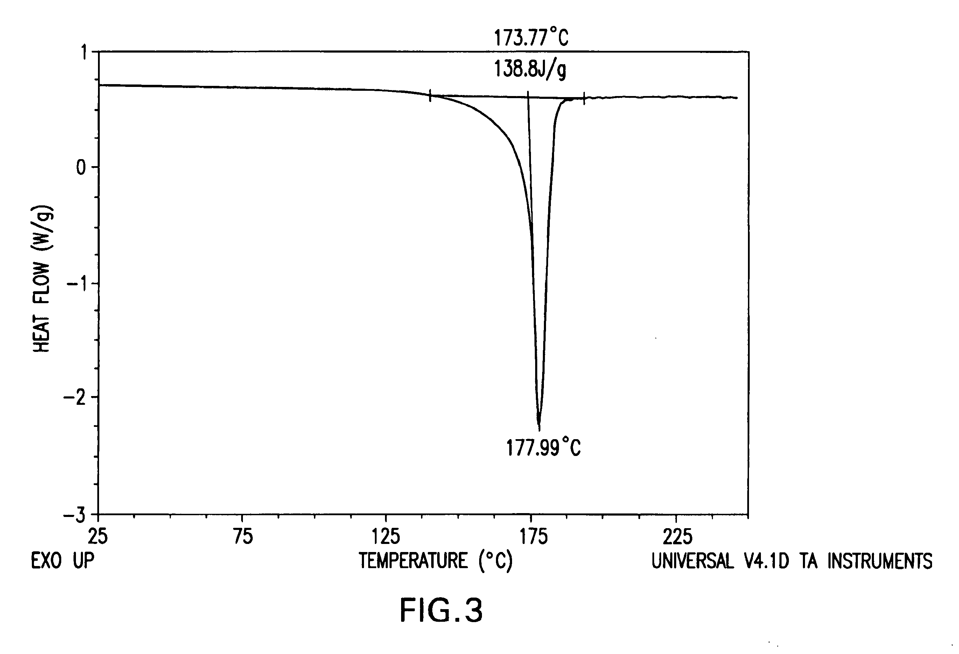

FIG. 3 is the Differential scanning calorimetry (DSC) curve for the anhydrous free base crystalline 1:1 ethanolate polymorphic Form I of Example 3.

### DETAILED DESCRIPTION OF THE INVENTION

The compounds of the present invention are represented by the compound of structural formula I: 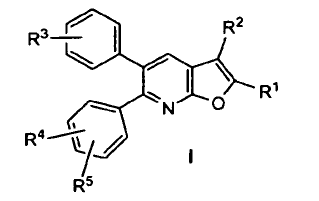 or a pharmaceutically acceptable salt thereof, wherein:
R¹ is selected from:
(1) phenyl,
(2) heteroaryl,
(3) -C(O)R^{a},
(4) -C(O)OR^{a},
(5) -C(O)NR^{b}R^{c}, and
(6) -S(O)₂R^{a},
wherein each phenyl and heteroaryl is unsubstituted or substituted with one to four substituents independently selected from -OH, -C₁₋₆alkyl, and halogen, and wherein R^{b} and R^{c} together with the atoms to which they are attached may form a 4-10 membered aromatic or non-aromatic mono- or bicyclic ring, wherein the 4-10 membered ring is unsubstituted or substituted with one to four substituents independently selected from -OH, -C₁₋₆alkyl, and halogen;
R2 is selected from:
(1) C₁₋₁₀alkyl,
(2) C₃₋₁₀cycloalkyl,
(3) cycloheteroalkyl,
(4) phenyl,
(5) heteroaryl,
(6) -C(O)C₁₋₁₀alkyl,
(7) -C(O)OR^{a},
(8) -C(O)N(R^{b})₂,
(9) -N(R^{b})₂, and
(10) -NR^{d}C(O)C₁₋₁₀alkyl,
wherein each alkyl, cycloalkyl, cycloheteroalkyl, phenyl and heteroaryl is unsubstituted or substituted with one to four substituents independently selected from -OH, C₁₋₆alkyl, and oxo;
R3 is selected from:
(1) hydrogen,
(2) C₁₋₁₀alkyl,
(3) halogen,
(4) -CN,
(5) -CF₃,
(6) -OCF₃,
(7) -C(O)C₁₋₄alkyl,
(8) -C(O)OC₁₋₄alkyl,
(9) -OC₁₋₄alkyl, and
(10) -SC₁₋₄alkyl;
R4 is selected from:
(1) pyrazole,
(2) oxadiazole,
(3) triazole,
(4) isoxazole,
(5) isothiazole, and
(6) thiadiazole, wherein each pyrazole, oxadiazole, triazole, isoxazole, isothiazole and thiadiazole is unsubstituted or substituted with one or three substituents selected from R⁶ and R⁷;
R5 is selected from:
(1) hydrogen,
(2) C₁₋₁₀alkyl,
(3) halogen,
(4) -CN,
(5) -CF₃,
(6) -OCF₃,
(7) -C(O)C₁₋₄alkyl,
(8) -C(O)OC₁₋₄alkyl,
(9) -OC₁₋₄alkyl, and
(10) -SC₁₋₄alkyl;
R6 is selected from:
(1) hydrogen,
(2) C₁₋₁₀alkyl,
(3) halogen,
(4) -CN,
(5) -C(O)C₁₋₆alkyl,
(6) -OC₁₋₆alkyl,
(7) -OCF₃, and
(8) -SC₁₋₆alkyl;
R⁷ is selected from:
(1) hydrogen,
(2) C₁₋₆alkyl, and
(3) C(O)C₁₋₁₀alkyl;
R^{a} is selected from:
(1) C₁₋₆alkyl, and
(2) C₃₋₇cycloalkyl,
wherein each alkyl and cycloalkyl is unsubstituted or substituted with one to four substituents independently selected from -OH, -C₁₋₆alkyl, and halogen;
R^{b} is selected from:
(1) hydrogen,
(2) C₁₋₆alkyl, and
(3) phenyl,
wherein each alkyl and phenyl is unsubstituted or substituted with one to four substituents independently selected from -OH, -C₁₋₆alkyl, and halogen;
R^{c} is selected from:
(1) C₁₋₆alkyl, and
(2) phenyl,
wherein each alkyl and phenyl is unsubstituted or substituted with one to four substituents independently selected from -OH, -C₁₋₆alkyl, and halogen; and
R^{d} is selected from:
(1) hydrogen, and
(2) C₁₋₆alkyl;
wherein each alkyl is unsubstituted or substituted with one to four substituents independently selected from -OH, -C₁₋₆alkyl, and halogen.

In one embodiment of the present invention, R¹ is selected from: phenyl, heteroaryl, - C(O)R^{a}, -C(O)OR^{a}, -C(O)NR^{b}R^{c}, and -S(O)₂R^{a}, wherein each phenyl and heteroaryl is unsubstituted or substituted with one to four substituents independently selected from -OH, -C₁₋₆alkyl, and halogen, and wherein R^{b} and R^{c} together with the atoms to which they are attached may form a 4-10 membered aromatic or non-aromatic mono- or bicyclic ring, wherein the 4-10 membered ring is unsubstituted or substituted with one to four substituents independently selected from -OH, -C₁₋₆alkyl, and halogen, provided that both R^{b} and R^{c} are not phenyl.

In another embodiment of the present invention, R¹ is selected from: phenyl, heteroaryl, - C(O)Ra, -C(O)OR^{a}, -C(O)NR^{b}R^{c}, and -S(O)₂R^{a}, wherein each phenyl and heteroaryl is unsubstituted or substituted with one to four substituents independently selected from -OH, -C₁₋₆alkyl, and halogen.

In another embodiment of the present invention, R¹ is selected from: phenyl, heteroaryl, - C(O)Ra, -C(O)OR^{a}, -C(O)NR^{b}R^{c}, and -S(O)₂R^{a}, wherein each phenyl and heteroaryl is unsubstituted or substituted with one to four substituents independently selected from -OH, -C_{1- 6}alkyl, and halogen, and wherein R^{b} and R^{c} together with the atoms to which they are attached may form a 4-6 membered aromatic or non-aromatic ring, wherein the 4-6 membered ring is unsubstituted or substituted with one to four substituents independently selected from -OH, -C₁₋₆alkyl, and halogen, provided that both R^{b} and R^{c} are not phenyl.

In another embodiment of the present invention, R¹ is selected from: -C(O)R^{a}, - C(O)OR^{a}, -C(O)NR^{b}R^{c}, and -S(O)₂R^{a}, wherein R^{b} and R^{c} together with the atoms to which they are attached may form a 4-6 membered aromatic or non-aromatic ring, wherein the 4-6 membered ring is unsubstituted or substituted with one to four substituents independently selected from -OH, -C₁₋₆alkyl, and halogen, provided that both R^{b} and R^{c} are not phenyl. In a class of this embodiment, R¹ is selected from: -C(O)R^{a}, and -S(O)₂R^{a}. In a subclass of this class, R¹ is selected from: -C(O)C(CH₃)₂OH, -C(O)CH(CH₃)₂, -C(O)C(CH₃)₃, - C(O)CH₂CH₃, -C(O)CH₂CH(CH₃)₂, and -S(O)₂C(CH₃)₃. In another subclass of this suclass, R¹ is selected from: -C(O)C(CH₃)₂OH, -C(O)CH(CH₃)₂, -C(O)C(CH₃)₃, -C(O)CH₂CH₃, - C(O)CH₂CH(CH₃)₂, and -S(O)₂C(CH₃)₃, wherein R¹ is unsubstituted or substituted with one to four substituents independently selected from -OH, -C₁₋₆alkyl, and halogen. In another subclass of this subclass, R¹ is selected from: -C(O)C(CH₃)₂OH, -C(O)CH(CH₃)₂, and - C(O)C(CH₃)₃. In another subclass of this subclass, R¹ is -C(O)C(CH₃)₃, wherein R¹ is unsubstituted or substituted with one to four substituents independently selected from -OH, -C₁₋₆alkyl, and halogen. In another subclass of this subclass, R¹ is -C(O)C(CH₃)₃. In another class of this embodiment, R¹ is -C(O)R^{a}. In a subclass of this class, R¹ is selected from: - C(O)C(CH₃)₂OH -C(O)CH(CH₃)₂, -C(O)C(CH₃)₃, -C(O)CH₂CH₃, and - C(O)CH₂CH(CH₃)₂. In another subclass of this subclass, R¹ is -C(O)C(CH₃)₃.

In another embodiment of the present invention, R¹ is selected from: phenyl, heteroaryl, - C(O)C₁₋₆alkyl, -C(O)C₃₋₇cycloalkyl, -C(O)OC₁₋₆alkyl, -C(O)OC₃₋₇cycloalkyl, -C(O)NR^{b}(C₁₋₆alkyl), -C(O)NR^{d}(phenyl), -S(O)₂C₁₋₆alkyl, and -S(O)₂C₃₋₇cycloalkyl, wherein each alkyl, cycloalkyl, phenyl and heteroaryl is unsubstituted or substituted with one to four substituents independently selected from -OH, -C₁₋₆alkyl, and halogen. In a class of this embodiment, R¹ is selected from: -C(O)C₁₋₆alkyl, -C(O)C₃₋₇cycloalkyl, -C(O)OC₁₋₆alkyl, -C(O)OC₃₋₇cycloalkyl, -C(O)NR^{b}(C₁₋₆alkyl), -C(O)NR^{d}(phenyl), -S(O)₂C₁₋₆alkyl, and -S(O)₂C₃₋₇cycloalkyl, wherein each alkyl, cycloalkyl and phenyl is unsubstituted or substituted with one to four substituents independently selected from -OH, -C₁₋₆alkyl, and halogen. In a subclass of this class, R¹ is selected from: -C(O)C₁₋₆alkyl, and -S(O)₂C₁₋₆alkyl, wherein each alkyl is unsubstituted or substituted with one to four substituents independently selected from -OH, -C₁₋₆alkyl, and halogen. In another subclass of this subclass, R¹ is selected from: - C(O)C(CH₃)₂OH, -C(O)CH(CH₃)₂, -C(O)C(CH₃)₃, -C(O)CH₂CH₃, -C(O)CH₂CH(CH₃)₂, and -S(O)₂C(CH₃)₃, wherein R¹ is unsubstituted or substituted with one to four substituents independently selected from -OH, -C₁₋₆alkyl, and halogen. In another subclass of this subclass, R¹ is selected from: -C(O)C(CH₃)₂OH, -C(O)CH(CH₃)₂, -C(O)C(CH₃)₃, -C(O)CH₂CH₃, - C(O)CH₂CH(CH₃)₂, and -S(O)₂C(CH₃)₃, wherein R¹ is unsubstituted or substituted with one to four substituents independently selected from -OH, -C₁₋₆alkyl, and halogen. In another subclass of this subclass, R¹ is selected from: -C(O)C(CH₃)₂OH, -C(O)CH(CH₃)₂, and - C(O)C(CH₃)₃. In another subclass of this subclass, R¹ is -C(O)C(CH₃)₃, wherein R¹ is unsubstituted or substituted with one to four substituents independently selected from -OH, -C₁₋₆alkyl, and halogen. In another subclass of this subclass, R¹ is -C(O)C(CH₃)₃. In another class of this embodiment, R¹ is selected from: -C(O)C₁₋₆alkyl, and -S(O)₂C₁₋₆alkyl, wherein each alkyl is unsubstituted or substituted with one to four substituents independently selected from - OH, -C₁₋₆alkyl, and halogen. In a subclass of this class, R¹ is selected from: -C(O)C(CH₃)₂OH, -C(O)CH(CH₃)₂, -C(O)C(CH₃)₃, -C(O)CH₂CH₃, -C(O)CH₂CH(CH₃)₂, and -S(O)₂C(CH₃)₃, wherein R¹ is unsubstituted or substituted with one to four substituents independently selected from -OH, -C₁₋₆alkyl, and halogen. In another subclass of this class, R¹ is selected from: - C(O)C(CH₃)₂OH, -C(O)CH(CH₃)₂, -C(O)C(CH₃)₃, -C(O)CH₂CH₃, -C(O)CH₂CH(CH₃)₂, and -S(O)₂C(CH₃)₃. In another subclass of this class, R¹ is selected from: -C(O)C(CH₃)₂OH, - C(O)CH(CH₃)₂, and -C(O)C(CH₃)₃. In another subclass of this class, R¹ is -C(O)C(CH₃)₃, wherein R¹ is unsubstituted or substituted with one to four substituents independently selected from -OH, -C₁₋₆alkyl, and halogen. In another subclass of this class, R¹ is -C(O)C(CH₃)₃.

In one embodiment of the present invention, R² is selected from: C₁₋₁₀alkyl, C₃₋₁₀cycloalkyl, cycloheteroalkyl, phenyl, heteroaryl, -C(O)C₁₋₁₀alkyl, -C(O)OR^{a}, -C(O)N(R^{b})₂, - N(R^{b})₂, and -NR^{d}C(O)C₁₋₁₀alkyl, wherein each alkyl, cycloalkyl, cycloheteroalkyl, phenyl and heteroaryl is unsubstituted or substituted with one to four substituents independently selected from -OH, C₁₋₆alkyl, and oxo. In a class of this embodiment, R² is selected from: C₁₋₁₀alkyl, cycloheteroalkyl, -C(O)C₁₋₁₀alkyl, -C(O)N(R^{b})₂, -N(R^{b})₂, and -NR^{d}C(O)C₁₋₁₀alkyl, wherein each alkyl and cycloheteroalkyl is unsubstituted or substituted with one to four substituents independently selected from -OH, and oxo. In a subclass of this class, R² is selected from: - CH₃, -CH₂CH₃, -CH₂OH, pyrrolidine-2-one, -C(O)CH₃, -C(O)NH₂, -C(O)NH(CH₃), -NH₂, - NHC(O)CH₃, -NHC(O)CH(CH₃)₂, -NHC(O)C(CH₃)₂OH, -NHC(O)CH(CH₃)OH, NHC(O)CH₂OH, NHC(O)CH₂OC(O)CH₃, -NHC(O)CH(CH₃)OC(O)CH₃, and - NHC(O)C(CH₃)₂OC(O)CH₃. In a subclass of this subclass, R² is selected from: -CH₂OH, - C(O)CH₃, -C(O)NH₂, and -NHC(O)CH₃. In another subclass of this subclass, R² is -C(O)NH₂. In another subclass of this class, R² is selected from: -CH₃, -CH₂CH₃, -CH₂OH, pyrrolidine-2-one, -C(O)CH₃, -C(O)NH₂, -C(O)NH(CH₃), -NH₂, -NHC(O)CH₃, -NHC(O)CH(CH₃)₂, - NHC(O)C(CH₃)₂OH, -NHC(O)CH(CH₃)OH, and -NHC(O)CH₂OH. In a subclass of this subclass, R² is selected from: -CH₂OH -C(O)CH₃, -C(O)NH₂, and -NHC(O)CH₃. In another subclass of this subclass, R² is -C(O)NH₂. In another class of this embodiment, R² is selected from: C₁₋₁₀alkyl, -C(O)C₁₋₁₀akyl, -C(O)N(R^{b})₂, and -NR^{d}C(O)C₁₋₁₀alkyl, wherein each alkyl is unsubstituted or substituted with one to four substituents independently selected from - OH. In a subclass of this class, R² is selected from: -CH₃, -CH₂CH₃, -CH₂OH, -C(O)CH₃, - C(O)NH₂, -C(O)NH(CH₃), -NHC(O)CH₃, -NHC(O)CH(CH₃)₂, -NHC(O)C(CH₃)₂OH, - NHC(O)CH(CH₃)OH, NHC(O)CH₂OH, and -NHC(O)CH₂OC(O)CH₃, - NHC(O)CH(CH₃)OC(O)CH₃, -NHC(O)C(CH₃)₂OC(O)CH₃. In another subclass of this class, R2 is selected from: -CH₃, -CH₂CH₃, -CH₂OH, -C(O)CH₃, -C(O)NH₂, -C(O)NH(CH₃), - NHC(O)CH₃, -NHC(O)CH(CH₃)₂, -NHC(O)C(CH₃)₂OH, -NHC(O)CH(CH₃)OH, and NHC(O)CH₂OH. In a subclass of this subclass, R² is selected from: -CH₂OH, -C(O)CH₃, - C(O)NH₂, and -NHC(O)CH₃. In another subclass of this subclass, R² is -C(O)NH₂.

In another embodiment of the present invention, R² is -C(O)N(R^{b})₂. In a class of this embodiment, R² is selected from: -C(O)NH₂, and -C(O)NH(CH₃). In a subclass of this class, R2 is -C(O)NH₂.

In another embodiment of the present invention, R³ is selected from: hydrogen, C₁₋₁₀alkyl, halogen, -CN, -CF₃, -OCF₃, -C(O)C₁₋₄alkyl, -C(O)OC₁₋₄alkyl, -OC₁₋₄alkyl, and - SC₁₋₄alkyl. In a class of this embodiment, R³ is selected from: C₁₋₆alkyl, halogen, -CN, -CF₃, -OCF₃, -C(O)C₁₋₂alkyl, -C(O)OC₁₋₂alkyl, -OC₁₋₂alkyl, and -SC₁₋₂alkyl. In a subclass of this class, R³ is selected from: halogen, -CN, -CF₃, -OCF₃, and -OC₁₋₂alkyl. In another subclass of this class, R³ is halogen or CN. In another subclass of this class, R³ is halogen. In a subclass of this subclass, R³ is selected from: Cl, Br, and F. In another subclass of this subclass, R³ is Cl. In yet another subclass of this subclass, R³ is 4-chloro.

In another embodiment of the present invention, R⁴ is selected from:
pyrazole, oxadiazole, triazole, isoxazole, isothiazole, and thiadiazole, wherein each pyrazole, oxadiazole, triazole, isoxazole, isothiazole and thiadiazole is unsubstituted or substituted with one or two substituents selected from R⁶ and R⁷. In a class of this embodiment, R⁴ is selected from: 1*H*-pyrazol-3-yl, 1*H*-pyrazol-4-yl, 1,2,4-oxadiazol-3-yl, 1*H*-pyrazol-1-yl, 4*H*-1,2,4-triazol-3-yl, 2*H*-1,2,3-triazol-4-yl, isoxazol-5-yl, isoxazol-3-yl, isothiazole, 1,2,4-thiadiazole, and 1,2,4-oxadiazole. In another class of this embodiment, R⁴ is selected from: 4-pyrazole, 4-oxadiazole, 4-triazole, 4-isoxazole, 4-isothiazole, and 4-thiadiazole, wherein each pyrazole, oxadiazole, triazole, isoxazole, isothiazole and thiadiazole is unsubstituted or substituted with one or two substituents selected from R⁶ and R⁷. In a class of this embodiment, R⁴ is selected from: para-1*H*-pyrazol-3-yl, para-1*H*-pyrazol-4-yl, para-1,2,4-oxadiazol-3-yl, para-1*H*-pyrazol-1-yl, para-4*H*-1,2,4-triazol-3-yl, para-2*H*-1,2,3-triazol-4-yl, para-isoxazol-5-yl, para-isoxazol-3-yl, para-isothiazole, para-1,2,4-thiadiazole, and para-1,2,4-oxadiazole.

In another embodiment of the present invention, R⁴ is selected from: pyrazole, and oxadiazole, wherein each pyrazole and oxadiazole is unsubstituted or substituted with one or two substituents selected from R⁶ and R⁷. In a class of this embodiment, R⁴ is selected from: 1*H-*pyrazol-3-yl, 1*H*-pyrazol-4-yl, 1,2,4-oxadiazol-3-yl, and 1,2,4-oxadiazole. In another class of this embodiment, R⁴ is selected from: 1*H*-pyrazol-3-yl, 1*H*-pyrazol-4-yl and 1,2,4-oxadiazol-3-yl. In another class of this embodiment of the present invention, R⁴ is selected from: 4-pyrazole and 4-oxadiazole, wherein each pyrazole and oxadiazole is unsubstituted or substituted with one or two substituents selected from R⁶ and R⁷. In a subclass of this class, R⁴ is selected from: para-1*H*-pyrazol-3-yl, para-1*H*-pyrazol-4-yl, para-1,2,4-oxadiazol-3-yl, and para-1,2,4-oxadiazole. In another class of this embodiment, R⁴ is selected from: para-1*H*-pyrazol-3-yl, para-1*H*-pyrazol-4-yl and para-1,2,4-oxadiazol-3-yl.

In another embodiment of the present invention, R⁴ is pyrazole, wherein each pyrazole is unsubstituted or substituted with one or two substituents selected from R⁶ and R⁷. In a class of this embodiment, R⁴ is selected from: 1*H*-pyrazol-3-yl and 1*H*-pyrazol-4-yl. In another class of this embodiment, R⁴ is 1*H*-pyrazol-3-yl. In another class of this embodiment, R⁴ is 1*H*-pyrazol-4-yl. In another class of this embodiment of the present invention, R⁴ is 4-pyrazole, wherein each pyrazole is unsubstituted or substituted with one or two substituents selected from R⁶ and R⁷. In a subclass of this class, R⁴ is selected from: para-1*H*-pyrazol-3-yl and para-1*H*-pyrazol-4-yl. In another subclass of this class, R⁴ is para-1*H*-pyrazol-3-yl. In another subclass of this class, R⁴ is para-1*H*-pyrazol-4-yl.

In another embodiment of the present invention, R⁴ is oxadiazole, wherein each oxadiazole is unsubstituted or substituted with one or two substituents selected from R⁶ and R⁷. In a class of this embodiment, R⁴ is 1,2,4-oxadiazol-3-yl and 1,2,4-oxadiazole. In another class of this embodiment, R⁴ is 1,2,4-oxadiazol-3-yl. In a class of this embodiment, R⁴ is 4-oxadiazole, wherein each oxadiazole is unsubstituted or substituted with one or two substituents selected from R⁶ and R⁷. In a subclass of this class, R⁴ is para-1,2,4-oxadiazol-3-yl and para-1,2,4-oxadiazole. In another subclass of this class, R⁴ is para-1,2,4-oxadiazol-3-yl.

In another embodiment of the present invention, R⁴ is selected from:

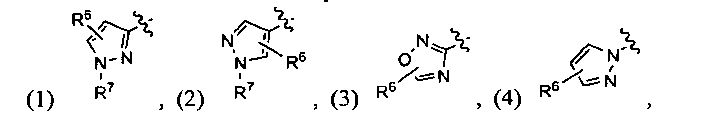

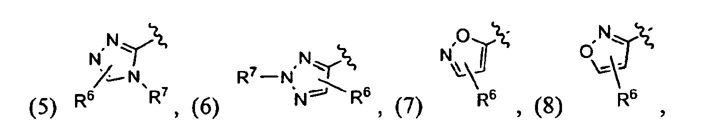

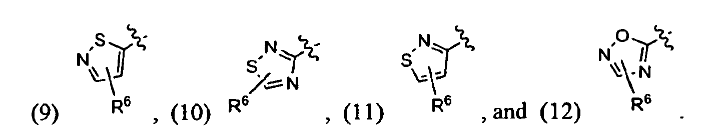

In a subclass of this class, R⁷ is hydrogen.
In a class of this embodiment, R⁴ is selected from:

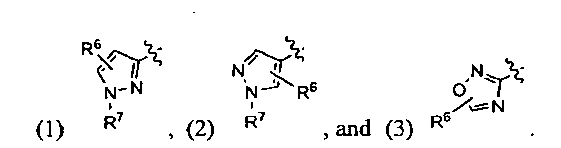

In a subclass of this class, R⁷ is hydrogen.
In another embodiment of the present invention, R⁴ is selected from:

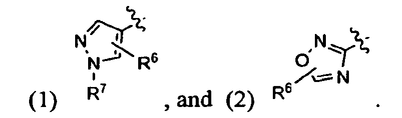

In a class of this embodiment, R⁷ is hydrogen.

In another class of this embodiment of the present invention, R⁴ is selected from:

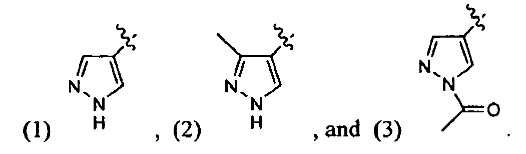

In a subclass of this class, R⁴ is:

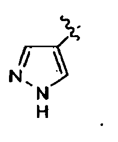

In another subclass of this class, R⁴ is:

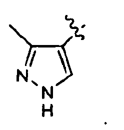

In another subclass of this class, R⁴ is:

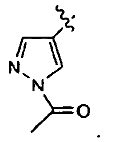

In another class of this embodiment, R⁴ is:

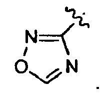

In another embodiment of the present invention, R⁴ is selected from:

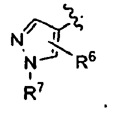

In a class of this embodiment of the present invention, R⁴ is selected from:

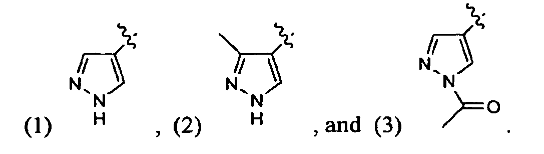

In a subclass of this class, R⁴ is:

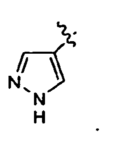

In another subclass of this class, R⁴ is:

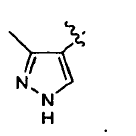

In another subclass of this class, R⁴ is:

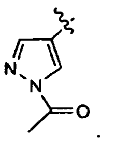

In another embodiment of the present invention, R⁴ is selected from:

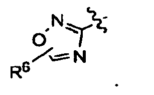

In a class of this embodiment, R⁴ is:

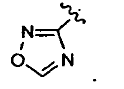

In another embodiment of the present invention, R⁵ is selected from: hydrogen, C₁₋₁₀alkyl, halogen, -CN, -CF₃, -OCF₃, -C(O)C₁₋₄alkyl, -C(O)OC₁₋₄alkyl, -OC₁₋₄alkyl, and - SC₁₋₄alkyl. In a class of this embodiment, R⁵ is selected from: C₁₋₆alkyl, halogen, -CN, -CF₃, -OCF3, -C(O)C₁₋₂alkyl, -C(O)OC₁₋₂alkyl, -OC₁₋₂alkyl, and -SC₁₋₂alkyl. In a subclass of this class, R⁵ is selected from: halogen, -CN, -CF₃, -OCF₃, and -OC₁₋₂alkyl. In another subclass of this class, R⁵ is halogen or CN. In another subclass of this class, R⁵ is halogen. In a subclass of this subclass, R⁵ is selected from: Cl, Br, and F. In another subclass of this subclass, R⁵ is Cl. In yet another subclass of this subclass, R⁵ is 2-chloro.

In another embodiment of the present invention, R⁶ is selected from: hydrogen, C₁₋₄alkyl, halogen, -CN, -C(O)C₁₋₂alkyl, -OC₁₋₆alkyl, -OCF₃, and -SC₁₋₆alkyl. In a class of this embodiment, R⁶ is selected from: hydrogen, -CH₃, halogen, -CN, -C(O)CH₃, -OCH₃, - OCH₂CH₃, -OCH(CH₃)₂, -OCF₃, and -SCH₃.

In another embodiment of the present invention, R⁶ is selected from: hydrogen, and C₁₋₁₀alkyl. In a class of this embodiment, R⁶ is selected from: hydrogen and C₁₋₄alkyl. In another class of this embodiment, R⁶ is selected from: hydrogen, and -CH₃. In another class of this embodiment, R⁶ is hydrogen. In another class of this embodiment, R⁶ is -CH₃.

In another embodiment of the present invention, R⁷ is selected from: hydrogen, -C₁₋₆alkyl, and -C(O)C₁₋₁₀alkyl. In a class of this embodiment, R⁷ is selected from: hydrogen, -C₁₋₄alkyl, and -C(O)C₁₋₂alkyl In another class of this embodiment, R⁷ is selected from: hydrogen and -C(O)C₁₋₁₀alkyl. In a subclass of this class, R⁷ is selected from: hydrogen and -C(O)C_{1- 2}alkyl. In another subclass of this class, R⁷ is selected from: hydrogen and -C(O)CH₃. In another class of this embodiment, R⁷ is hydrogen. In another class of this embodiment, R⁷ is C(O)C₁₋₁₀alkyl. In a subclass of this class, R⁷ is -C(O)C₁₋₂alkyl. In another subclass of this class, R⁷ is -C(O)CH₃.

In another embodiment of the present invention, the invention relates to a compound of formula I 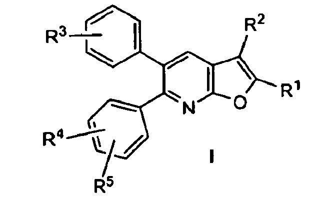 or a pharmaceutically acceptable salt thereof, wherein:
R¹ is selected from: -C(O)R^{a}, and -S(O)₂R^{a};
R² is selected from: C₁₋₁₀alkyl, cycloheteroalkyl, -C(O)C₁₋₁₀alkyl, -C(O)N(R^{b})₂, -N(R^{b})₂, and -NR^{d}C(O)C₁₋₁₀alkyl, wherein each alkyl and cycloheteroalkyl is unsubstituted or substituted with one to four substituents independently selected from -OH and oxo;
R3 is halogen;
R⁴ is selected from: pyrazole, and oxadiazole, wherein each pyrazole and oxadiazole is unsubstituted or substituted with one or two substituents selected from R⁶ and R⁷;
R⁵ is halogen;
R⁶ is selected from: hydrogen, C₁₋₁₀alkyl, halogen, -CN, -C(O)C₁₋₆alkyl, -OC₁₋₆alkyl, -OCF₃, and -SC₁₋₆alkyl ;
R⁷ is selected from: hydrogen, C₁₋₆alkyl, and C(O)C₁₋₁₀alkyl;
R^{a} is selected from: C₁₋₆alkyl, and C₃₋₇cycloalkyl, wherein each alkyl and cycloalkyl is unsubstituted or substituted with one to four substituents independently selected from -OH, -C₁₋₆alkyl, and halogen;
R^{b} is selected from: hydrogen, C₁₋₆alkyl, and phenyl, wherein each alkyl and phenyl is unsubstituted or substituted with one to four substituents independently selected from -OH, -C₁₋₆alkyl, and halogen;
R^{c} is selected from: C₁₋₆alkyl, and phenyl, wherein each alkyl and phenyl is unsubstituted or substituted with one to four substituents independently selected from -OH, -C₁₋₆alkyl, and halogen; and
R^{d} is selected from: hydrogen, and C₁₋₆alkyl, wherein each alkyl is unsubstituted or substituted with one to four substituents independently selected from -OH, -C₁₋₆alkyl, and halogen.

In a class of this embodiment, R¹ is selected from: -C(O)C₁₋₆alkyl, and -S(O)₂C₁₋₆alkyl, wherein each alkyl is unsubstituted or substituted with one to four substituents independently selected from -OH, -C₁₋₆alkyl, and halogen.

In another embodiment of the present invention, the invention relates to the compound of structural formula I, or a pharmaceutically acceptable salt thereof, wherein:
R¹ is selected from: -C(O)C(CH₃)₂OH, -C(O)CH(CH₃)₂, and -C(O)C(CH₃)₃;
R² is selected from: C₁₋₁₀alkyl, -C(O)C₁₋₁₀alkyl, -C(O)N(R^{b})₂, and -NR^{d}C(O)C₁₋₁₀alkyl, wherein each alkyl is unsubstituted or substituted with one to four substituents independently selected from -OH;
R3 is 4-chloro;
R5 is 2-chloro;
R6 is hydrogen or methyl; and
R⁷ is hydrogen.

In another embodiment of the present invention, the invention relates to the compound of structural formula IE: 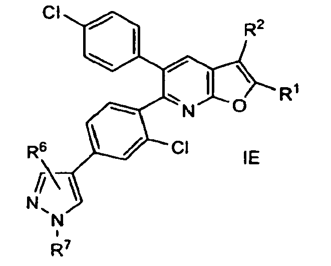 or a pharmaceutically acceptable salt thereof, wherein:
R¹ is selected from: -C(O)C(CH₃)₂OH, -C(O)CH(CH₃)₂, and -C(O)C(CH₃)₃;
R² is selected from: C₁₋₁₀alkyl, -C(O)C₁₋₁₀alkyl, -C(O)N(R^{b})₂, and -NR^{d}C(O)C₁₋₁₀alkyl, wherein each alkyl is unsubstituted or substituted with one to four substituents independently selected from -OH;
R6 is hydrogen or methyl; and
R⁷ is hydrogen.

Another embodiment of the present invention comprises a compound of structural formula IA:

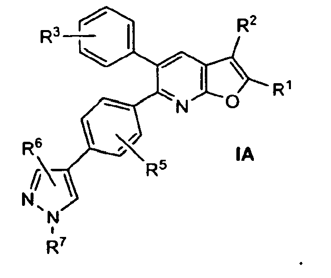

Another embodiment of the present invention comprises a compound of structural formula IB:

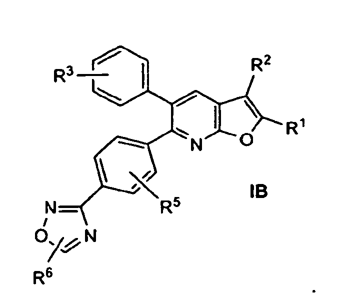

Another embodiment of the present invention comprises a compound of structural formula IC:

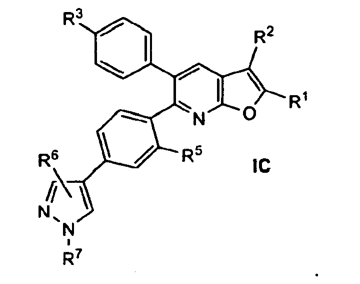

Another embodiment of the present invention comprises a compound of structural formula ID:

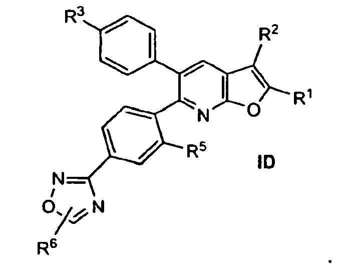

Another embodiment of the present invention comprises a compound of structural formula IE:

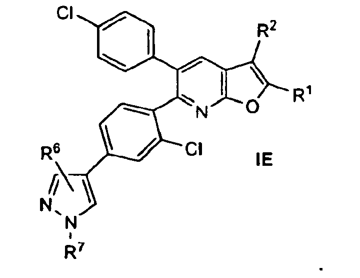

Another embodiment of the present invention comprises a compound of structural formula IF:

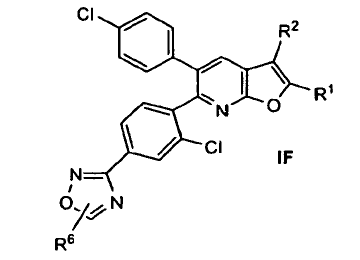

"Alkyl", as well as other groups having the prefix "alk", such as alkoxy, alkanoyl, means carbon chains of up to 10 carbons which may be linear or branched or combinations thereof. Examples of alkyl groups include methyl, ethyl, n-propyl, isopropyl, butyl, isobutyl, sec- and tert-butyl, pentyl, hexyl, heptyl, octyl, nonyl, and the like.

"Alkenyl" means carbon chains which contain at least one carbon-carbon double bond, and which may be linear or branched or combinations thereof. Examples of alkenyl include vinyl, allyl, isopropenyl, pentenyl, hexenyl, heptenyl, 1-propenyl, 2-butenyl, 2-methyl-2-butenyl, and the like.

"Alkynyl" means carbon chains which contain at least one carbon-carbon triple bond, and which may be linear or branched or combinations thereof. Examples of alkynyl include ethynyl, propargyl, 3-methyl-1-pentynyl, 2-heptynyl and the like.

"Cycloalkyl" means mono- or bicyclic or bridged saturated carbocyclic rings, each having from 3 to 10 carbon atoms. Examples of cycloalkyl include cyclopropyl, cyclobutyl, cyclopentyl, cyclohexyl, cycloheptyl, cyclooxtyl, tetrahydronaphthyl, decahydronaphthyl, bicycloand the like. In one embodiment of the present invention, cycloalkyl is selected from cyclopropyl, cyclobutyl, cyclopentyl, cyclohexyl, and 1,2,3,4-tetrahydronaphthyl.

"Cycloalkenyl" means nonaromatic, mono- or bicyclic or bridged carbocyclic rings, each having from 3 to 10 carbon atoms and at least one degree of unsaturation. Examples of cycloalkyl include cyclopropenyl, cyclobutenyl, cyclopentenyl, cyclohexenyl, cycloheptenyl, cyclooxtenyl, decahydronaphthyl, bicyclo[2.2.1]hept-5-en-2-yl, and the like. In one embodiment of the present invention, cycloalkenyl is selected from cyclopropenyl, cyclobutenyl, cyclopentenyl, cyclohexenyl, and bicyclo[2.2.1]hept-5-en-2-yl, and the like.

"Aryl" means mono- or bicyclic aromatic rings containing only carbon atoms. Examples of aryl include phenyl, naphthyl, and the like.

"Heteroaryl" means an aromatic or partially aromatic heterocycle that contains at least one ring heteratom selected from O, S, and N. Heteroaryls thus inclue heteroaryls fused to other kinds of rings, such as aryls, cycloalkyls, and cycloheteroalkyls that are not aromatic. Examples of heteroaryl groups include: pyrrolyl, isoxazolyl, isothiazaolyl, pyrazolyl, pyridyl, oxazolyl, oxadiazolyl, thiadiazolyl, thiazolyl, imidazolyl, triazolyl, tetrazolyl, furyl, triazinyl, thienyl, pyrimidyl, pyridazinyl, pyrazinyl, benzoxazolyl, benzothiazolyl, benzimidazolyl, benzofuranyl, dibenzylfuranyl, isobenzylfuranyl, benzopyrazolyl, benzothienyl, benzothiazolyl, furo(2,3-*b*)pyridyl, quinolyl, indolyl, isoquinolyl, oxazolidinyl, imidazothiathiazolyl, pyrazolylpyridyl, benzotriazolyl, methylenedioxyphenyl, hexahydrothieno-pyridinyl, thienopyridinyl, and the like. In one embodiment of the present invention, heteroaryl is selected from pyridyl, furyl, thienyl, pyrazolyl, oxazolyl, isoxazolyl, oxadiazolyl, indazolyl, oxadiazolyl, tetrazolyl, imidazolyl, indolyl, benzimidazolyl, triazolyl, and benzopyrazolyl.

"Cycloheteroalkyl" refers to a saturated or unsaturated non-aromatic ring or ring system containing at least one heteroatom selected from O, S and N, further including the oxidized forms of sulfur, namely SO and SO₂, in which the point of attachment may be carbon or nitrogen. Examples of heterocycloalkyl include tetrahydrofuranyl, azetidinyl, perhydroazepinyl, dihydrofuranyl, dioxanyl, oxanyl, morpholinyl, 1,4-dithianyl, piperazinyl, piperidinyl, 1,3-dioxolanyl, imidazolidinyl, imidazolinyl, pyrrolinyl, pyrrolidinyl, pyranyl, tetrahydropyranyl, dihydropyranyl, oxathiolanyl, dithiolanyl, 1,3-dithianyl, oxathianyl, thiomorpholinyl, dioxidoisothiazolidinyl, azacycloheptyl, diazobicyclo[3.2.1]-octane, and hexahydroindazolyl. The cycloheteroalkyl ring may be substituted on the ring carbons and/or the ring nitrogens. In one embodiment of the present invention, cycloheteroalkyl is selected from tetrahydrofuranyl, imidazolidinyl, piperidinyl, pyrrolidinyl, isothiazolidinyl morpholinyl and thiomorpholinyl.

"Halogen" includes fluorine, chlorine, bromine and iodine.

When any variable (e.g., R¹, R^{d}, etc.) occurs more than one time in any constituent or in formula I, its definition on each occurrence is independent of its definition at every other occurrence. Also, combinations of substituents and/or variables are permissible only if such combinations result in stable compounds. A squiggly line across a bond in a substituent variable represents the point of attachment.

Under standard nomenclature used throughout this disclosure, the terminal portion of the designated side chain is described first, followed by the adjacent functionality toward the point of attachment. For example, a C₁₋₅ alkylcarbonylamino C₁₋₆ alkyl substituent is equivalent to:

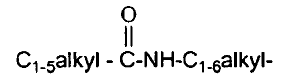

In choosing compounds of the present invention, one of ordinary skill in the art will recognize that the various substituents, i.e. R¹, R², etc., are to be chosen in conformity with well-known principles of chemical structure connectivity and stability.

The term "substituted" shall be deemed to include multiple degrees of substitution by a named substitutent. Where multiple substituent moieties are disclosed or claimed, the substituted compound can be independently substituted by one or more of the disclosed or claimed substituent moieties, singly or plurally. By independently substituted, it is meant that the (two or more) substituents can be the same or different.

Compounds of Formula I may contain one or more asymmetric centers and can thus occur as racemates and racemic mixtures, single enantiomers, diastereomeric mixtures and individual diastereomers. The present invention is meant to comprehend all such isomeric forms of the compounds of Formula I.

Some of the compounds described herein contain olefinic double bonds, and unless specified otherwise, are meant to include both E and Z geometric isomers.

Tautomers are defined as compounds that undergo rapid proton shifts from one atom of the compound to another atom of the compound. Some of the compounds described herein may exist as tautomers with different points of attachment of hydrogen. Such an example may be a ketone and its enol form known as keto-enol tautomers. The individual tautomers as well as mixture thereof are encompassed with compounds of Formula I.

Compounds of the Formula I may be separated into diastereoisomeric pairs of enantiomers by, for example, fractional crystallization from a suitable solvent, for example MeOH or ethyl acetate or a mixture thereof. The pair of enantiomers thus obtained may be separated into individual stereoisomers by conventional means, for example by the use of an optically active amine as a resolving agent or on a chiral HPLC column.

Alternatively, any enantiomer of a compound of the general Formula I may be obtained by stereospecific synthesis using optically pure starting materials or reagents of known configuration.

Furthermore, some of the crystalline forms for compounds of the present invention may exist as polymorphs and as such are intended to be included in the present invention. Polymorphs are compounds having the same chemical composition but different crystal structures. Polymorphism is the ability of the same chemical substance to exist as different crystalline structures. The anhydrous free base crystalline 1:1 ethanolate of 6-[2-chloro-4-(3-methyl-1*H*-pyrazol-4-yl)phenyl]-5-(4-chlorophenyl)-2-(2,2-dimethylpropanoyl)furo[2,3-*b*]pyridine-3-carboxamide has been found to exist in polymorphic Form I. The present invention further relates to the compound which is the anhydrous free base crystalline 1:1 ethanol solvate polymorphic form I of 6-[2-chloro-4-(3-methyl-1*H*-pyrazol-4-yl)phenyl]-5-(4-chlorophenyl)-2-(2,2-dimethylpropanoyl)furo[2,3-*b*]pyridine-3-carboxamide.

In addition, some of the compounds of the instant invention may form solvates with water or common organic solvents. The solvates with water, or hydrates, include, but are not limited to, all full, multiple, partial and fractional hydrates of the compounds of the present invention, including, but not limited to, the mono hydrate, hemi-hydrate and bis hydrate, and pharmaceutically acceptable salts thereof. The term "common organic solvents" refers to solvents including, but not limited, to ethanol, methanol, isopropyl acetate and *N,N*-dimethyl formamide. The compounds of the instant invention may form solvates, including, but not limited to, the ethanol solvate or ethanolate, the methanol solvate, the methanol/water solvate, the isopropyl acetate solvate and the *N,N*-dimethyl formamide solvate, and pharmaceutically acceptable salts thereof. Preferred are pharmaceutically acceptable solvates, such as the ethanol solvate or ethanolate. The solvates include, but are not limited to, all full, multiple, partial and fractional molar ratios of solvate to compound, including a 4:1; a 3:1; a 2:1; 0.5:1; a 0.75:1, a 1:0.5; a 1:0.75, a 4:1, a 3:1, a 2:1 and a 1:1 molar ratio of solvate to compound. For example, an ethanol solvate includes, but is not limited to, a 0.5:1 molar ratio of ethanol to compound; 1:1 molar ratio of ethanol to compound; a 2:1 molar ratio of ethanol to compound; a 1:2 molar ratio of ethanol to compound, a 3:1 molar ratio of ethanol to compound; a 1:3 molar ratio of ethanol to compound, a 4:1 molar ratio of ethanol to compound; and a 1:4 molar ratio of ethanol to compound. The term "solvate" is meant to include compound forms containing solvent molecules within the crystal structure of the compounds of the present invention, or solvent molecules bound to or associated with the compounds of the present invention. Such solvates are encompassed within the scope of this invention.

It is generally preferable to administer compounds of the present invention as enantiomerically pure formulations. Racemic mixtures can be separated into their individual enantiomers by any of a number of conventional methods. These include chiral chromatography, derivatization with a chiral auxiliary followed by separation by chromatography or crystallization, and fractional crystallization of diastereomeric salts.

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. The term "pharmaceutically acceptable salt" further includes all acceptable salts such as acetate, lactobionate, benzenesulfonate, laurate, benzoate, malate, L-malate, bicarbonate, maleate, bisulfate, mandelate, bitartrate, mesylate, besylate, borate, methylbromide, bromide, methylnitrate, calcium edetate, methylsulfate, camsylate, mucate, carbonate, napsylate, 2-napsylate, chloride, nitrate, clavulanate, N-methylglucamine, citrate, ammonium salt, dihydrochloride, oleate, edetate, oxalate, edisylate, pamoate (embonate), estolate, palmitate, esylate, pantothenate, fumarate, phosphate/diphosphate, phosphate, gluceptate, polygalacturonate, gluconate, salicylate, glutamate, stearate, glycollylarsanilate, sulfate, hexylresorcinate, subacetate, hydrabamine, succinate, hydrobromide, tannate, hydrochloride, tartrate, hydroxynaphthoate, teoclate, iodide, tosylate, p-tosylate, isothionate, triethiodide, lactate, panoate, valerate, and the like which can be used as a dosage form for modifying the solubility or hydrolysis characteristics or can be used in sustained release or pro-drug formulations.

It will be understood that, as used herein, references to the compounds of Formula I are meant to also include the pharmaceutically acceptable salts.

Compounds of the present invention are modulators of the CB1 receptor. In particular, the compounds of structural formula I are antagonists or inverse agonists of the CB1 receptor.

An "agonist" is a compound (hormone, neurotransmitter or synthetic compound) which binds to a receptor and mimics the effects of the endogenous regulatory compound, such as contraction, relaxation, secretion, change in enzyme activity, etc. An "antagonist" is a compound, devoid of intrinsic regulatory activity, which produces effects by interfering with the binding of the endogenous agonist or inhibiting the action of an agonist. An "inverse agonist" is a compound which acts on a receptor but produces the opposite effect produced by the agonist of the particular receptor.

Compounds of this invention are modulators of the CB1 receptor and as such are useful as centrally acting drugs in the treatment of psychosis, memory deficits, cognitive disorders, Alzheimer's disease, migraine, neuropathy, neuro-inflammatory disorders including multiple sclerosis and Guillain-Barre syndrome and the inflammatory sequelae of viral encephalitis, cerebral vascular accidents, and head trauma, anxiety disorders, stress, epilepsy, Parkinson's disease, Huntington's disease, movement disorders, and schizophrenia. In particular, the compounds of this invention are antagonists/inverse agonists of the CB1 receptor. The compounds are also useful for the treatment of substance abuse disorders, particularly to opiates, alcohol, marijuana, and nicotine. In particular, the compounds of the invention are useful for smoking cessation. The compounds are also useful for the treatment of obesity or eating disorders associated with excessive food intake and complications associated therewith, including left ventricular hypertrophy, as well as treating or preventing obesity in other mammalian species, including canines and felines. The compounds are also useful for the treatment of constipation and chronic intestinal pseudo-obstruction. The compounds are also useful for the treatment of cirrhosis of the liver, non-alcoholic fatty liver disease (NAFLD), non-alcoholic steatohepatitis (NASH), promotion of wakefulness and treatment of asthma.

The terms "administration of" and or "administering a" compound should be understood to mean providing a compound of the invention or a prodrug of a compound of the invention to the individual in need of treatment.

The administration of the compound of structural formula I in order to practice the present methods of therapy is carried out by administering an effective amount of the compound of structural formula I to the mammalian patient in need of such treatment or prophylaxis. The need for a prophylactic administration according to the methods of the present invention is determined via the use of well known risk factors. The effective amount of an individual compound is determined, in the final analysis, by the physician or veterinarian in charge of the case, but depends on factors such as the exact disease to be treated, the severity of the disease and other diseases or conditions from which the patient suffers, the chosen route of administration other drugs and treatments which the patient may concomitantly require, and other factors in the physician's judgment.

The usefulness of the present compounds in these diseases or disorders may be demonstrated in animal disease models that have been reported in the literature. The following are examples of such animal disease models: a) suppression of food intake and resultant weight loss in rats (Life Sciences 1998, 63, 113-117); b) reduction of sweet food intake in marmosets (Behavioural Pharm. 1998, 9, 179-181); c) reduction of sucrose and ethanol intake in mice (Psychopharm. 1997, 132, 104-106); d) increased motor activity and place conditioning in rats (Psychopharm. 1998, 135, 324-332; Psychopharmacol 2000, 151: 25-30); e) spontaneous locomotor activity in mice (J. Pharm. Exp. Ther. 1996, 277, 586-594); f) reduction in opiate self-administration in mice (Sci. 1999, 283, 401-404); g) bronchial hyperresponsiveness in sheep and guinea pigs as models for the various phases of asthma (for example, see W. M. Abraham et al., "α4-Integrins mediate antigen-induced late bronchial responses and prolonged airway hyperresponsiveness in sheep." J. Clin. Invest. 93, 776 (1993) and A. A. Y. Milne and P. P. Piper, "Role of VLA-4 integrin in leucocyte recruitment and bronchial hyperresponsiveness in the guinea-pig." Eur. J. Pharmacol., 282, 243 (1995)); h) mediation of the vasodilated state in advanced liver cirrhosis induced by carbon tetrachloride (Nature Medicine, 2001, 7 (7), 827-832); i) amitriptyline-induced constipation in cynomolgus monkeys is beneficial for the evaluation of laxatives (Biol. Pharm. Bulletin (Japan), 2000, 23(5), 657-9); j) neuropathology of paediatric chronic intestinal pseudo-obstruction and animal models related to the neuropathology of paediatric chronic intestinal pseudo-obstruction (Journal of Pathology (England), 2001, 194 (3), 277-88).

The magnitude of prophylactic or therapeutic dose of a compound of Formula I will, of course, vary with the nature of the severity of the condition to be treated and with the particular compound of Formula I and its route of administration. It will also vary according to the age, weight and response of the individual patient. In general, the daily dose range lie within the range of from about 0.001 mg to about 100 mg per kg body weight of a mammal, preferably 0.01 mg to about 50 mg per kg, and most preferably 0.1 to 10 mg per kg, in single or divided doses. On the other hand, it may be necessary to use dosages outside these limits in some cases.

For use where a composition for intravenous administration is employed, a suitable dosage range is from about 0.001 mg to about 100 mg in one embodiment from about 0.01 mg to about 50 mg, and in another embodiment from 0.1 mg to 10 mg of a compound of Formula I per kg of body weight per day.

In the case where an oral composition is employed, a suitable dosage range is, e.g. from about 0.01 mg to about 1000 mg of a compound of Formula I per day. In one embodiment, the range is from about 0.1 mg to about 10 mg per day. For oral administration, the compositions are preferably provided in the form of tablets containing from 0.01 to 1,000 mg, preferably 0.01, 0.05, 0.1, 0.5, 1, 2, 2.5, 3, 4, 5, 6, 7, 8, 9, 10, 12, 12.5, 15, 20, 25, 30, 40, 50, 100, 250, 500, 750 or 1000 milligrams of the active ingredient for the symptomatic adjustment of the dosage to the patient to be treated.

Another aspect of the present invention provides pharmaceutical compositions which comprises a compound of Formula I and a pharmaceutically acceptable carrier. The term "composition", as in pharmaceutical composition, is intended to encompass a product comprising the active ingredient(s), and the inert ingredient(s) (pharmaceutically acceptable excipients) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of Formula I, additional active ingredient(s), and pharmaceutically acceptable excipients.

Any suitable route of administration may be employed for providing a mammal, particularly a human or companion animal such as a dog or cat, with an effective dosage of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like.

The pharmaceutical compositions of the present invention comprise a compound of Formula I, including a polymorph, solvate or salt thereof, as an active ingredient or a pharmaceutically acceptable salt thereof, and may also contain a pharmaceutically acceptable carrier and optionally other therapeutic ingredients. By "pharmaceutically acceptable" it is meant the carrier, diluent or excipient must be compatible with the other ingredients of the formulation and not deleterious to the recipient thereof. The compositions include compositions suitable for oral, rectal, topical, parenteral (including subcutaneous, intramuscular, and intravenous), ocular (ophthalmic), pulmonary (aerosol inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

For administration by inhalation, the compounds of the present invention are conveniently delivered in the form of an aerosol spray presentation from pressurized packs or nebulizers, or as powders which may be formulated and the powder composition may be inhaled with the aid of an insufflation powder inhaler device. The preferred delivery systems for inhalation are metered dose inhalation (MDI) aerosol, which may be formulated as a suspension or solution of a compound of Formula I in suitable propellants, such as fluorocarbons or hydrocarbons and dry powder inhalation (DPI) aerosol, which may be formulated as a dry powder of a compound of Formula I with or without additional excipients.

Suitable topical formulations of a compound of formula I include transdermal devices, aerosols, creams, solutions, ointments, gels, lotions, dusting powders, and the like. The topical pharmaceutical compositions containing the compounds of the present invention ordinarily include about 0.005% to 5% by weight of the active compound in admixture with a pharmaceutically acceptable vehicle. Transdermal skin patches useful for administering the compounds of the present invention include those well known to those of ordinary skill in that art.

In practical use, the compounds of Formula I, including polymorphs, solvates and salts of the compounds of Formula I, can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets, with the solid oral preparations being preferred over the liquid preparations. Because of their ease of administration, tablets and capsules represent the most advantageous oral dosage unit form in which case solid pharmaceutical carriers are obviously employed. If desired, tablets may be coated by standard aqueous or nonaqueous techniques.

In addition to the common dosage forms set out above, the compounds of Formula I may also be administered by controlled release means and/or delivery devices such as those described in U.S. Patent Nos. 3,845,770; 3,916,899; 3,536,809; 3,598,123; 3,630,200 and 4,008,719.

Pharmaceutical compositions of the present invention suitable for oral administration may be presented as discrete units such as capsules (including timed release and sustained release formulations), pills, cachets, powders, granules or tablets each containing a predetermined amount of the active ingredient, as a powder or granules or as a solution or a suspension in an aqueous liquid, a non-aqueous liquid, an oil-in-water emulsion or a water-in-oil liquid emulsion, including elixirs, tinctures, solutions, suspensions, syrups and emulsions. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Desirably, each tablet cachet or capsule contains from about 0.01 to 1,000 mg, particularly 0.01, 0.05, 0.1, 0.5, 1.0, 2, 2.5, 3, 4, 5, 6, 7, 8, 9, 10, 12, 15, 25, 30, 40, S0, 75, 100, 125, 150, 175, 180, 200, 225, 250, 500, 750 and 1,000 milligrams of the active ingredient for the symptomatic adjustment of the dosage to the patient to be treated.

Additional suitable means of administration of the compounds of the present invention include injection, intravenous bolus or infusion, intraperitoneal, subcutaneous, intramuscular, intranasal, and topical, with or without occlusion.

Exemplifying the invention is a pharmaceutical composition comprising any of the compounds described above and a pharmaceutically acceptable carrier. Also exemplifying the invention is a pharmaceutical composition made by combining any of the compounds described above and a pharmaceutically acceptable carrier. An illustration of the invention is a process for making a pharmaceutical composition comprising combining any of the compounds described above and a pharmaceutically acceptable carrier.

The dose may be administered in a single daily dose or the total daily dosage may be administered in divided doses of two, three or four times daily. Furthermore, based on the properties of the individual compound selected for administration, the dose may be administered less frequently, e.g., weekly, twice weekly, monthly, etc. The unit dosage will, of course, be correspondingly larger for the less frequent administration.

When administered via intranasal routes, transdermal routes, by rectal or vaginal suppositories, or through a continual intravenous solution, the dosage administration will, of course, be continuous rather than intermittent throughout the dosage regimen.

The following are examples of representative pharmaceutical dosage forms for the compounds of Formula 1:

| Injectable Suspension (I.M.) | mg/mL | Tablet | mg/tablet |
|---|---|---|---|
| Compound of Formula I | 10 | Compound of Formula I | 25 |
| Methylcellulose | 5.0 | Microcrystalline Cellulose | 415 |
| Tween 80 | 0.5 | Povidone | 14.0 |
| Benzyl alcohol | 9.0 | Pregelatinized Starch | 43.5 |
| Benzalkonium chloride | 1.0 | Magnesium Stearate | 2.5 |
| Water for injection to a total volume of 1 mL | | | 500 |

| Capsule | mg/capsule | Aerosol | Per canister |
|---|---|---|---|
| Compound of Formula I | 25 | Compound of Formula I | 24 mg |
| Lactose Powder | 573.5 | Lecithin, NF Liq. Conc. | 1.2 mg |
| Magnesium Stearate | 1.5 | Trichlorofluoromethane, NF | 4.025 g |
| | 600 | Dichlorodifluoromethane, NF | 12.15 g |

Compounds of Formula I may be used in combination with other drugs that are used in the treatment/prevention/suppression or amelioration of the diseases or conditions for which compounds of Formula I are useful. Such other drugs may be administered, by a route and in an amount commonly used therefor, contemporaneously or sequentially with a compound of Formula I. When a compound of Formula I is used contemporaneously with one or more other drugs, a pharmaceutical composition containing such other drugs in addition to the compound of Formula I is preferred. Accordingly, the pharmaceutical compositions of the present invention include those that also contain one or more other active ingredients, in addition to a compound of Formula I. Examples of other active ingredients that may be combined with a compound of Formula I include, but are not limited to: antipsychotic agents, cognition enhancing agents, anti-migraine agents, anti-asthmatic agents, antiinflammatory agents, anxiolytics, anti-Parkinson's agents, anti-epileptics, anorectic agents, serotonin reuptake inhibitors, other anti-obesity agents, as well as antidiabetic agents, lipid lowering agents, and antihypertensive agents which may be administered separately or in the same pharmaceutical compositions.

The present invention also provides a compound for use in a method for the treatment or prevention of a CB1 receptor modulator mediated disease, which method comprises administration to a patient in need of such treatment or at risk of developing a CB1 receptor modulator mediated disease of an amount of a CB1 receptor modulator and an amount of one or more active ingredients, such that together they give effective relief.

In a further aspect of the present invention, there is provided a pharmaceutical composition comprising a CB1 receptor modulator and one or more active ingredients, together with at least one pharmaceutically acceptable carrier or excipient.

Thus, according to a further aspect of the present invention there is provided the use of a CB1 receptor modulator and one or more active ingredients for the manufacture of a medicament for the treatment or prevention of a CB1 receptor modulator mediated disease. In a further or alternative aspect of the present invention, there is therefore provided a product comprising a CB1 receptor modulator and one or more active ingredients as a combined preparation for simultaneous, separate or sequential use in the treatment or prevention of CB1 receptor modulator mediated disease. Such a combined preparation may be, for example, in the form of a twin pack.

It will be appreciated that for the treatment or prevention of eating disorders, including obesity, bulimia nervosa and compulsive eating disorders, a compound of the present invention may be used in conjunction with other anorectic agents.

The present invention also provides a compound for use in a method for the treatment or prevention of eating disorders, which method comprises administration to a patient in need of such treatment an amount of a compound of the present invention and an amount of an anorectic agent, such that together they give effective relief.

Suitable anorectic agents of use in combination with a compound of the present invention include, but are not limited to, aminorex, amphechloral, amphetamine, benzphetamine, chlorphentermine, clobenzorex, cloforex, clominorex, clortermine, cyclexedrine, dexfenfluramine, dextroamphetamine, diethylpropion, diphemethoxidine, *N*-ethylamphetamine, fenbutrazate, fenfluramine, fenisorex, fenproporex, fludorex, fluminorex, furfurylmethylamphetamine, levamfetamine, levophacetoperane, mazindol, mefenorex, metamfepramone, methamphetamine, norpseudoephedrine, pentorex, phendimetrazine, phenmetrazine, phentermine, phenylpropanolamine, picilorex and sibutramine; and pharmaceutically acceptable salts thereof. A particularly suitable class of anorectic agent are the halogenated amphetamine derivatives, including chlorphentermine, cloforex, clortermine, dexfenfluramine, fenfluramine, picilorex and sibutramine; and pharmaceutically acceptable salts thereof. Particular halogenated amphetamine derivatives of use in combination with a compound of the present invention include: fenfluramine and dexfenfluramine, and pharmaceutically acceptable salts thereof.

The present invention also provides a compound for use in a method for the treatment or prevention of obesity, which method comprises administration to a patient in need of such treatment an amount of a compound of the present invention and an amount of another agent useful in treating obesity and obesity-related conditions, such that together they give effective relief.

Suitable agents of use in combination with a compound of the present invention, include, but are not limited to:
(a) anti-diabetic agents such as (1) PPARγ agonists such as glitazones (e.g. ciglitazone; darglitazone; englitazone; isaglitazone (MCC-555); pioglitazone (ACTOS); rosiglitazone (AVANDIA); troglitazone; rivoglitazone, BRL49653; CLX-0921; 5-BTZD, GW-0207, LG-100641, R483, and LY-300512, and the like and compounds disclosed in WO97/10813, 97/27857, 97/28115, 97/28137, 97/27847, 03/000685, and 03/027112 and SPPARMS (selective PPAR gamma modulators) such as T131 (Amgen), FK614 (Fujisawa), netoglitazone, and metaglidasen; (2) biguanides such as buformin; metformin; and phenformin, and the like; (3) protein tyrosine phosphatase-1B (PTP-1B) inhibitors such as ISIS 113715, A-401674, A-364504, IDD-3, IDD 2846, KP-40046, KR61639, MC52445, MC52453, C7, OC-060062, OC-86839, OC29796, TTP-277BC1, and those agents disclosed in WO 04/041799, 04/050646, 02/26707, 02/26743, 04/092146, 03/048140, 04/089918, 03/002569, 04/065387, 04/127570, and US 2004/167183; (4) sulfonylureas such as acetohexamide; chlorpropamide; diabinese; glibenclamide; glipizide; glyburide; glimepiride; gliclazide; glipentide; gliquidone; glisolamide; tolazamide; and tolbutamide, and the like; (5) meglitinides such as repaglinide, metiglinide (GLUFAST) and nateglinide, and the like; (6) alpha glucoside hydrolase inhibitors such as acarbose; adiposine; camiglibose; emiglitate; miglitol; voglibose; pradimicin-Q; salbostatin; CKD-711; MDL-25,637; MDL-73,945; and MOR 14, and the like; (7) alpha-amylase inhibitors such as tendamistat, trestatin, and A1-3688, and the like; (8) insulin secreatagogues such as linogliride nateglinide, mitiglinide (GLUFAST), ID1101 A-4166, and the like; (9) fatty acid oxidation inhibitors, such as clomoxir, and etomoxir, and the like; (10) A2 antagonists, such as midaglizole; isaglidole; deriglidole; idazoxan; earoxan; and fluparoxan, and the like; (11) insulin or insulin mimetics, such as biota, LP-100, novarapid, insulin detemir, insulin lispro, insulin glargine, insulin zinc suspension (lente and ultralente); Lys-Pro insulin, GLP-1 (17-36), GLP-1 (73-7) (insulintropin); GLP-1 (7-36)-NH₂) exenatide/Exendin-4, Exenatide LAR, Linaglutide, AVE0010, CJC 1131, BIM51077, CS 872, THO318 BAY-694326, GPO10, ALBUGON (GLP-1 fused to albumin), HGX-007 (Epac agonist), S-23521, and compounds disclosed in WO 04/022004, WO 04/37859, and the like; (12) non-thiazolidinediones such as JT-501, and farglitazar (GW-2570/GI-262579), and the like; (13) PPARα/γ dual agonists such as AVE 0847, CLX-0940, GW-1536, GW1929, GW-2433, KRP-297, L-796449, LBM 642, LR-90, LY510919, MK-0767, ONO 5129, SB 219994, TAK-559, TAK-654, 677954 (GlaxoSmithkline), E-3030 (Eisai), LY510929 (Lilly), AK109 (Asahi), DRF2655 (Dr. Reddy), DRF8351 (Dr. Reddy), MC3002 (Maxocore), TY51501 (ToaEiyo), naveglitazar, muraglitizar, peliglitazar, tesaglitazar (GALIDA), reglitazar (JTT-501), chiglitazar, and those disclosed in WO 99/16758, WO 99/19313, WO 99/20614, WO 99/38850, WO 00/23415, WO 00/23417, WO 00/23445, WO 00/50414, WO 01/00579, WO 01/79150, WO 02/062799, WO 03/033481, WO 03/033450, WO 03/033453; and (14) other insulin sensitizing drugs; (15) VPAC2 receptor agonists; (16) GLK modulators, such as PSN105, RO 281675, RO 274375 and those disclosed in WO 03/015774, WO 03/000262, WO 03/055482, WO 04/046139, WO 04/045614, WO 04/063179, WO 04/063194, WO 04/050645, and the like; (17) retinoid modulators such as those disclosed in WO 03/000249; (18) GSK 3beta/GSK 3 inhibitors such as 4-[2-(2-bromophenyl)-4-(4-fluorophenyl-1*H-*imidazol-5-yl]pyridine, CT21022, CT20026, CT-98023, SB-216763, Sub410111, SB-675236, CP-70949, XD4241 and those compounds disclosed in WO 03/037869, 03/03877, 03/037891, 03/024447, 05/000192, 05/019218 and the like; (19) glycogen phosphorylase (HGLPa) inhibitors, such as AVE 5688, PSN 357, GPi-879, those disclosed in WO 03/037864, WO 03/091213, WO 04/092158, WO 05/013975, WO 05/013981, US 2004/0220229, and JP 2004-196702, and the like; (20) ATP consumption promotors such as those disclosed in WO 03/007990; (21) fixed combinations of PPAR γ agonists and metformin such as AVANDAMET; (22) PPAR pan agonists such as GSK 677954; (23) GPR40 (G-protein coupled receptor 40) also called SNORF 55 such as BG 700, and those disclosed in WO 04/041266, 04/022551, 03/099793; (24) GPR119 (G-protein coupled receptor 119, also called RUP3; SNORF 25) such as RUP3, HGPRBMY26, PFI 007, SNORF 25; (25) adenosine receptor 2B antagonists such as ATL-618, ATI-802, E3080, and the like; (26) carnitine palmitoyl transferase inhibitors such as ST 1327, and ST 1326, and the like; (27) Fructose 1,6-bisphospohatase inhibitors such as CS-917, MB7803, and the like; (28) glucagon antagonists such as AT77077, BAY 694326, GW 4123X, NN2501, and those disclosed in WO 03/064404, WO 05/00781, US 2004/0209928, US 2004/029943, and the like; (30) glucose-6-phosphase inhibitors; (31) phosphoenolpyruvate carboxykinase (PEPCK) inhibitors; (32) pyruvate dehydrogenase kinase (PDK) activators; (33) RXR agonists such as MC1036, CS00018, JNJ 10166806, and those disclosed in WO 04/089916, US 6759546, and the like; (34) SGLT inhibitors such as AVE 2268, KGT 1251, T1095/RWJ 394718; (35) BLX-1002; (36) alpha glucosidase inhibitors; (37) glucagon receptor agonists; (38) glucokinase activators; and (39) dipeptidyl peptidase IV (DPP-4) inhibitors, including but not limited to, isoleucine thiazolidide, valine pyrrolidide, sitagliptin, saxagliptin, NVP-DPP728, LAF237 (vildagliptin), P93/01, TSL 225, TMC-2A/2B/2C, FE 999011, P9310/K364, VIP 0177, SDZ 274-444, GSK 823093, E 3024, SYR 322, TS021, SSR 162369, GRC 8200, K579, NN7201, CR 14023, PHX 1004, PHX 1149, PT-630, and SK-0403;
(b) lipid lowering agents such as (1) bile acid sequestrants such as, cholestyramine, colesevelem, colestipol, dialkylaminoalkyl derivatives of a cross-linked dextran; Colestid®; LoCholest®; and Questran®, and the like; (2) HMG-CoA reductase inhibitors such as atorvastatin, itavastatin, pitavastatin, fluvastatin, lovastatin, pravastatin, rivastatin, rosuvastatin, simvastatin, rosuvastatin (ZD-4522), and the like, particularly simvastatin; (3) HMG-CoA synthase inhibitors; (4) cholesterol absorption inhibitors such as FMVP4 (Forbes Medi-Tech), KT6-971 (Kotobuki Pharmaceutical), FM-VA12 (Forbes Medi-Tech), FM-VP-24 (Forbes Medi-Tech), stanol esters, beta-sitosterol, sterol glycosides such as tiqueside; and azetidinones such as ezetimibe, and those disclosed in WO 04/005247 and the like; (5) acyl coenzyme A -cholesterol acyl transferase (ACAT) inhibitors such as avasimibe, eflucimibe, pactimibe (KY505), SMP 797 (Sumitomo), SM32504 (Sumitomo), and those disclosed in WO 03/091216, and the like; (6) CETP inhibitors such as JTT 705 (Japan Tobacco), torcetrapib, CP 532,632, BAY63-2149 (Bayer), SC 591, SC 795, and the like; (7) squalene synthetase inhibitors; (8) anti-oxidants such as probucol, and the like; (9) PPARα agonists such as beclofibrate, benzafibrate, ciprofibrate, clofibrate, etofibrate, fenofibrate, gemcabene, and gemfibrozil, GW 7647, BM 170744 (Kowa), LY518674 (Lilly), GW590735 (GlaxoSmithkline), KRP-101 (Kyorin), DRF10945 (Dr. Reddy), NS-220/R1593 (Nippon Shinyaku/Roche, ST1929 (Sigma Tau) MC3001/MC3004 (MaxoCore Pharmaceuticals, gemcabene calcium, other fibric acid derivatives, such as Atromid®, Lopid® and Tricor®, and those disclosed in US 6,548,538, and the like; (10) FXR receptor modulators such as GW 4064 (GlaxoSmithkline), SR 103912, QRX401, LN-6691 (Lion Bioscience), and those disclosed in WO 02/064125, WO 04/045511, and the like; (11) LXR receptor modulators such as GW 3965 (GlaxoSmithkline), T9013137, and XTCO179628 (X-Ceptor Therapeutics/Sanyo), and those disclosed in WO 03/031408, WO 03/063796, WO 04/072041, and the like; (12) lipoprotein synthesis inhibitors such as niacin; (13) renin angiotensin system inhibitors; (14) PPAR δ partial agonists, such as those disclosed in WO 03/024395; (15) bile acid reabsorption inhibitors, such as BARI 1453, SC435, PHA384640, S8921, AZD7706, and the like; and bile acid sequesterants such as colesevelam (WELCHOL/ CHOLESTAGEL), (16) PPARδ agonists such as GW 501516 (Ligand, GSK), GW 590735, GW-0742 (GlaxoSmithkline), T659 (Amgen/Tularik), LY934 (Lilly), NNC610050 (Novo Nordisk) and those disclosed in WO97/28149, WO 01/79197, WO 02/14291, WO 02/46154, WO 02/46176, WO 02/076957, WO 03/016291, WO 03/033493, WO 03/035603, WO 03/072100, WO 03/097607, WO 04/005253, WO 04/007439, and JP10237049, and the like; (17) triglyceride synthesis inhibitors; (18) microsomal triglyceride transport (MTTP) inhibitors, such as implitapide, LAB687, JTT130 (Japan Tobacco), CP346086, and those disclosed in WO 03/072532, and the like; (19) transcription modulators; (20) squalene epoxidase inhibitors; (21) low density lipoprotein (LDL) receptor inducers; (22) platelet aggregation inhibitors; (23) 5-LO or FLAP inhibitors; and (24) niacin receptor agonists including HM74A receptor agonists; (25) PPAR modulators such as those disclosed in WO 01/25181, WO 01/79150, WO 02/79162, WO 02/081428, WO 03/016265, WO 03/033453; (26) niacin-bound chromium, as disclosed in WO 03/039535; (27) substituted acid derivatives disclosed in WO 03/040114; (28) infused HDL such as LUV/ETC-588 (Pfizer), APO-A1 Milano/ETC216 (Pfizer), ETC-642 (Pfizer), ISIS301012, D4F (Bruin Pharma), synthetic trimeric ApoA1, Bioral Apo A1 targeted to foam cells, and the like; (29) IBAT inhibitors such as BARI143/HMR145A/ HMR1453 (Sanofi-Aventis, PHA384640E (Pfizer), S8921 (Shionogi) AZD7806 (AstrZeneca), AK105 (Asah Kasei), and the like; (30) Lp-PLA2 inhibitors such as SB480848 (GlaxoSmithkline), 659032 (GlaxoSmithkline), 677116 (GlaxoSmithkline), and the like; (31) other agents which affect lipic composition including ETC1001/ESP31015 (Pfizer), ESP-55016 (Pfizer), AGI1067 (AtheroGenics), AC3056 (Amylin), AZD4619 (AstrZeneca); and
(c) anti-hypertensive agents such as (1) diuretics, such as thiazides, including chlorthalidone, chlorthiazide, dichlorophenamide, hydroflumethiazide, indapamide, and hydrochlorothiazide; loop diuretics, such as bumetanide, ethacrynic acid, furosemide, and torsemide; potassium sparing agents, such as amiloride, and triamterene; and aldosterone antagonists, such as spironolactone, epirenone, and the like; (2) beta-adrenergic blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, propanolol, sotalol, tertatolol, tilisolol, and timolol, and the like; (3) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodepine, nisoldipine, nitrendipine, manidipine, pranidipine, and verapamil, and the like; (4) angiotensin converting enzyme (ACE) inhibitors such as benazepril; captopril; cilazapril; delapril; enalapril; fosinopril; imidapril; losinopril; moexipril; quinapril; quinaprilat; ramipril; perindopril; perindropril; quanipril; spirapril; tenocapril; trandolapril, and zofenopril, and the like; (5) neutral endopeptidase inhibitors such as omapatrilat, cadoxatril and ecadotril, fosidotril, sampatrilat, AVE7688, ER4030, and the like; (6) endothelin antagonists such as tezosentan, A308165, and YM62899, and the like; (7) vasodilators such as hydralazine, clonidine, minoxidil, and nicotinyl alcohol, and the like; (8) angiotensin II receptor antagonists such as candesartan, eprosartan, irbesartan, losartan, pratosartan, tasosartan, telmisartan, valsartan, and EXP-3137, FI6828K, and RNH6270, and the like; (9) α/β adrenergic blockers as nipradilol, arotinolol and amosulalol, and the like; (10) alpha 1 blockers, such as terazosin, urapidil, prazosin, bunazosin, trimazosin, doxazosin, naftopidil, indoramin, WHIP 164, and XEN010, and the like; (11) alpha 2 agonists such as lofexidine, tiamenidine, moxonidine, rilmenidine and guanobenz, and the like; (12) aldosterone inhibitors, and the like; (13) angiopoietin-2-binding agents such as those disclosed in WO 03/030833; and
(d) anti-obesity agents, such as (1) 5HT (serotonin) transporter inhibitors, such as paroxetine, fluoxetine, fenfluramine, fluvoxamine, sertraline, and imipramine, and those disclosed in WO 03/00663, as well as serotonin/noradrenaline re uptake inhibitors such as sibutramine (MERIDIA/REDUCTIL) and dopamine uptake inhibitor/Norepenephrine uptake inhibitors such as radafaxine hydrochloride, 353162 (GlaxoSmithkline), and the like; (2) NE (norepinephrine) transporter inhibitors, such as GW 320659, despiramine, talsupram, and nomifensine; (3) CB1 (cannabinoid-1 receptor) antagonist/inverse agonists, such as rimonabant (ACCOMPLIA Sanofi Synthelabo), SR-147778 (Sanofi Synthelabo), AVE1625 (Sanofi-Aventis), BAY 65-2520 (Bayer), SLV 319 (Solvay), SLV326 (Solvay), CP945598 (Pfizer), E-6776 (Esteve), 01691 (Organix), ORG14481 (Organon), VER24343 (Vernalis), NESS0327 (Univ of Sassari/Univ of Cagliari), and those disclosed in US Patent Nos. 4,973,587, 5,013,837, 5,081,122, 5,112,820, 5,292,736, 5,532,237, 5,624,941, 6,028,084, and 6,509367; and WO 96/33159, WO97/29079, WO98/31227, WO 98/33765, WO98/37061, WO98/41519, WO98/43635, WO98/43636, WO99/02499, WO00/10967, WO00/10968, WO 01/09120, WO 01/58869, WO 01/64632, WO 01/64633, WO 01/64634, WO 01/70700, WO 01/96330, WO 02/076949, WO 03/006007, WO 03/007887, WO 03/020217, WO 03/026647, WO 03/026648, WO 03/027069, WO 03/027076, WO 03/027114, WO 03/037332, WO 03/040107, WO 04/096763, WO 04/111039, WO 04/111033, WO 04/111034, WO 04/111038, WO 04/013120, WO 05/000301, WO 05/016286, WO 05/066126 and EP-658546 and the like; (4) ghrelin agonists/antagonists, such as BVT81-97 (BioVitrum), RC1291 (Rejuvenon), SRD-04677 (Sumitomo), unacylated ghrelin (TheraTechnologies), and those disclosed in WO 01/87335, WO 02/08250, WO 05/012331, and the like; (5) H3 (histamine H3) antagonist/inverse agonists, such as thioperamide, 3-(1*H-*imidazol-4-yl)propyl N-(4-pentenyl)carbamate), clobenpropit, iodophenpropit, imoproxifan, GT2394 (Gliatech), and A331440, and those disclosed in WO 02/15905; and O-[3-(1*H-*imidazol-4-yl)propanol]carbamates (Kiec-Kononowicz, K. et al., Pharmazie, 55:349-55 (2000)), piperidine-containing histamine H3-receptor antagonists (Lazewska, D. et al., Pharmazie, 56:927-32 (2001), benzophenone derivatives and related compounds (Sasse, A. et al., Arch. Pharm.(Weinheim) 334:45-52 (2001)), substituted N-phenylcarbamates (Reidemeister, S. et al., Pharmazie, 55:83-6 (2000)), and proxifan derivatives (Sasse, A. et al., J. Med. Chem.. 43:3335-43 (2000)) and histamine H3 receptor modulators such as those disclosed in WO 03/024928 and WO 03/024929; (6) melanin-concentrating hormone 1 receptor (MCH1R) antagonists, such as T-226296 (Takeda), T71 (Takeda/Amgen), AMGN-608450, AMGN-503796 (Amgen), 856464 (GlaxoSmithkline), A224940 (Abbott), A798 (Abbott), ATC0175/AR224349 (Arena Pharmaceuticals), GW803430 (GlaxoSmithkine), NBI-1A (Neurocrine Biosciences), NGX-1 (Neurogen), SNP-7941 (Synaptic), SNAP9847 (Synaptic), T-226293 (Schering Plough), TPI-1361-17 (Saitama Medical School/University of California Irvine), and those disclosed WO 01/21169, WO 01/82925, WO 01/87834, WO 02/051809, WO 02/06245, WO 02/076929, WO 02/076947, WO 02/04433, WO 02/51809, WO 02/083134, WO 02/094799, WO 03/004027, WO 03/13574, WO 03/15769, WO 03/028641, WO 03/035624, WO 03/033476, WO 03/033480, WO 04/004611, WO 04/004726, WO 04/011438, WO 04/028459, WO 04/034702, WO 04/039764, WO 04/052848, WO 04/087680; and Japanese Patent Application Nos. JP 13226269, JP 1437059, JP2004315511, and the like; (7) MCH2R (melanin concentrating hormone 2R) agonist/antagonists; (8) NPY1 (neuropeptide Y Y1) antagonists, such as BMS205749, BIBP3226, J-115814, BIBO 3304, LY-357897, CP-671906, and GI-264879A; and those disclosed in U.S. Patent No. 6,001,836; and WO 96/14307, WO 01/23387, WO 99/51600, WO 01/85690, WO 01/85098, WO 01/85173, and WO 01/89528; (9) NPY5 (neuropeptide Y Y5) antagonists, such as 152,804, S2367 (Shionogi), E-6999 (Esteve), GW-569180A, GW-594884A (GlaxoSmithkline), GW-587081X, GW-548118X; FR 235,208; FR226928, FR 240662, FR252384; 1229U91, GI-264879A, CGP71683A, C-75 (Fasgen) LY-377897, LY366377, PD-160170, SR-120562A, SR-120819A,S2367 (Shionogi), JCF-104, and H409/22; and those compounds disclosed in U.S. Patent Nos. 6,140,354, 6,191,160, 6,258,837, 6,313,298, 6,326,375, 6,329,395, 6,335,345, 6,337,332, 6,329,395, and 6,340,683 ; and EP-01010691, EP-01044970, and FR252384; and PCT Publication Nos. WO 97/19682, WO 97/20820, WO 97/20821, WO 97/20822, WO 97/20823, WO 98/27063, WO 00/107409, WO 00/185714, WO 00/185730, WO 00/64880, WO 00/68197, WO 00/69849, WO 01/09120, WO 01/14376, WO 01/85714, WO 01/85730, WO 01/07409, WO 01/02379, WO 01/02379, WO 01/23388, WO 01/23389, WO 01/44201, WO 01/62737, WO 01/62738, WO 01/09120, WO 02/20488, WO 02/22592, WO 02/48152, WO 02/49648, WO 02/051806, WO 02/094789, WO 03/009845, WO 03/014083, WO 03/022849, WO 03/028726, WO 05/014592, WO 05/01493; and Norman et al., J. Med. Chem. 43:4288-4312 (2000); (10) leptin, such as recombinant human leptin (PEG-OB, Hoffman La Roche) and recombinant methionyl human leptin (Amgen); (11) leptin derivatives, such as those disclosed in Patent Nos. 5,552,524; 5,552,523; 5,552,522; 5,521,283; and WO 96/23513; WO 96/23514; WO 96/23515; WO 96/23516; WO 96/23517; WO 96/23518; WO 96/23519; and WO 96/23520; (12) opioid antagonists, such as nalmefene (Revex ®), 3-methoxynaltrexone, naloxone, and naltrexone; and those disclosed in WO 00/21509; (13) orexin antagonists, such as SB-334867-A (GlaxoSmithkline); and those disclosed in WO 01/96302, 1/68609, 02/44172, 02/51232, 02/51838, 02/089800, 02/090355, 03/023561, 03/032991, 03/037847, 04/004733, 04/026866, 04/041791, 04/085403, and the like; (14) BRS3 (bombesin receptor subtype 3) agonists; (15) CCK-A (cholecystokinin-A) agonists, such as AR-R 15849, GI 181771, JMV-180, A-71378, A-71623, PD170292, PD 149164, SR146131, SR125180, butabindide, and those disclosed in US 5,739,106; (16) CNTF (ciliary neurotrophic factors), such as GI-181771 (Glaxo-SmithKline); SR146131 (Sanofi Synthelabo); butabindide; and PD170,292, PD 149164 (Pfizer); (17) CNTF derivatives, such as axokine (Regeneron); and those disclosed in WO 94/09134, WO 98/22128, and WO 99/43813; (18) GHS (growth hormone secretagogue receptor) agonists, such as NN703, hexarelin, MK-0677, SM-130686, CP-424,391, L-692,429 and L-163,255, and those disclosed in U.S. Patent No. 6358951, U.S. Patent Application Nos. 2002/049196 and 2002/022637; and WO 01/56592, and WO 02/32888; (19) 5HT2c (serotonin receptor 2c) agonists, such as APD3546/AR10A (Arena Pharmaceuticals), ATH88651 (Athersys), ATH88740 (Athersys), BVT933 (Biovitrum/GSK), DPCA37215 (BMS), IK264; LY448100 (Lilly), PNU 22394; WAY 470 (Wyeth), WAY629 (Wyeth), WAY161503 (Biovitrum), R-1065, VR1065 (Vernalis/Roche) YM 348; and those disclosed in U.S. Patent No. 3,914,250; and PCT Publications 01/66548, 02/36596, 02/48124, 02/10169, 02/44152; 02/51844, 02/40456, 02/40457, 03/057698, 05/000849, and the like; (20) Mc3r (melanocortin 3 receptor) agonists; (21) Mc4r (melanocortin 4 receptor) agonists, such as CHIR86036 (Chiron), CHIR915 (Chiron); ME-10142 (Melacure), ME-10145 (Melacure), HS-131 (Melacure), NBI72432 (Neurocrine Biosciences), NNC 70-619 (Novo Nordisk), TTP2435 (Transtech)and those disclosed in PCT Publications WO 99/64002, 00/74679, 01/991752, 01/0125192, 01/52880, 1/74844, 1/70708, 1/70337, 1/91752, 01/010842, 02/059095, 02/059107, 02/059108, 02/059117, 02/062766, 02/069095, 02/12166, 02/11715, 02/12178, 02/15909, 02/38544, 02/068387, 02/068388, 02/067869, 02/081430, 03/06604, 03/007949, 03/009847, 03/009850, 03/013509, 03/031410, 03/094918, 04/028453, 04/048345, 04/050610, 04/075823, 04/083208, 04/089951, 05/000339, and EP 1460069, and US 2005049269, and JP2005042839, and the like; (22) monoamine reuptake inhibitors, such as sibutratmine (Meridia ®/Reductil®) and salts thereof, and those compounds disclosed in U.S. Patent Nos. 4,746,680, 4,806,570, and 5,436,272, and U.S. Patent Publication No. 2002/0006964, and WO 01/27068, and WO 01/62341; (23) serotonin reuptake inhibitors, such as dexfenfluramine, fluoxetine, and those in U.S. Patent No. 6,365,633, and WO 01/27060, and WO 01/162341; (24) GLP-1 (glucagon-like peptide 1) agonists; (25) Topiramate (Topimax®); (26) phytopharm compound 57 (CP 644,673); (27) ACC2 (acetyl-CoA carboxylase-2) inhibitors; (28) β3 (beta adrenergic receptor 3) agonists, such as rafebergron/AD9677/TAK677 (Dainippon/ Takeda), CL-316,243, SB 418790, BRL-37344, L-796568, BMS-196085, BRL-35135A, CGP12177A, BTA-243, GRC1087 (Glenmark Pharmaceuticals) GW 427353 (solabegron hydrochloride), Trecadrine, Zeneca D7114, N-5984 (Nisshin Kyorin), LY-377604 (Lilly), KT07924 (Kissei), SR 59119A, and those disclosed in US Patent Nos. 5,705,515, US 5,451,677; and WO94/18161, WO95/29159, WO97/46556, WO98/04526 WO98/32753, WO 01/74782, WO 02/32897, WO 03/014113, WO 03/016276, WO 03/016307, WO 03/024948, WO 03/024953, WO 03/037881, WO 04/108674, and the like; (29) DGAT1 (diacylglycerol acyltransferase 1) inhibitors; (30) DGAT2 (diacylglycerol acyltransferase 2)inhibitors; (31) FAS (fatty acid synthase) inhibitors, such as Cerulenin and C75; (32) PDE (phosphodiesterase) inhibitors, such as theophylline, pentoxifylline, zaprinast, sildenafil, amrinone, milrinone, cilostamide, rolipram, and cilomilast, as well as those described in WO 03/037432, WO 03/037899; (33) thyroid hormone β agonists, such as KB-2611 (KaroBioBMS), and those disclosed in WO 02/15845; and Japanese Patent Application No. JP 2000256190; (34) UCP-1 (uncoupling protein 1), 2, or 3 activators, such as phytanic acid, 4-[(E)-2-(5,6,7,8-tetrahydro-5,5,8,8-tetramethyl-2-napthalenyl)-1-propenyl]benzoic acid (TTNPB), and retinoic acid; and those disclosed in WO 99/00123; (35) acyl-estrogens, such as oleoyl-estrone, disclosed in del Mar-Grasa, M. et al., Obesity Research, 9:202-9 (2001); (36) glucocorticoid receptor antagonists, such as CP472555 (Pfizer), KB 3305, and those disclosed in WO 04/000869, WO 04/075864, and the like; (37) 11β HSD-1 (11-beta hydroxy steroid dehydrogenase type 1) inhibitors, such as BVT 3498 (AMG 331), BVT 2733, 3-(1-adamantyl)-4-ethyl-5-(ethylthio)-4*H-*1,2,4-triazole, 3-(1-adamantyl)-5-(3,4,5-trimethoxyphenyl)-4-methyl-4*H-*1,2,4-triazole, 3-adamantanyl-4,5,6,7,8,9,10,11,12,3a-decahydro-1,2,4-triazolo[4,3-a][11]annulene, and those compounds disclosed in WO 01/90091, 01/90090, 01/90092, 02/072084, 04/011410, 04/033427, 04/041264, 04/027047, 04/056744, 04/065351, 04/089415, 04/037251, and the like; (38) SCD-1 (stearoyl-CoA desaturase-1) inhibitors; (39) dipeptidyl peptidase IV (DPP-4) inhibitors, such as isoleucine thiazolidide, valine pyrrolidide, sitagliptin, saxagliptin, NVP-DPP728, LAF237 (vildagliptin), P93/01, TSL 225, TMC-2A/2B/2C, FE 999011, P9310/K364, VIP 0177, SDZ 274-444, GSK 823093, E 3024, SYR 322, TS021, SSR 162369, GRC 8200, K579, NN7201, CR 14023, PHX 1004, PHX 1149, PT-630, SK-0403; and the compounds disclosed in WO 02/083128, WO 02/062764, WO 02/14271, WO 03/000180, WO 03/000181, WO 03/000250, WO 03/002530, WO 03/002531, WO 03/002553, WO 03/002593, WO 03/004498, WO 03/004496, WO 03/005766, WO 03/017936, WO 03/024942, WO 03/024965, WO 03/033524, WO 03/055881, WO 03/057144, WO 03/037327, WO 04/041795, WO 04/071454, WO 04/0214870, WO 04/041273, WO 04/041820, WO 04/050658, WO 04/046106, WO 04/067509, WO 04/048532, WO 04/099185, WO 04/108730, WO 05/009956, WO 04/09806, WO 05/023762, US 2005/043292, and EP 1 258 476; (40) lipase inhibitors, such as tetrahydrolipstatin (orlistat/XENICAL), ATL962 (Alizyme/Takeda), GT389255 (Genzyme/Peptimmune)Triton WR1339, RHC80267, lipstatin, teasaponin, and diethylumbelliferyl phosphate, FL-386, WAY-121898, Bay-N-3176, valilactone, esteracin, ebelactone A, ebelactone B, and RHC 80267, and those disclosed in WO 01/77094, WO 04/111004, and U.S. Patent Nos. 4,598,089, 4,452,813, 5,512,565, 5,391,571, 5,602,151, 4,405,644, 4,189,438, and 4,242,453, and the like; (41) fatty acid transporter inhibitors; (42) dicarboxylate transporter inhibitors; (43) glucose transporter inhibitors; and (44) phosphate transporter inhibitors; (45) anorectic bicyclic compounds such as 1426 (Aventis) and 1954 (Aventis), and the compounds disclosed in WO 00/18749, WO 01/32638, WO 01/62746, WO 01/62747, and WO 03/015769; (46) peptide YY and PYY agonists such as PYY336 (Nastech/Merck), AC162352 (IC Innovations/Curis/Amylin), TM30335/TM30338 (7TM Pharma), PYY336 (Emisphere Tehcnologies), pegylated peptide YY3-36, those disclosed in WO 03/026591, 04/089279, and the like; (47) lipid metabolism modulators such as maslinic acid, erythrodiol, ursolic acid uvaol, betulinic acid, betulin, and the like and compounds disclosed in WO 03/011267; (48) transcription factor modulators such as those disclosed in WO 03/026576; (49) Mc5r (melanocortin 5 receptor) modulators, such as those disclosed in WO 97/19952, WO 00/15826, WO 00/15790, US 20030092041, and the like; (50) Brain derived neutotropic factor (BDNF), (51) Mc1r (melanocortin 1 receptor modulators such as LK-184 (Proctor & Gamble), and the like; (52) 5HT6 antagonists such as BVT74316 (BioVitrum), BVT5182c (BioVitrum), E-6795 (Esteve), E-6814 (Esteve), SB399885 (GlaxoSmithkline), SB271046 (GlaxoSmithkline), RO-046790 (Roche), and the like; (53) fatty acid transport protein 4 (FATP4); (54) acetyl-CoA carboxylase (ACC) inhibitors such as CP640186, CP610431, CP640188 (Pfizer); (55) C-terminal growth hormone fragments such as AOD9604 (Monash Univ/Metabolic Pharmaceuticals), and the like; (56) oxyntomodulin; (57) neuropeptide FF receptor antagonists such as those disclosed in WO 04/083218, and the like; (58) amylin agonists such as Symlin/pramlintide/AC137 (Amylin); (59) Hoodia and trichocaulon extracts; (60) BVT74713 and other gut lipid appetite suppressants; (61) dopamine agonists such as bupropion (WELLBUTRIN/GlaxoSmithkline); (62) zonisamide (ZONEGRAN/Dainippon/Elan), and the like.

Specific compounds of use in combination with a compound of the present invention include: simvastatin, mevastatin, ezetimibe, atorvastatin, sitagliptin, metformin, sibutramine, orlistat, Qnexa, topiramate, naltrexone, bupriopion, phentermine, and losartan, losartan with hydrochlorothiazide. Specific CB1 antagonists/inverse agonists of use in combination with a compound of the present invention include: those described in WO03/077847, including: *N-*[3-(4-chlorophenyl)-2(*S*)-phenyl-1(*S*)-methylpropyl]-2-(4-trifluoromethyl-2-pyrimidyloxy)-2-methylpropanamide, *N-*[3-(4-chlorophenyl)-2-(3-cyanophenyl)-1-methylpropyl]-2-(5-trifluoromethyl-2-pyridyloxy)-2-methylpropanamide, *N-*[3-(4-chlorophenyl)-2-(5-chloro-3-pyridyl)-1-methylpropyl]-2-(5-trifluoromethyl-2-pyridyloxy)-2-methylpropanamide, and pharmaceutically acceptable salts thereof; as well as those in WO05/000809, which includes the following: 3-{1-[bis(4-chlorophenyl)methyl]azetidin-3-ylidene}-3-(3,5-difluorophenyl)-2,2-dimethylpropanenitrile, 1-{1-[1-(4-chlorophenyl)pentyl]azetidin-3-yl}-1-(3,5-difluorophenyl)-2-methylpropan-2-ol. 3-((S)-(4-chlorophenyl) {3-[(1S)-1-(3,5-difluorophenyl)-2-hydroxy-2-methylpropyl]azetidin-1-yl}methyl)benzonitrile, 3-((S)-(4-chlorophenyl){3-[(1S)-1-(3,5-difluorophenyl)-2-fluoro-2-methylpropyl]azetidin-1-yl}methyl)benzonitrile, 3-((4-chlorophenyl){3-[1-(3,5-difluorophenyl)-2,2-dimethylpropyl]azetidin-1-yl}methyl)benzonitrile, 3-((1S)-1-{1-[(S)-(3-cyanophenyl)(4-cyanophenyl)methyl]azetidin-3-yl}-2-fluoro-2-methylpropyl)-5-fluorobenzonitrile, 3-[(S)-(4-chlorophenyl)(3-{(1S)-2-fluoro-1-[3-fluoro-5-(4H-1,2,4-triazol-4-yl)phenyl]-2-methylpropyl}azetidin-1-yl)methyl]benzonitrile, and 5-((4-chlorophenyl){3-[(1S)-1-(3,5-difluorophenyl)-2-fluoro-2-methylpropyl]azetidin-1-yl}methyl)thiophene-3-carbonitrile, and pharamecueitcally acceptable salts thereof; as well as: 3-[(*S*)-(4-chlorophenyl)(3-{(1*S*)-2-fluoro-1-[3-fluoro-5-(5-oxo-4,5-dihydro-1,3,4-oxadiazol-2-yl)phenyl]-2-methylpropyl} azetidin-1-yl)methyl]benzonitrile, 3-[(*S*)-(4-chlorophenyl)(3-{(1*S*)-2-fluoro-1-[3-fluoro-5-(1,3,4-oxadiazol-2-yl)phenyl]-2-methylpropyl}azetidin-1-yl)methyl]benzonitrile, 3-[(*S*)-(3-{(1*S*)-1-[3-(5-amino-1,3,4-oxadiazol-2-yl)-5-fluorophenyl]-2-fluoro-2-methylpropyl} azetidin-1-yl)(4-chlorophenyl)methyl]benzonitrile, 3-[(*S*)-(4-cyanophenyl)(3-{(1*S*)-2-fluoro-1-[3-fluoro-5-(5-oxo-4,5-dihydro-1,3,4-oxadiazol-2-yl)phenyl]-2-methylpropyl}azetidin-1-yl)methyl]benzonitrile, 3-[(*S*)-(3-{(1*S*)-1-[3-(5-amino-1,3,4-oxadiazol-2-yl)-5-fluorophenyl]-2-fluoro-2-methylpropyl}azetidin-1-yl)(4-cyanophenyl)methyl]benzonitrile, 3-[(*S*)-(4-cyanophenyl)(3-{(1*S*)-2-fluoro-1-[3-fluoro-5-(1,3,4-oxadiazol-2-yl)phenyl]-2-methylpropyl}azetidin-1-yl)methyl]benzonitrile, 3-[(*S*)-(4-chlorophenyl)(3-{(1*S*)-2-fluoro-1-[3-fluoro-5-(1,2,4-oxadiazol-3-yl)phenyl]-2-methylpropyl}azetidin-1-yl)methyl]benzonitrile, 3-[(1*S*)-1-(1-{(*S*)-(4-cyanophenyl)[3-(1,2,4-oxadiazol-3-yl)phenyl]-methyl}azetidin-3-yl)-2-fluoro-2-methylpropyl]-5-fluorobenzonitrile, 5-(3-{1-[1-(diphenylmethyl)azetidin-3-yl]-2-fluoro-2-methylpropyl}-5-fluorophenyl)-1*H-*tetrazole, 5-(3-{1-[1-(diphenylmethyl)azetidin-3-yl]-2-fluoro-2-methylpropyl}-5-fluorophenyl)-1-methyl-1*H-*tetrazole, 5-(3-{1-[1-(diphenylmethyl)azetidin-3-yl]-2-fluoro-2-methylpropyl}-5-fluorophenyl)-2-methyl-2H-tetrazole, 3-[(4-chlorophenyl)(3-{2-fluoro-1-[3-fluoro-5-(2-methyl-2H-tetrazol-5-yl)phenyl]-2-methylpropyl}azetidin-1-yl)methyl]benzonitrile, 3-[(4-chlorophenyl)(3-{2-fluoro-1-[3-fluoro-5-(1-methyl-1*H-*tetrazol-5-yl)phenyl]-2-methylpropyl}azetidin-1-yl)methyl]benzonitrile, 3-[(4-cyanophenyl)(3-{2-fluoro-1-[3-fluoro-5-(1-methyl-1*H-*tetrazol-5-yl)phenyl]-2-methylpropyl}azetidin-1-yl)methyl]benzonitrile, 3-[(4-cyanophenyl)(3-{2-fluoro-1-[3-fluoro-5-(2-methyl-2*H-*tetrazol-5-yl)phenyl]-2-methylpropyl}azetidin-1-yl)methyl]benzonitrile, 5-{3-[(*S*)-{3-[(1*S*)-1-(3-bromo-5-fluorophenyl)-2-fluoro-2-methylpropyl]azetidin-1-yl}(4-chlorophenyl)methyl]phenyl}-1,3,4-oxadiazol-2(3*H*)-one, 3-[(1*S*)-1-(1-{(*S*)-(4-chlorophenyl)[3-(5-oxo-4,5-dihydro-1,3,4-oxadiazol-2-yl)phenyl]methyl} azetidin-3-yl)-2-fluoro-2-methylpropyl]-5-fluorobenzonitrile, 3-[(1*S*)-1-(1-{(*S*)-(4-cyanophenyl)[3-(5-oxo-4,5-dihydro-1,3,4-oxadiazol-2-yl)phenyl]methyl} azetidin-3-yl)-2-fluoro-2-methylpropyl]-5-fluorobenzonitrile, 3-[(1*S*)-1-(1-{(*S*)-(4-cyanophenyl)[3-(1,3,4-oxadiazol-2-yl)phenyl]methyl}azetidin-3-yl)-2-fluoro-2-methylpropyl]-5-fluorobenzonitrile, 3-[(1*S*)-1-(1-{(*S*)-(4-chlorophenyl)[3-(1,3,4-oxadiazol-2-yl)phenyl]methyl}azetidin-3-yl)-2-fluoro-2-methylpropyl]-5-fluorobenzonitrile, 3-((1*S*)-1-{1-[(*S*)-[3-(5-amino-1,3,4-oxadiazol-2-yl)phenyl](4-chlorophenyl)methyl]azetidin-3-yl}-2-fluoro-2-methylpropyl)-5-fluorobenzonitrile, 3-((1*S*)-1-{1-[(*S*)-[3-(5-amino-1,3,4-oxadiazol-2-yl)phenyl](4-cyanophenyl)methyl]azetidin-3-yl}-2-fluoro-2-methylpropyl)-5-fluorobenzonitrile, 3-[(1*S*)-1-(1-{(*S*)-(4-cyanophenyl)[3-(1,2,4-oxadiazol-3-yl)phenyl]methyl}azetidin-3-yl)-2-fluoro-2-methylpropyl]-5-fluorobenzonitrile, 3-[(1*S*)-1-(1-{(*S*)-(4-chlorophenyl)[3-(1,2,4-oxadiazol-3-yl)phenyl]methyl}azetidin-3-yl)-2-fluoro-2-methylpropyl]-5-fluorobenzonitrile, 5-[3-((*S*)-(4-chlorophenyl){3-[(1*S*)-1-(3,5-difluorophenyl)-2-fluoro-2-methylpropyl] azetidin-1-yl}methyl)phenyl]-1,3,4-oxadiazol-2(3H)-one, 5-[3-((*S*)-(4-chlorophenyl) {3-[(1*S*)-1-(3,5-difluorophenyl)-2-fluoro-2-methylpropyl]azetidin-1-yl}methyl)phenyl]-1,3,4-oxadiazol-2(3H)-one, 4-{(*S*)-{3-[(1*S*)-1-(3,5-difluorophenyl)-2-fluoro-2-methylpropyl]azetidin-1-yl}[3-(5-oxo-4,5-dihydro-1,3,4-oxadiazol-2-yl)phenyl]methyl}-benzonitrile, and pharmaceutically acceptable salts thereof.

Specific NPY5 antagonists of use in combination with a compound of the present invention include: 3-oxo-N-(5-phenyl-2-pyrazinyl)-spiro[isobenzofuran-1(3H),4'-piperidine]-1'-carboxamide, 3-oxo-N-(7-trifluoromethylpyrido[3,2-b]pyridin-2-yl)spiro-[isobenzofuran-1(3H),4'-piperidine]-1'-carboxamide, N-[5-(3-fluorophenyl)-2-pyrimidinyl]-3-oxospiro-[isobenzofuran-1 (3H),4'-piperidine]-1'-carboxamide, trans-3'-oxo-N-(5-phenyl-2-pyrimidinyl)spiro[cyclohexane-1,1'(3'H)-isobenzofuran]-4-carboxamide, trans-3'-oxo-N-[1-(3-quinolyl)-4-imidazolyl]spiro[cyclohexane-1,1'(3'H)-isobenzofuran]-4-carboxamide, trans-3-oxoN-(5-phenyl-2-pyrazinyl)spiro[4-azaiso-benzofuran- 1(3H),1'-cyclohexane]-4'-carboxamide, trans-N-[5-(3-fluorophenyl)-2-pyrimidinyl]-3-oxospiro[5-azaisobenzofuran-1(3H),1'-cyclohexane]-4'-carboxamide, trans-N-[5-(2-fluorophenyl)-2-pyrimidinyl]-3-oxospiro[5-azaisobenzofuran-1(3H),1'-cyclohexane]-4'-carboxamide, trans-N-[1-(3,5-difluorophenyl)-4-imidazolyl]-3-oxospiro[7-azaisobenzofuran-1(3H),1'-cyclohexane]-4'-carboxamide, trans-3-oxoN-(1-phenyl-4-pyrazolyl)spiro[4-azaisobenzofuran-1(3H),1'-cyclohexane]-4'-carboxamide, trans-N-[1-(2-fluorophenyl)-3-pyrazolyl]-3-oxospiro[6-azaisobenzofuran-1(3H),1'-cyclohexane]-4'-carboxamide, trans-3-oxo-N-(1-phenyl-3-pyrazolyl)spiro[6-azaisobenzofuran-1(3H),1'-cyclohexane]-4'-carboxamide, trans-3-oxo-N-(2-phenyl-1,2,3-triazol-4-yl)spiro[6-azaisobenzofuran-1(3H),1'-cyclohexane]-4'-carboxamide, and pharmaceutically acceptable salts and esters thereof.

Specific ACC-1/2 inhibitors of use in combination with a compound of the present invention include: 1'-[(4,8-dimethoxyquinolin-2-yl)carbonyl]-6-(1*H*-tetrazol-5-yl)spiro[chroman-2,4'-piperidin]-4-one; (5-{1'-[(4,8-dimethoxyquinolin-2-yl)carbonyl]-4-oxospiro[chroman-2,4'-piperidin]-6-yl}-2*H-*tetrazol-2-yl)methyl pivalate; 5-{1'-[(8-cyclopropyl-4-methoxyquinolin-2-yl)carbonyl]-4-oxospiro[chroman-2,4'-piperidin]-6-yl}nicotinic acid; 1'-(8-methoxy-4-morpholin-4-yl-2-naphthoyl)-6-(1*H-*tetrazol-5-yl)spiro[chroman-2,4'-piperidin]-4-one; and 1'-[(4-ethoxy-8-ethylquinolin-2-yl)carbonyl]-6-(1*H-*tetrazol-5-yl)spiro[chroman-2,4'-piperidin]-4-one; and pharmaceutically acceptable salts and esters thereof.

Specific MCH1R antagonist compounds of use in combination with a compound of the persent invention include: 1-{4-[(1-ethylazetidin-3-yl)oxy]phenyl}-4-[(4-fluorobenzyl)oxy]pyridin-2(1*H*)-one, 4-[(4-fluorobenzyl)oxy]-1-{4-[(1-isopropylazetidin-3-yl)oxy]phenyl}pyridin-2(1*H*)-one, 1-[4-(azetidin- 3-yloxy)phenyl]-4-[(5-chloropyridin-2-yl)methoxy]pyridin-2(1*H-*one, 4-[(5-chloropyridin-2-yl)methoxy]-1-{4-[(1-ethylazetidin-3-yl)oxy]phenyl}pyridin-2(1*H-*one, 4-[(5-chloropyridin-2-yl)methoxy]-1-{4-[(1-propylazetidin-3-yl)oxy]phenyl}pyridin-2(1*H*)-one, and 4-[(5-chloropyridin-2-yl)methoxy]-1-(4-{[(2*S*)-1-ethylazetidin-2-yl]methoxy}phenyl)pyridin-2(1*H*)-one, or a pharmaceutically acceptable salt thereof.

Specific DP-IV inhibitors of use in combination with a compound of the present invention are selected from 7-[(3R)-3-amino-4-(2,4,5-trifluorophenyl)butanoyl]-3-(trifluoromethyl)-5,6,7,8-tetrahydro-1,2,4-triazolo[4,3-a]pyrazine. In particular, the compound of formula I is favorably combined with 7-[(3R)-3-amino-4-(2,4,5-trifluorophenyl)butanoyl]-3-(trifluoromethyl)-5,6,7,8-tetrahydro-1,2,4-triazolo[4,3-a]pyrazine, and pharmaceutically acceptable salts thereof.

Specific H3 (histamine H3) antagonists/inverse agonists of use in combination with a compound of the present invention include: those described in WO05/077905, including:3-{4-[(1-cyclobutyl-4-piperidinyl)oxy]phenyl}-2-ethylpyrido[2,3-d]-pyrimidin-4(3H)-one, 3-{4-[(1-cyclobutyl-4-piperidinyl)oxy]phenyl}-2-methylpyrido[4,3-d]pyrimidin-4(3H)-one, 2-ethyl-3-(4-{3-[(3S)-3-methylpiperidin-1-yl]propoxy}phenyl)pyrido[2,3-d]pyrimidin-4(3H)-one 2-methyl-3-(4-{3-[(3S)-3-methylpiperidin-1-yl]propoxy}phenyl)pyrido[4,3-d]pyrimidin-4(3H)-one, 3-{4-[(1-cyclobutyl-4-piperidinyl)oxy]phenyl}-2,5-dimethyl-4(3H)-quinazolinone, 3-{4-[(1-cyclobutyl-4-piperidinyl)oxy]phenyl}-2-methyl-5-trifluoromethyl-4(3H)-quinazolinone, 3-{4-[(1-cyclobutyl-4-piperidinyl)oxy]phenyl}-5-methoxy-2-methyl-4(3H)-quinazolinone, 3-{4-[(1-cyclobutylpiperidin-4-yl)oxy]phenyl}-5-fluoro-2-methyl-4(3H)-quinazolinone, 3-{4-[(1-cyclobutylpiperidin-4-yl)oxy]phenyl}-7-fluoro-2-methyl-4(3H)-quinazolinone, 3-{4-[(1-cyclobutylpiperidin-4-yl)oxy]phenyl}-6-methoxy-2-methyl-4(3H)-quinazolinone, 3-{4-[(1-cyclobutylpiperidin-4-yl)oxy]phenyl}-6-fluoro-2-methyl-4(3H)-quinazolinone, 3-{4-[(1-cyclobutylpiperidin-4-yl)oxy]phenyl}-8-fluoro-2-methyl-4(3H)-quinazolinone, 3-{4-[(1-cyclopentyl-4-piperidinyl)oxy]phenyl}-2-methylpyrido[4,3-d]pyrimidin-4(3H)-one, 3-{4-[(1-cyclobutylpiperidin-4-yl)oxy]phenyl}-6-fluoro-2-methylpyrido[3,4-d]pyrimidin-4(3H)-one, 3-{4-[(1-cyclobutyl-4-piperidinyl)oxy]phenyl}-2-ethylpyrido[4,3-d]pyrimidin-4(3H)-one, 6-methoxy-2-methyl-3-{4-[3-(1-piperidinyl)propoxy]phenyl}pyrido[3,4-d]pyrimidin-4(3H)-one, 6-methoxy-2-methyl-3-{4-[3-(1-pyrrolidinyl)propoxy]phenyl}pyrido[3,4-d]pyrimidin-4(3H)-one, 2,5-dimethyl-3-{4-[3-(1-pyrrolidinyl)propoxy]phenyl}-4(3H)-quinazolinone, 2-methyl-3-{4-[3-(1-pyrrolidinyl)propoxy]phenyl}-5-trifluoromethyl-4(3H)-quinazolinone, 5-fluoro-2-methyl-3-{4-[3-(1-piperidinyl)propoxy]phenyl}-4(3H)-quinazolinone, 6-methoxy-2-methyl-3-{4-[3-(1-piperidinyl)propoxy]phenyl}-4(3H)-quinazolinone, 5-methoxy-2-methyl-3-(4-{3-[(3S)-3-methylpiperidin-1-yl]propoxy}phenyl)-4(3H)-quinazolinone, 7-methoxy-2-methyl-3-(4-{3-[(3S)-3-methylpiperidin-1-yl]propoxy}phenyl)-4(3H)-quinazolinone, 2-methyl-3-(4-{3-[(3S)-3-methylpiperidin-1-yl]propoxy}phenyl)pyrido[2,3-d]pyrimidin-4(3H)-one, 5-fluoro-2-methyl-3-(4-{3-[(2R)-2-methylpyrrolidin-1-yl]propoxy}phenyl)-4(3H)-quinazolinone, 2-methyl-3-(4-{3-[(2R)-2-methylpyrrolidin-1-yl]propoxy}phenyl)pyrido[4,3-d]pyrimidin-4(3H)-one, 6-methoxy-2-methyl-3-(4-{3-[(2R)-2-methylpyrrolidin-1-yl]propoxy}phenyl)-4(3H)-quinazolinone, 6-methoxy-2-methyl-3-(4-{3-[(2S)-2-methylpyrrolidin-1-yl]propoxy}phenyl)-4(3H)-quinazolinone, and pharmaceutically acceptable salts thereof.

Specific CCK1R agonists of use in combination with a compound of the present invention include: 3-(4-{[1-(3-ethoxyphenyl)-2-(4-methylphenyl)-1*H-*imidazol-4-yl]carbonyl}-1-piperazinyl)-1-naphihoic acid; 3-(4-{[1-(3-ethoxyphenyl)-2-(2-fluoro-4-methylphenyl)-1*H-*imidazol-4-yl]carbonyl}-1-piperazinyl)-1-naphthoic acid; 3-(4-{[1-(3-ethoxyphenyl)-2-(4-fluorophenyl)-1*H-*imidazol-4-yl]carbonyl}-1-piperazinyl)-1-naphthoic acid; 3-(4-{[1-(3-ethoxyphenyl)-2-(2,4-difluorophenyl)-1*H-*imidazol-4-yl]carbonyl}-1-piperazinyl)-1-naphthoic acid; and 3-(4-{[1-(2,3-dihydro-1,4-benzodioxin-6-yl)-2-(4-fluorophenyl)-1*H-*imidazol-4-yl]carbonyl}-1-piperazinyl)-1-naphthoic acid; and pharmaceutically acceptable salts thereof.

Specific MC4R agonists of use in combination with a compound of the present invention include: 1) (5*S*)-1'-{[(3*R,*4*R*)-1-*tert*-butyl-3-(2,3,4-trifluorophenyl)piperidin-4-yl]carbonyl}-3-chloro-2-methyl-5-[1-methyl-1-(1-methyl-1*H-*1,2,4-triazol-5-yl)ethyl]-5*H-*spiro[furo[3,4-*b*]pyridine-7,4'-piperidine]; 2)(5*R*)-1'-{[(3*R*,4*R*)-1-*tert*-butyl-3-(2,3,4-trifluorophenyl)-piperidin-4-yl]carbonyl-3-chloro-2-methyl-5-[1-methyl-1-(1-methyl-1*H-*1,2,4-triazol-5-yl)ethyl]-5*H-*spiro[furo[3,4-*b*]pyridine-7,4'-piperidine]; 3)2-(1'-{[(3*S*,4*R*)-1-*tert*-butyl-4-(2,4-difluorophenyl)pyrrolidin-3-yl]carbonyl}-3-chloro-2-methyl-5*H-*spiro[furo[3,4-*b*]pyridine-7,4'-piperidin]-5-yl)-2-methylpropanenitrile; 4) 1'-{[(3*S*,4*R*)-1-*tert*-butyl-4-(2,4-difluorophenyl)pyrrolidin-3-yl]carbonyl}-3-chloro-2-methyl-5-[1-methyl-1-(1-methyl-1*H-*1,2,4-triazol-5-yl)ethyl]-5*H-*spiro[furo[3,4-*b*]pyridine-7,4'-piperidine]; 5) *N-*[(3*R*,4*R*)-3-({3-chloro-2-methyl-5-[1-methyl-1-(1-methyl-1*H-*1,2,4-triazol-5-yl)ethyl]-1'*H,*5*H-*spiro[furo-[3,4-*b*]pyridine-7,4'-piperidin]-1'-yl}carbonyl)-4-(2,4-difluorophenyl)-cyclopentyl]-*N*-methyltetrahydro-2*H-*pyran-4-amine; 6) 2-[3-chloro-1-({(1*R*,2*R*)-2-(2,4-difluorophenyl)-4-[methyl(tetrahydro-2*H-*pyran-4-yl)amino]-cyclopentyl}-carbonyl)-2-methyl-5*H-*spiro[furo[3,4-*b*]pyridine-7,4'-piperidin]-5-yl]-2-methyl-propane-nitrile; and pharmaceutically acceptable salts thereof.

"Obesity" is a condition in which there is an excess of body fat. The operational definition of obesity is based on the Body Mass Index (BMI), calculated as body weight per height in meters squared (kg/m²). "Obesity" refers to a condition whereby an otherwise healthy subject has a Body Mass Index (BMI) greater than or equal to 30 kg/m², or a condition whereby a subject with at least one co-morbidity has a BMI greater than or equal to 27 kg/m². An "obese subject" is an otherwise healthy subject with a Body Mass Index (BMI) greater than or equal to 30 kg/m² or a subject with at least one co-morbidity with a BMI greater than or equal to 27 kglm². A "subject at risk for obesity" is an otherwise healthy subject with a BMI of 25 kg/m² to less than 30 kg/m² or a subject with at least one co-morbidity with a BMI of 25 kg/m² to less than 27 kg/m².

The increased risks associated with obesity occur at a lower Body Mass Index (BMI) in Asians. In Asian countries, including Japan, "obesity" refers to a condition whereby a subject with at least one obesity-induced or obesity-related co-morbidity that requires weight reduction or that would be improved by weight reduction, has a BMI greater than or equal to 25 kg/m². In Asian countries, including Japan, an "obese subject" refers to a subject with at least one obesity-induced or obesity-related co-morbidity that requires weight reduction or that would be improved by weight reduction, with a BMI greater than or equal to 25 kg/m². In Asian countries, a "subject at risk of obesity" is a subject with a BMI of greater than 23 kg/m² to less than 25 kg/m².

As used herein, the term "obesity" is meant to encompass all of the above definitions of obesity.

Obesity-induced or obesity-related co-morbidities include, but are not limited to, diabetes, non-insulin dependent diabetes mellitus - type 2, impaired glucose tolerance, impaired fasting glucose, insulin resistance syndrome, dyslipidemia, hypertension, hyperuricacidemia, gout, coronary artery disease, myocardial infarction, angina pectoris, sleep apnea syndrome, Pickwickian syndrome, fatty liver; cerebral infarction, cerebral thrombosis, transient ischemic attack, orthopedic disorders, arthritis deformans, lumbodynia, emmeniopathy, and infertility. In particular, co-morbidities include: hypertension, hyperlipidemia, dyslipidemia, glucose intolerance, cardiovascular disease, sleep apnea, diabetes mellitus, and other obesity-related conditions.

"Treatment" (of obesity and obesity-related disorders) refers to the administration of the compounds of the present invention to reduce or maintain the body weight of an obese subject. One outcome of treatment may be reducing the body weight of an obese subject relative to that subject's body weight immediately before the administration of the compounds of the present invention. Another outcome of treatment may be preventing body weight regain of body weight previously lost as a result of diet, exercise, or pharmacotherapy. Another outcome of treatment may be decreasing the occurrence of and/or the severity of obesity-related diseases. The treatment may suitably result in a reduction in food or calorie intake by the subject, including a reduction in total food intake, or a reduction of intake of specific components of the diet such as carbohydrates or fats; and/or the inhibition of nutrient absorption; and/or the inhibition of the reduction of metabolic rate; and in weight reduction in patients in need thereof. The treatment may also result in an alteration of metabolic rate, such as an increase in metabolic rate, rather than or in addition to an inhibition of the reduction of metabolic rate; and/or in minimization of the metabolic resistance that normally results from weight loss.

"Prevention" (of obesity and obesity-related disorders) refers to the administration of the compounds of the present invention to reduce or maintain the body weight of a subject at risk of obesity. One outcome of prevention may be reducing the body weight of a subject at risk of obesity relative to that subject's body weight immediately before the administration of the compounds of the present invention. Another outcome of prevention may be preventing body weight regain of body weight previously lost as a result of diet, exercise, or pharmacotherapy. Another outcome of prevention may be preventing obesity from occurring if the treatment is administered prior to the onset of obesity in a subject at risk of obesity. Another outcome of prevention may be decreasing the occurrence and/or severity of obesity-related disorders if the treatment is administered prior to the onset of obesity in a subject at risk of obesity. Moreover, if treatment is commenced in already obese subjects, such treatment may prevent the occurrence, progression or severity of obesity-related disorders, such as, but not limited to, arteriosclerosis, Type II diabetes, polycystic ovarian disease, cardiovascular diseases, osteoarthritis, dermatological disorders, hypertension, insulin resistance, hypercholesterolemia, hypertriglyceridemia, and cholelithiasis.

The obesity-related disorders herein are associated with, caused by, or result from obesity. Examples of obesity-related disorders include overeating and bulimia, hypertension, diabetes, elevated plasma insulin concentrations and insulin resistance, dyslipidemias, hyperlipidemia, endometrial, breast, prostate and colon cancer, osteoarthritis, obstructive sleep apnea, cholelithiasis; gallstones, heart disease, abnormal heart rhythms and arrythmias, myocardial infarction, congestive heart failure, coronary heart disease, sudden death, stroke, polycystic ovarian disease, craniopharyngioma, the Prader-Willi Syndrome, Frohlich's syndrome, GH-deficient subjects, normal variant short stature, Turner's syndrome, and other pathological conditions showing reduced metabolic activity or a decrease in resting energy expenditure as a percentage of total fat-free mass, e.g, children with acute lymphoblastic leukemia. Further examples of obesity-related disorders are metabolic syndrome, also known as syndrome X, insulin resistance syndrome, sexual and reproductive dysfunction, such as infertility, hypogonadism in males and hirsutism in females, gastrointestinal motility disorders, such as obesity-related gastro-esophageal reflux, respiratory disorders, such as obesity-hypoventilation syndrome (Pickwickian syndrome), cardiovascular disorders, inflammation, such as systemic inflammation of the vasculature, arteriosclerosis, hypercholesterolemia, hyperuricaemia, lower back pain, gallbladder disease, gout, and kidney cancer. The compounds of the present invention are also useful for reducing the risk of secondary outcomes of obesity, such as reducing the risk of left ventricular hypertrophy.

The compounds of formula I are also useful for treating or preventing obesity and obesity-related disorders in cats and dogs. As such, the term "mammal" includes companion animals such as cats and dogs.

The term "diabetes," as used herein, includes both insulin-dependent diabetes mellitus (IDDM, also known as type I diabetes) and non-insulin-dependent diabetes mellitus (NIDDM, also known as Type II diabetes). Type I diabetes, or insulin-dependent diabetes, is the result of an absolute deficiency of insulin, the hormone which regulates glucose utilization. Type II diabetes, or insulin-independent diabetes (i.e., non-insulin-dependent diabetes mellitus), often occurs in the face of normal, or even elevated levels of insulin and appears to be the result of the inability of tissues to respond appropriately to insulin. Most of the Type II diabetics are also obese. The compounds of the present invention are useful for treating both Type I and Type II diabetes. The compounds are especially effective for treating Type II diabetes. The compounds of the present invention are also useful for treating and/or preventing gestational diabetes mellitus.

It will be appreciated that for the treatment or prevention of migraine, a compound of the present invention may be used in conjunction with other anti-migraine agents, such as ergotamines or 5-HT₁ agonists, especially sumatriptan, naratriptan, zolmatriptan or rizatriptan.

It will be appreciated that for the treatment of depression or anxiety, a compound of the present invention may be used in conjunction with other anti-depressant or anti-anxiety agents.

Suitable classes of anti-depressant agents include norepinephrine reuptake inhibitors, selective serotonin reuptake inhibitors (SSRIs), monoamine oxidase inhibitors (MAOIs), reversible inhibitors of monoamine oxidase (RIMAs), serotonin and noradrenaline reuptake inhibitors (SNRIs), corticotropin releasing factor (CRF) antagonists, α-adrenoreceptor antagonists, neurokinin-1 receptor antagonists and atypical anti-depressants.

Suitable norepinephrine reuptake inhibitors include tertiary amine tricyclics and secondary amine tricyclics. Suitable examples of tertiary amine tricyclics include: amitriptyline, clomipramine, doxepin, imipramine and trimipramine, and pharmaceutically acceptable salts thereof. Suitable examples of secondary amine tricyclics include: amoxapine, desipramine, maprotiline, nortriptyline and protriptyline, and pharmaceutically acceptable salts thereof.

Suitable selective serotonin reuptake inhibitors include: fluoxetine, fluvoxamine, paroxetine, imipramine and sertraline, and pharmaceutically acceptable salts thereof.

Suitable monoamine oxidase inhibitors include: isocarboxazid, phenelzine, tranylcypromine and selegiline, and pharmaceutically acceptable salts thereof.

Suitable reversible inhibitors of monoamine oxidase include: moclobemide, and pharmaceutically acceptable salts thereof.

Suitable serotonin and noradrenaline reuptake inhibitors of use in the present invention include: venlafaxine, and pharmaceutically acceptable salts thereof.

Suitable CRF antagonists include those compounds described in International Patent Specification Nos. WO 94/13643, 94/13644, 94/13661, 94/13676 and 94/13677. Still further, neurokinin-1 (NK-1) receptor antagonists may be favorably employed with the CB1 receptor modulators of the present invention. NK-1 receptor antagonists of use in the present invention are fully described in the art. Specific neurokinin-1 receptor antagonists of use in the present invention include: (±)-(2R3R,2S3S)-N-{[2-cyclopropoxy-5-(trifluoromethoxy)-phenyl]methyl}-2-phenylpiperidin-3-amine; 2-(R)-(1-(R)-(3,5-bis(trifluoromethyl)phenyl)ethoxy)-3-(S)-(4-fluorophenyl)-4-(3-(5-oxo-1*H*,4*H-*1,2,4-triazolo)methyl)morpholine; aperpitant; CJ17493; GW597599; GW679769; R673; RO67319; R1124; R1204; SSR146977; SSR240600; T-2328; and T2763.; or a pharmaceutically acceptable salts thereof.

Suitable atypical anti-depressants include: bupropion, lithium, nefazodone, trazodone and viloxazine, and pharmaceutically acceptable salts thereof.

Suitable classes of anti-anxiety agents include benzodiazepines and 5-HT_{1A} agonists or antagonists, especially 5-HT_{1A} partial agonists, and corticotropin releasing factor (CRF) antagonists. Suitable benzodiazepines include: alprazolam, chlordiazepoxide, clonazepam, chlorazepate, diazepam, halazepam, lorazepam, oxazepam and prazepam, and pharmaceutically acceptable salts thereof. Suitable 5-HT_{1A} receptor agonists or antagonists include, in particular, the 5-HT_{1A} receptor partial agonists buspirone, flesinoxan, gepirone and ipsapirone, and pharmaceutically acceptable salts thereof. Suitable corticotropin releasing factor (CRF) antagonists include those previously discussed herein.

As used herein, the term "substance abuse disorders" includes substance dependence or abuse with or without physiological dependence. The substances associated with these disorders are: alcohol, amphetamines (or amphetamine-like substances), caffeine, cannabis, cocaine, hallucinogens, inhalants, marijuana, nicotine, opioids, phencyclidine (or phencyclidine-like compounds), sedative-hypnotics or benzodiazepines, and other (or unknown) substances and combinations of all of the above.

In particular, the term "substance abuse disorders" includes drug withdrawal disorders such as alcohol withdrawal with or without perceptual disturbances; alcohol withdrawal delirium; amphetamine withdrawal; cocaine withdrawal; nicotine withdrawal; opioid withdrawal; sedative, hypnotic or anxiolytic withdrawal with or without perceptual disturbances; sedative, hypnotic or anxiolytic withdrawal delirium; and withdrawal symptoms due to other substances.

It will be appreciated that reference to treatment of nicotine withdrawal includes the treatment of symptoms associated with smoking cessation.

Other "substance abuse disorders" include substance-induced anxiety disorder with onset during withdrawal; substance-induced mood disorder with onset during withdrawal; and substance-induced sleep disorder with onset during withdrawal.

In particular, compounds of structural formula I are useful for aiding in stopping consumption of tobacco and are useful in treating nicotine dependence and nicotine withdrawal. The compounds of formula I produce in consumers of nicotine, such as tobacco smokers, a total or partial abstinence from smoking. Further, withdrawal symptoms are lessened and the weight gain that generally accompanies quitting tobacco comsumption is reduced or nonexistent. For smoking cessation, the compound of form I may be used in combination with a nicotine agonist or a partial nicotine agonist, including varenicline and selective alpha-4 beta 2 nicotinic partial agonists such as SSR 591813, or a monoamine oxidase inhibitor (MAOI), or another active ingredient demonstrating efficacy in aiding cessation of tobacco consumption; for example, an antidepressant such as bupropion, doxepine, omortriptyline; or an anxiolytic such as buspirone or clonidine.

It will be appreciated that a combination of a conventional antipsychotic drug with a CB1 receptor modulator may provide an enhanced effect in the treatment of mania. Such a combination would be expected to provide for a rapid onset of action to treat a manic episode thereby enabling prescription on an "as needed basis". Furthermore, such a combination may enable a lower dose of the antispychotic agent to be used without compromising the efficacy of the antipsychotic agent, thereby minimizing the risk of adverse side-effects. A yet further advantage of such a combination is that, due to the action of the CB1 receptor modulator, adverse side-effects caused by the antipsychotic agent such as acute dystonias, dyskinesias, akathesia and tremor may be reduced or prevented.

Thus, according to a further aspect of the present invention there is provided the use of a CB1 receptor modulator and an antipsychotic agent for the manufacture of a medicament for the treatment or prevention of mania.

The present invention also provides a compound for use in a method for the treatment or prevention of mania, which method comprises administration to a patient in need of such treatment or at risk of developing mania of an amount of a CB1 receptor modulator and an amount of an antipsychotic agent, such that together they give effective relief.

In a further aspect of the present invention, there is provided a pharmaceutical composition comprising a CB1 receptor modulator and an antipsychotic agent, together with at least one pharmaceutically acceptable carrier or excipient, wherein the CB1 receptor modulator and the antipsychotic agent may be present as a combined preparation for simultaneous, separate or sequential use for the treatment or prevention of mania. Such combined preparations may be, for example, in the form of a twin pack.

In a further or alternative aspect of the present invention, there is therefore provided a product comprising a CB1 receptor modulator and an antipsychotic agent as a combined preparation for simultaneous, separate or sequential use in the treatment or prevention of mania.

It will be appreciated that when using a combination of the present invention, the CB1 receptor modulator and the antipsychotic agent may be in the same pharmaceutically acceptable carrier and therefore administered simultaneously. They may be in separate pharmaceutical carriers such as conventional oral dosage forms which are taken simultaneously. The term "combination" also refers to the case where the compounds are provided in separate dosage forms and are administered sequentially. Therefore, by way of example, the antipsychotic agent may be administered as a tablet and then, within a reasonable period of time, the CB1 receptor modulator may be administered either as an oral dosage form such as a tablet or a fast-dissolving oral dosage form. By a "fast-dissolving oral formulation" is meant, an oral delivery form which when placed on the tongue of a patient, dissolves within about 10 seconds.

CB1 receptor modulators can be used in combination with an antipsychotic agent in the treatment or prevention of hypomania.

It will be appreciated that a combination of a conventional antipsychotic drug with a CB1 receptor modulator may provide an enhanced effect in the treatment of schizophrenic disorders. Such a combination would be expected to provide for a rapid onset of action to treat schizophrenic symptoms thereby enabling prescription on an "as needed basis". Furthermore, such a combination may enable a lower dose of the CNS agent to be used without compromising the efficacy of the antipsychotic agent, thereby minimizing the risk of adverse side-effects. A yet further advantage of such a combination is that, due to the action of the CB1 receptor modulator, adverse side-effects caused by the antipsychotic agent such as acute dystonias, dyskinesias, akathesia and tremor may be reduced or prevented.

As used herein, the term "schizophrenic disorders" includes paranoid, disorganized, catatonic, undifferentiated and residual schizophrenia; schizophreniform disorder; schizoaffective disorder; delusional disorder; brief psychotic disorder; shared psychotic disorder; substance-induced psychotic disorder; and psychotic disorder not otherwise specified.

Other conditions commonly associated with schizophrenic disorders include self-injurious behavior (e.g. Lesch-Nyhan syndrome) and suicidal gestures.

Suitable antipsychotic agents of use in combination with a CB1 receptor modulator include the phenothiazine, thioxanthene, heterocyclic dibenzazepine, butyrophenone, diphenylbutylpiperidine and indolone classes of antipsychotic agent. Suitable examples of phenothiazines include chlorpromazine, mesoridazine, thioridazine, acetophenazine, fluphenazine, perphenazine and trifluoperazine. Suitable examples of thioxanthenes include chlorprothixene and thiothixene. Suitable examples of dibenzazepines include clozapine and olanzapine. An example of a butyrophenone is haloperidol. An example of a diphenylbutylpiperidine is pimozide. An example of an indolone is molindolone. Other antipsychotic agents include loxapine, sulpiride and risperidone. It will be appreciated that the antipsychotic agents when used in combination with a CB1 receptor modulator may be in the form of a pharmaceutically acceptable salt, for example, chlorpromazine hydrochloride, mesoridazine besylate, thioridazine hydrochloride, acetophenazine maleate, fluphenazine hydrochloride, flurphenazine enathate, fluphenazine decanoate, trifluoperazine hydrochloride, thiothixene hydrochloride, haloperidol decanoate, loxapine succinate and molindone hydrochloride. Perphenazine, chlorprothixene, clozapine, olanzapine, haloperidol, pimozide and risperidone are commonly used in a non-salt form.

Other classes of antipsychotic agent of use in combination with a CB1 receptor modulator include dopamine receptor antagonists, especially D2, D3 and D4 dopamine receptor antagonists, and muscarinic ml receptor agonists. An example of a D3 dopamine receptor antagonist is the compound PNU-99194A. An example of a D4 dopamine receptor antagonist is PNU-101387. An example of a muscarinic ml receptor agonist is xanomeline.

Another class of antipsychotic agent of use in combination with a CB1 receptor modulator is the 5-HT_{2A} receptor antagonists, examples of which include MDL100907 and fananserin. Also of use in combination with a CB1 receptor modulator are the serotonin dopamine antagonists (SDAs) which are believed to combine 5-HT_{2A} and dopamine receptor antagonist activity, examples of which include olanzapine and ziperasidone.

Still further, NK-1 receptor antagonists may be favorably employed with the CB1 receptor modulators of the present invention. Preferred NK-1 receptor antagonists for use in the present invention are selected from the classes of compounds described previously.

It will be appreciated that a combination of a conventional anti-asthmatic drug with a CB1 receptor modulator may provide an enhanced effect in the treatment of asthma, and may be used for the treatment or prevention of asthma, which method comprises administration to a patient in need of such treatment an amount of a compound of the present invention and an amount of an anti-asthmatic agent, such that together they give effective relief.

Suitable anti-asthmatic agents of use in combination with a compound of the present invention include, but are not limited to: (a) VLA-4 antagonists such as natalizumab and the compounds described in US 5,510,332, WO97/03094, WO97/02289, WO96/40781, WO96/22966, WO96/20216, WO96/01644, WO96/06108, WO95/15973 and WO96/31206; (b) steroids and corticosteroids such as beclomethasone, methylprednisolone, betamethasone, prednisone, dexamethasone, and hydrocortisone; (c) antihistamines (H1-histamine antagonists) such as bromopheniramine, chlorpheniramine, dexchlorpheniramine, triprolidine, clemastine, diphenhydramine, diphenylpyraline, tripelennamine, hydroxyzine, methdilazine, promethazine, trimeprazine, azatadine, cyproheptadine, antazoline, pheniramine pyrilamine, astemizole, terfenadine, loratadine, desloratadine, cetirizine, fexofenadine, descarboethoxyloratadine, and the like; (d) non-steroidal anti-asthmatics including β2-agonists (such as terbutaline, metaproterenol, fenoterol, isoetharine, albuterol, bitolterol, salmeterol, epinephrine, and pirbuterol), theophylline, cromolyn sodium, atropine, ipratropium bromide, leukotriene antagonists (such as zafirlukast, montelukast, pranlukast, iralukast, pobilukast, and SKB-106,203), and leukotriene biosynthesis inhibitors (such as zileuton and BAY-1005); (e) anti-cholinergic agents including muscarinic antagonists (such as ipratropium bromide and atropine); and (f) antagonists of the chemokine receptors, especially CCR-3; and pharmaceutically acceptable salts thereof.

It will be appreciated that a combination of a conventional anti-constipation drug with a CB1 receptor modulator may provide an enhanced effect in the treatment of constipation or chronic intestinal pseudo-obstruction, and for use for the manufacture of a medicament for the treatment or prevention of constipation or chronic intestinal pseudo-obstruction.

The present invention also provides a compound for use in a method for the treatment or prevention of constipation, which method comprises administration to a patient in need of such treatment an amount of a compound of the present invention and an amount of an anti-constipation agent, such that together they give effective relief.

Suitable anti-constipation agents of use in combination with a compound of the present invention include, but are not limited to, osmotic agents, laxatives and detergent laxatives (or wetting agents), bulking agents, and stimulants; and pharmaceutically acceptable salts thereof. A particularly suitable class of osmotic agents include, but are not limited to sorbitol, lactulose, polyethylene glycol, magnesium, phosphate,and sulfate; and pharmaceutically acceptable salts thereof. A particularly suitable class of laxatives and detergent laxatives, include, but are not limited to, magnesium, and docusate sodium; and pharmaceutically acceptable salts thereof. A particularly suitable class of bulking agents include, but are not limited to, psyllium, methylcellulose, and calcium polycarbophil; and pharmaceutically acceptable salts thereof. A particularly suitable class of stimulants include, but are not limited to, anthroquinones, and phenolphthalein; and pharmaceutically acceptable salts thereof.

It will be appreciated that a combination of a conventional anti-cirrhosis drug with a CB1 receptor modulator may provide an enhanced effect in the treatment or prevention of cirrhosis of the liver, and for use for the manufacture of a medicament for the treatment or prevention of cirrhosis of the liver, as well as non-alcoholic fatty liver disease (NAFLD) and non-alcoholic steatohepatitis (NASH).

The present invention also provides a compound for use in a method for the treatment or prevention of cirrhosis of the liver, which method comprises administration to a patient in need of such treatment an amount of a compound of the present invention and an anti-cirrhosis agent, such that together they give effective relief.

Suitable anti-cirrhosis agents of use in combination with a compound of the present invention include, but are not limited to, corticosteroids, penicillamine, colchicine, interferon-γ, 2-oxoglutarate analogs, prostaglandin analogs, and other anti-inflammatory drugs and antimetabolites such as azathioprine, methotrexate, leflunamide, indomethacin, naproxen, and 6-mercaptopurine; and pharmaceutically acceptable salts thereof.

The compound of this invention can be used in a method of modulating the CB1 receptor and treating CB1 receptor mediated diseases by administering to a patient in need of such treatment a non-toxic therapeutically effective amount of a compound of this invention that selectively antagonizes the CB1 receptor in preference to the other CB or G-protein coupled receptors. The present invention further comprises a compound for use in a method of treating or preventing a condition ameliorated by antagonism or inverse agonism of the CB1 receptor in a patient in need thereof comprising administration of a therapeutically effective amount of a compound of this invention. The present invention further comprises a compound for use in a method of treating or preventing obesity in a patient at risk for obesity comprising administration of about 0.01 mg to about 50 mg of a compound of this invention.

The term "therapeutically effective amount" means the amount the compound of structural formula I that will elicit the biological or medical response of a tissue, system, animal or human that is being sought by the researcher, veterinarian, medical doctor or other clinician, which includes alleviation of the symptoms of the disorder being treated. The novel methods of treatment of this invention are for disorders known to those skilled in the art. The term "mammal" includes humans, and companion animals such as dogs and cats.

The weight ratio of the compound of the Formula I to the second active ingredient may be varied and will depend upon the effective dose of each ingredient. Generally, an effective dose of each will be used. Thus, for example, when a compound of the Formula I is combined with a second active ingredient, the weight ratio of the compound of the Formula I to the second active ingredient will generally range from about 1000:1 1 to about 1:1000, preferably about 200:1 to about 1:200. Combinations of a compound of the Formula I and other active ingredients will generally also be within the aforementioned range, but in each case, an effective dose of each active ingredient should be used.

The following reaction schemes illustrate methods which may be employed for the synthesis of the novel furo[2,3*-b*]pyridines of structural formula **I** described in this invention. All substituents are as defined above unless indicated otherwise. Several strategies based upon synthetic transformations known in the literature of organic synthesis may be employed for the preparation of the title compounds of general formula **I**. A preferred synthetic process, shown retrosynthetically in reaction Scheme 1, proceeds through substituted 3-cyano-2-pyridones of general formula **2**. In some cases the corresponding 3-acyl-2-pyridones of general formula **3**, which are derived from the 3-cyano-2-pyridones of general formula **2**, are also intermediates in the synthesis depending on the selection of the R² substituent. The 3-cyano-2-pyridones of general formula **2** are in turn derived from 1,2-diphenylethanones of general formula **1**. Reaction Schemes 2-7 illustrate in detail the preferred methods for the synthesis of the title compounds of general formula **I** in the forward sense.

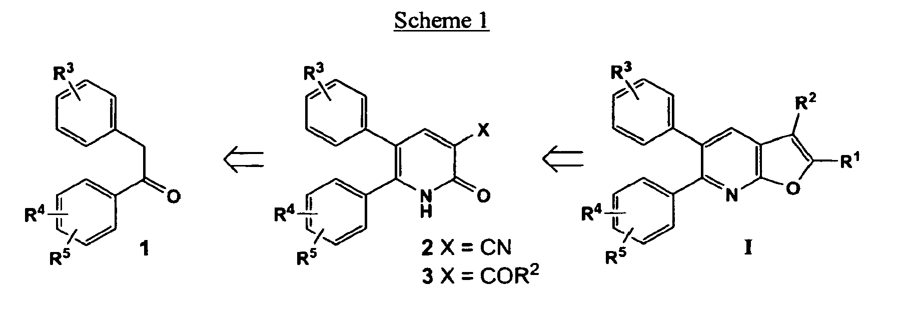

1,2-Diphenylethanones of general formula **1** may be available commercially or they can be synthesized using one of several methods known in the art of organic synthesis. Scheme 2 illustrates two methods for the synthesis of the 1,2-diphenylethanones of general formula **1**. In the first example (equation 1), a substituted benzyl bromide of general formula **4** is converted to a Grignard reagent with magnesium metal in a solvent such as THF at a temperature between room temperature and the refluxing temperature of the solvent. The resulting Grignard reagent is then added to a substituted benzonitrile of general formula **5**. Acidic hydrolysis of the reaction mixture followed by extraction of the organic product affords a 1,2-diphenylethanone of general formula **1** as shown. An alternative synthesis of 1,2-diphenylethanones **1** which is preferred when either of the phenyl groups are optionally substituted with functional groups that are reactive with Grignard reagents is shown at the bottom of reaction Scheme 2 (equation 2). Here a substituted phenylacetic acid of general formula **6** is reacted at low temperature (-78° to -50°C) with two equivalents of a strong base such as lithium bis(trimethylsilylamide) in an aprotic solvent such as THF. This doubly deprotonates the phenylacetic acid **5** and generates a dianion which undergoes a Dieckmann reaction when the substituted benzoate ester of general formula **7** is added. In this modification of the Dieckmann reaction, the intermediate β-keto acid smoothly decarboxylates and a 1,2-diphenylethanone of general formula **1** is produced. 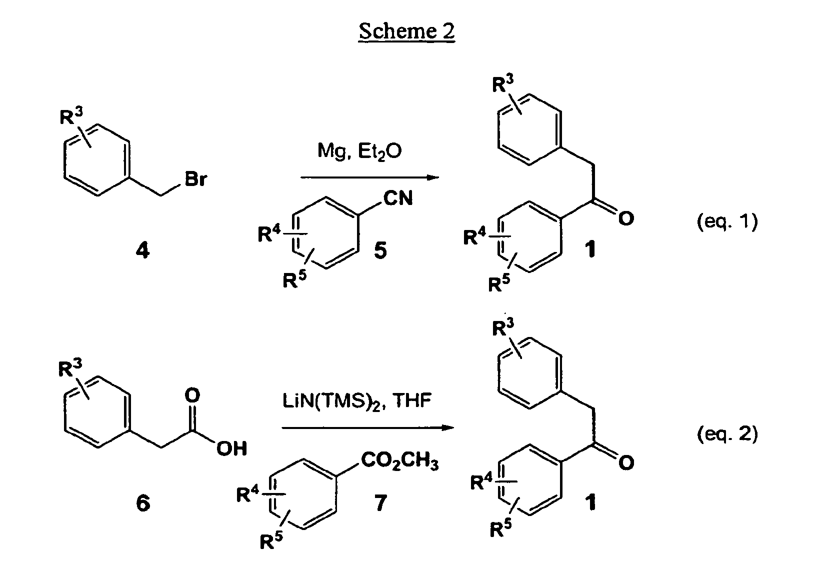 Reaction Scheme 3 illustrates a method for the conversion of the 1,2-diphenylethanone of general formula **1** into the 3-cyano-2-pyridones of general formula **2** and the corresponding **3-**acyl-2-pyridones of general formula **3**. A 1,2-diphenylethanone of general formula **1** is first converted to a vinylogous amide of general formula **8** by reaction with *N*,*N-*dimethylformamide dimethylacetal as shown. The condensation reaction is conducted in DMF using DMF acetal at an elevated temperature, typically between room temperature and 150°C, and the vinylogous amide **8** is produced as a mixture of *E* and *Z* diastereoisomers. In the second step of this sequence, the vinylogous amide **8** is condensed with cyanoacetamide to afford the 3-cyano-2-pyridone of general formula **2**. The reaction is usually conducted in a polar aprotic solvent such as DMF in the presence of a strong base such as an alkali metal hydride or alkoxide. When it is desired to prepare compounds of general formula **I** wherein the R² substituent is connected through a carbon-carbon bond to the 3-position of the furo[2,3-*b*]pyridine ring (e.g. R² = alkyl, aryl, acyl etc.), then the 3-cyano-2-pyridone of general formula **2** is next converted to a 3-acyl-2-pyridone of general formula **3** as shown in Reaction Scheme 3. This transformation is usually accomplished by addition of an organometallic reagent, such as a Grignard reagent, to the cyano group, followed by a hydrolytic workup. In this reaction, one equivalent of the organometallic reagent is consumed in deprotonation of the starting pyridone and a second equivalent is needed to add to the cyano group. A useful variant of this reaction involves the initial silylation of the 3-cyano-2-pyridone of general formula **2** with a silylating agent such as hexamethyldisilazane. The resulting silylated pyridone has considerably improved solubility and may then be reacted with one equivalent of the selected organometallic agent at lower temperatures.

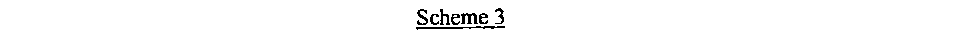

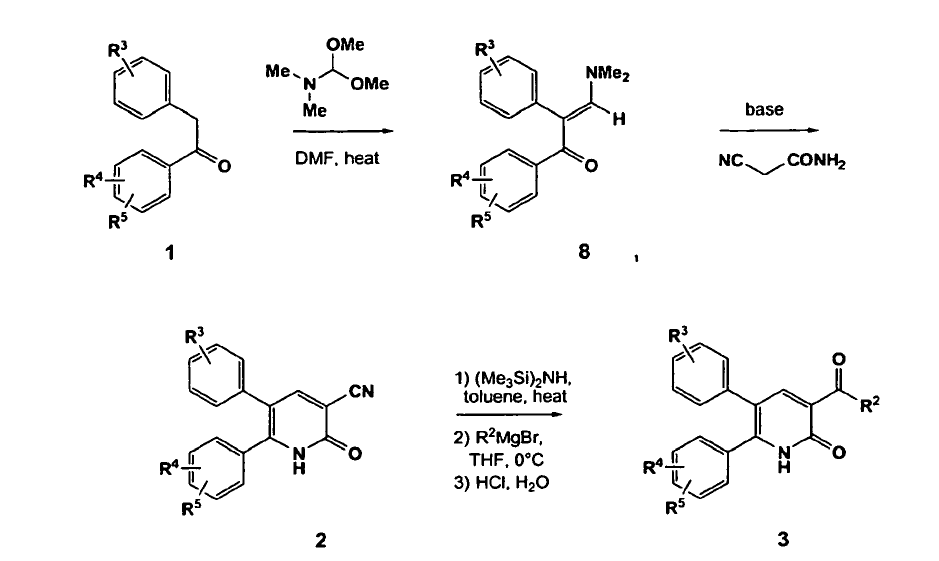

The next stage of the synthesis of the novel compounds of general formula **I** is illustrated in reaction Schemes 4 and 5 for intermediates **2** and **3** respectively. In reaction Scheme 4, the 3-cyano-2-pyridone of general formula **2** is subjected to an alkylation reaction with an electrophilic reagent of general formula **9**. In general formula **9**, the R¹ substituent is as defined above and the group Y is a leaving group such as a halogen, mesylate, triflate or the like. The alkylation of the 2-pyridone (**2**) is performed in a polar, aprotic solvent such as DMF using one of a variety of bases such as an alkali metal carbonate or hydroxide. Deprotonation of the 2-pyridone of general formula **2** affords an ambident anion, which upon alkylation affords a mixture of the *O-*alkylated product of general formula **10** and the *N*-alkylated product of general formula **11**. The desired product is the *O*-alkylated isomer of general formula **10**, which may be purified from the reaction mixture using standard methods such as silica gel chromatography. When the R¹ substituent is an electron-withdrawing group, the pKa of the methylene adjacent the R¹ substituent may be sufficiently low that it is deprotonated following the alkylation reaction. In such an instance, the *O-*alkylated product of general formula **10** cyclizes via an intramolecular nucleophilic attack of the deprotonated methylene group upon the adjacent nitrile and the title compound of general formula **I** where R² is an amino group **12** is produced. In cases where the cyclization of the *O-*alkylated product of general formula **10** is not spontaneous, it is first purified from the reaction mixture and then subjected to treatment with a strong base such as lithium bis(trimethylsilylamide) in an aprotic solvent such as THF to afford compounds of general formula **12**. The primary amino group of compounds of general formula **12** derived using these procedures may be further synthetically modified to afford functional groups that are within the scope of the definition of the substituent R² defined above using methods known in the art of organic synthesis. For instance the amino group in compounds of general formula **12** may be alkylated or used in reductive amination reactions to afford substituted amino groups and it may be acylated to afford amide derivatives.

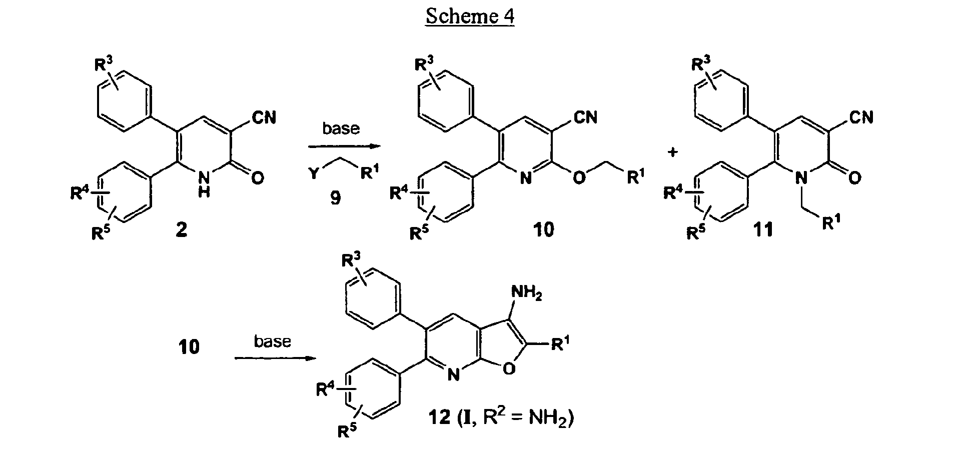

Similarly in reaction Scheme 5, the 3-acyl-2-pyridone of general formula **3** is subjected to an alkylation reaction with an electrophilic reagent of general formula **9** as described above. The alkylation of the 3-acyl-2-pyridone (**3**) is also performed in a polar, aprotic solvent such as DMF using one of a variety of bases such as an alkali metal carbonate or hydroxide and affords a mixture of the *O-*alkylated product of general formula **13** and the *N*-alkylated product of general formula **14**. The desired *O-*alkylated isomer of general formula **13** is usually purified from the reaction mixture using standard methods such as silica gel chromatography. When the R¹ substituent is an electron-withdrawing group, the pKa of the methylene adjacent the R¹ substituent may also be sufficiently low that it is deprotonated following the alkylation reaction. In such an instance, the *O-*alkylated product of general formula **13** may cyclize via an intramolecular nucleophilic attack of the deprotonated methylene group upon the acyl substituent at the 3-position of the pyridone and the title compound of general formula **I** is produced. In cases where the cyclization of the *O-*alkylated product of general formula **13** is not spontaneous, it can be purified from the reaction mixture and then subjected to treatment with a base such as DBU or lithium bis(trimethylsilylamide) in an aprotic solvent such as DMF or THF to afford compounds of general formula **I**.

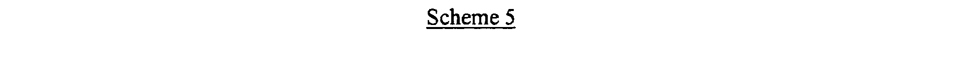

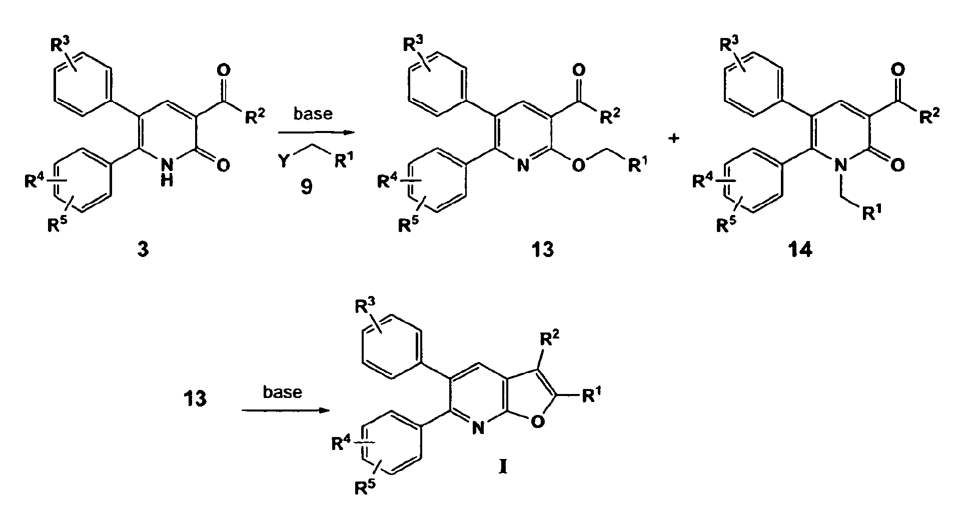

The title compounds of general formula **I** shown in reaction Scheme 5 are also useful intermediates for further synthetic manipulation. When the R² substituent of the compounds in reaction Scheme 5 is an alkyl group, it is possible to further functionalize this substituent using a variety of halogenation or oxidation reactions known in organic synthesis. For instance when the R² substituent is a methyl group, it may be readily converted to a bromomethyl group using *N-*bromosuccinimide as shown in reaction Scheme 6. The resulting bromomethyl derivative **15** may be hydrolyzed to afford compounds of general formula **I** wherein R² is a hydroxymethyl group, or **15** may be oxidized with *N-*methylmorpholine-*N*-oxide (NMO) in a solvent such DMSO, CH₃CN, CH₂Cl₂ or a mixture thereof to afford aldehydes of general formula **16** as shown. The aldehydes of general formula **16** may be converted to an ester **17** directly using Corey's procedure (Corey, E.J.; Gilman, N.W.; Ganem, B.E. J. Am. Chem. Soc. 1968, 90, 5616) in an alcoholic solvent R^{a}OH, where R^{a} is defined above. Alternatively, aldehydes of general formula 16 may be oxidized to the carboxylic acid by various methods such as sodium chlorite-hydrogen peroxide (Dalcanale E.; Montanari, F. J. Org. Chem. 1986, 51, 567). Finally, aldehydes of general formula **16** may be converted into carboxamides of general formula **18** in a two step procedure that involves initially converting the aldehyde to its corresponding oxime using hydroxylamine hydrochloride in the presence of a base. The oxime is then treated with a strong acid, for instance methanesulfonic acid, and water at elevated temperature. The reaction produces the carboxamide **18** as shown, possibly through a mechanism that involves dehydration of the oxime to the corresponding nitrile, followed by hydrolysis of the nitrile to afford **18**.

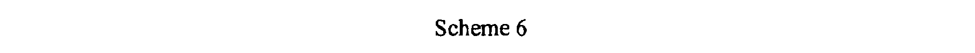

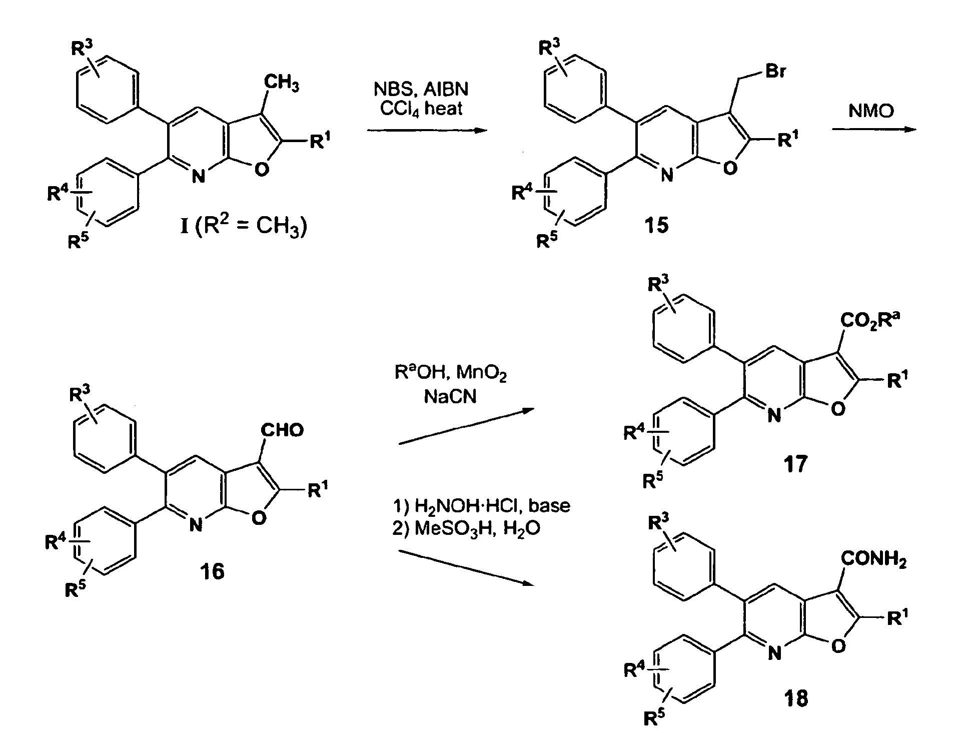

The final stage of the synthesis of the novel compounds of general formula **I** that are the subject of this invention is incorporation of the R⁴ substituent on the phenyl group attached to the 6-position of the furo[2,3*-b*]pyridine ring system. Typically the R³ and R⁵ substituents are present in the starting materials (e.g. compounds **4, 5, 6** & **7**) used to prepare the 1,2-diphenylethanones of general formula **1** as shown in reaction Scheme 2. It is also customary to include an atom or a functional group that may be later elaborated into the final R⁴ substituent in those starting materials (e.g. compounds **5** & **7**). The selection of a bromine atom as the precursor to the R⁴ substituent is a preferred method for the synthesis of the novel compounds of general formula **I**, because it is unreactive throughout the transformations described above in reaction Schemes 2-6, but it may be readily converted into the final R⁴ substituent using a palladium-catalyzed cross coupling process. Reaction Scheme 7 illustrates the final stage of the synthesis of the compounds of general formula **I** from intermediate **19** which is prepared as described above. One preferred strategy involves the palladium-catalyzed Suzuki cross coupling reactions of the aryl bromide **19** with a heteroarylboronic acid **20** containing the R⁴ substituent. This reaction is typically conducted using a palladium catalyst such as tetrakistriphenylphosphine palladium (0) in the presence of a mixed solvent system containing an organic solvent and a basic aqueous solution, and the product **21** is a compound of general formula **I** wherein the R⁴ substituent is a heteroaryl group as defined above. An alternative strategy for the preparation of compounds of general formula **I** when it is desired that the R⁴ substituent be a 1,2,4-oxadiazol-3-yl group is also shown in reaction Scheme 7. In this example, the compound of general formula **19** is first converted to the cyano derivative **22** with a palladium catalyst in the presence of a cyanide source such as sodium cyanide. The cyano group of the compound of general formula **22** is then converted to its corresponding amidoxime using hydroxylamine hydrochloride in the presence of a base such as triethylamine and a solvent such as ethanol. Finally, the intermediate amidoxime is heated in the presence of an ortho-ester to afford a compound of general formula **23** which corresponds to a title compound of general formula **I** wherein the R⁴ substituent is a 1,2,4-oxadiazol-3-yl group.

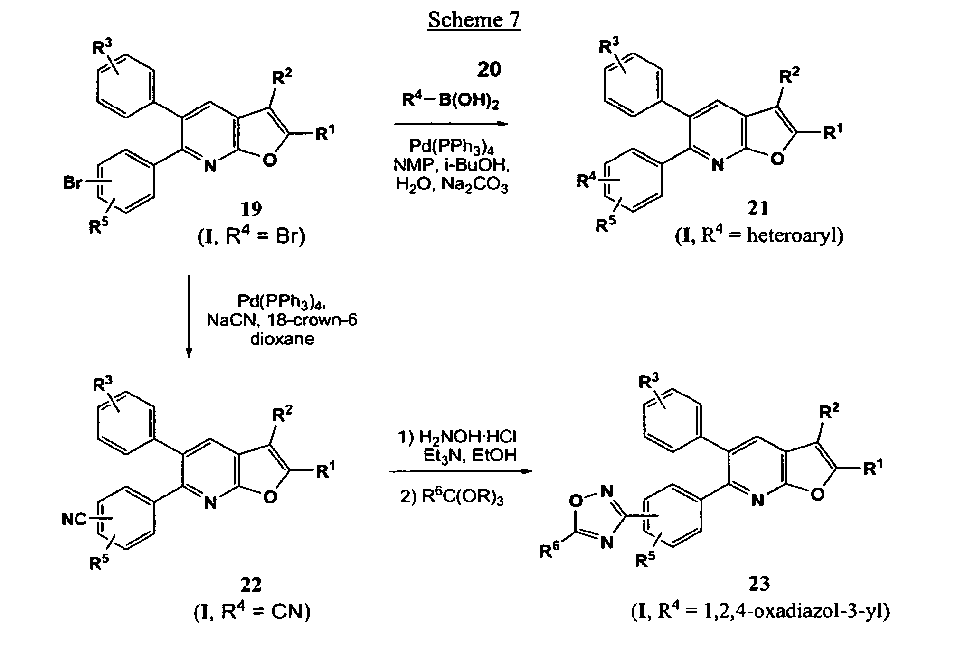

In order to illustrate the invention, the following examples are included. These examples do not limit the invention. They are only meant to suggest a method of reducing the invention to practice. Those skilled in the art may find other methods of practicing the invention which are readily apparent to them. However, those methods are also deemed to be within the scope of this invention.

### General Procedures

Reactions sensitive to moisture or air were performed under nitrogen or argon using anhydrous solvents and reagents. The progress of reactions was determined by either analytical thin layer chromatography (TLC) performed with E. Merck precoated TLC plates, silica gel 60F-254, layer thickness 0.25 mm or liquid chromatography-mass spectrum (LC-MS). Mass analysis was performed on a Waters Micromass^{®} ZQ^{™} with electrospray ionization in positive ion detection mode. High performance liquid chromatography (HPLC) was conducted on an Agilent 1100 series HPLC on Waters C18 XTerra 3.5 µm 3.0 x50 mm column with gradient 10:90-100 v/v CH₃CN/H₂O + v 0.05 % TFA over 3.75 min then hold at 100 CH₃CN + v 0.05 % TFA for 1.75 min; flow rate 1.0 mL/min, UV wavelength 254 nm). Concentration of solutions was carried out on a rotary evaporator under reduced pressure. Flash chromatography was performed using a Biotage Flash Chromatography apparatus (Dyax Corp.) on silica gel (32-63 mM, 60 Å pore size) in pre-packed cartridges.

The X-ray powder diffraction pattern of the free base ethanolate crystalline form of Compound I, was generated on a Philips Analytical X'Pert PRO X-ray Diffraction System with PW3040/60 console. A PW3373/00 ceramic Cu LFF X-ray tube K-Alpha radiation was used as the source. The X-ray powder diffraction spectrum was recorded at ambient temperature.

DSC data were acquired using a TA Instruments DSC-2910 differential scanning calorimeter at a heating rate of 10°C/min under N2 flow. TA Instruments DSC 2910 or equivalent instrumentation. Between 2 and 6 mg sample is weighed into an open aluminum pan. This pan is then crimped and placed at the sample position in the calorimeter cell. The sample is heated in a closed pan. An empty pan is placed at the reference position. The calorimeter cell is closed and a flow of nitrogen is passed through the cell. The heating program is set to heat the sample at a heating rate of 10 °C/min to a temperature of approximately 250 °C. The heating program is started. When the run is completed, the data are analyzed using the DSC analysis program contained in the system software. The melting endotherm is integrated between baseline temperature points that are above and below the temperature range over which the endotherm is observed. The data reported are the onset temperature, peak temperature and enthalpy.

TGA data were acquired using Perkin Elmer TGA-7 thermogravimetric analyzer. Between 5 and 20 mg sample is weighed into a platinum pan. The furnace is raised and a flow of nitrogen is passed over the sample. The heating program is set to heat the sample under a nitrogen flow at a heating rate of 10°C/min to a temperature of approximately 250°C. The heating program is started. When the run is completed, the data are analyzed using the delta Y function in the analysis program contained in the system software. The percent weight loss by the sample is calculated from the onset of the heating program to the melt/decomposition of the sample.

Abbreviations: acetic acid (AcOH), acetate (OAc); aqueous (aq), (benzotriazol-1-yloxy)tripyrrolidinophosphonium hexafluorophosphate (PyBOP), 1,1'-bis(diphenylphosphino)ferrocene (dppf), ethyl acetate (EtOAc), ethanol (EtOH), diethyl ether (ether or Et₂O), *N,N*-diisopropylethylamine (DIEA), 1,2-dimethoxyethane (DME), *N,N-*dimethylformamide (DMF), 1,8-diazabicyclo[5.4.0]undec-7-ene (DBU), 4*-N,N-*dimethylaminopyridine (DMAP), *N*-bromosuccinimide (NBS), azobisisobutyronitrile (AIBN), dimethyl sulfoxide (DMSO), 1-hydroxybenzotriazole (HOBT or HOBt), 1-methyl-2-pyrrolidinone (NMP), *N*-methyl-morpholine-*N*-oxide (NMO), gram(s) (g), hour(s) (h or hr), microliter(s) (µL), milligram(s) (mg), milliliter(s) (mL), millimole (mmol), mass spectrum (ms or MS), methyl cyanide (MeCN), methanol (MeOH), sodium cyanide (NaCN), triethyl amine (NEt₃), 2-propanol (IPA), retention time (Rₜ), room temperature (rt), saturated aq sodium chloride solution (brine), trifluoroacetic acid (TFA), tetrahydrofuran (THF), tetra-*n-*propylammonium perruthenate (TPAP), minute(s) (min), *tert*-butyl methyl ether (MTBE); high pressure liquid chromatography (HPLC); molar (M); liter (L);bis(diisopropylphosphino)-ferrocene (dippf); and normal (N).

### Reference Example 1

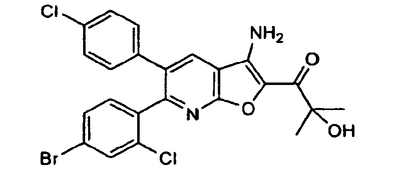

### 1-[3-Amino-6-(4-bromo-2-chlorophenyl)-5-(4-chlorophenyl)furo[2,3-b]pyridin-2-yl]-2-hydroxy-2-methylpropan-1-one

Step A: Methyl 4-bromo-2-chlorobenzoic ester. To 4-bromo-2-chlorobenzoic acid (425 g, 1.805 mol) in MeOH (4.5 L) was added concentrated sulfuric acid (50 mL, 938 mmol) and the suspension was heated at 64°C overnight. The reaction was cooled, diluted with water and extracted with EtOAc. The mixture was washed with saturated aq NaHCO₃, brine, dried (MgSO₄), filtered and concentrated. The residue was purified by flash chromatography on silica gel gradient eluted with 0-10% EtOAc in hexane affording the product.

Step B: 1-(4-Bromo-2-chlorophenyl)-2-(4-chlorophenyl)ethanone. A solution of sodium bis(trimethylsilyl)amide in THF (1 M, 4.70 L, 4.702 mol) was cooled to -78° C. 4-Chlorophenylacetic acid (297 g, 1.742 mol) in THF (1 L) was added dropwise followed by the dropwise addition of a solution of product from Step A (434.5 g, 1.742 mol) in THF (1L). The mixture was stirred overnight at rt. The reaction was quenched by adding it portion wise to HCl (2 N) and then extracted with EtOAc, washed with saturated aq NaHCO₃, brine, dried (Na₂SO₄), filtered and concentrated. The residue was purified by flash chromatography on silica gel gradient eluted with 0-100% EtOAc in hexane affording the product.

Step C: 3-Dimethylamino-1-(4-bromo-2-chlorophenyl)-2-(4-chlorophenyl)prop-2-en-1-one. A solution of product from Step B (421.6 g, 1.225 mol) in DMF (2.7 L) and dimethylformamide dimethylacetal (0.558 L, 4.167 mol) was heated at 90°C for 3 h. The reaction was concentrated to give the crude product which was used directly in the next step.

Step D: 6-(4-Bromo-2-chlorophenyl)-5-(4-chlorophenyl)-2-oxo-1,2-dihydropyridine-3-nitrile. 2-Cyanoacetamide (114 g, 1.360 mol) was dissolved in DMF (0.6 L) and a solution of sodium methoxide in MeOH (25 wt %, 0.880 L, 3.846 mol) was added dropwise and the slurry was stirred for 10 min. A solution of product from Step C (493.6 g, 1.237 mol ) in DMF (1.1 L) was added dropwise and the reaction was heated to 50 °C for 1 h. The reaction was cooled to 10 °C and added to HCl (2 N) and stirred for 30 min. The mixture was filtered and concentrated. The resulting yellow solid was washed with water and placed in a high vacuum oven for 3 days.

Step E: 1-Bromo-3-hydroxy-3-methylbutan-2-one. To 3-hydroxy-3-methyl-2-butanone (12.0 g, 0.12 mol) in ethyl ether (0.1 L) was added bromine (18.78 g, 0.1 mol) at rt. After a few minutes a strong exotherm began which necessitated ice/water cooling and the reaction color changed from dark red to light orange. The reaction mixture was concentrated and taken up in ethyl ether, followed by concentration (4 times), affording the bromo ketone product.

Step F: 1-[3-Amino-5-(4-chlorophenyl)-6-(4-bromo-2-chlorophenyl)furo[2,3-*b*]pyridin-2-yl]-2-hydroxy-2-methylpropan-1-one. To the bromoketone from Step E (6.46 g, 35.7 mmol), in DMF (15 mL), was added potassium carbonate (4.93 g, 35.7 mmol) and the product from Step D (5 g, 11.9 mmol). The reaction was stirred at rt for 1 h. Cesium carbonate (6 g, 18.42 mmol) was added, followed by heating at 60 °C for 1 h. The reaction mixture was allowed to cool to rt and diluted with EtOAc, then washed with water and brine, dried (Na₂SO₄), filtered and concentrated. The residue was purified by flash chromatography on silica gel gradient eluted with 0-10% EtOAc in CH₂Cl₂ affording the title compound. HPLC/MS: 519.1 (M+1), 521.0 (M+3); Rₜ = 3.93 min.

Using the procedure similar to that described in Reference Example 1 in Reference Example 1 and the appropriate α-bromoketone the following compounds were prepared:

| | Name | HPLC /MS *m*/*z* (M+1) *m*/*z* (M+3) Rₜ (min) | Structure |
|---|---|---|---|
| **Reference Example 2** | 1-[3-Amino-6-(4-bromo-2-chlorophenyl)-5-(4-chlorophenyl)furo[2,3-*b*]pyridin-2-yl]-2,2-dimethylpropan-1-one | 516.9 519.0 4.65 | 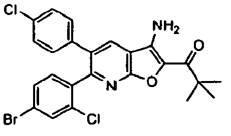 |
| **Reference Example 3** | 1-[3-Amino-6-(4-bromo-2-chlorophenyl)-5-(4-chlorophenyl)furo[2,3-*b*]pyridin-2-yl]propan-1-one | 489.0 491.0 4.06 | 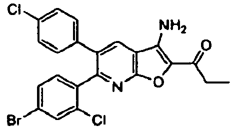 |

### Reference Example 4

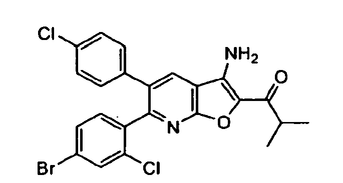

### 1-[3-Amino-6-(4-bromo-2-chlorophenyl)-5-(4-chlorophenyl)furo[2,3-b]pyridin-2-yl]-2-methylpropan-1-one

Step A: 1-Bromo-3-methylbutan-2-one. To 3-methyl-2-butanone (5.2 g, 61 mmol) in MeOH (65 mL) was added bromine (9.2 g, 58 mmol) under ice/water cooling. After an hour of cooling the reaction was still dark and was allowed to come to rt. After 20 min the reaction changed from dark red to light orange. The reaction mixture was concentrated and taken up in CH₂Cl₂ and washed with saturated aq NaHCO₃, brine, dried (Na₂SO₄), filtered and concentrated affording the product.

Step B: 1-[3-Amino-6-(4-bromo-2-chlorophenyl)-5-(4-chlorophenyl)furo[2,3-*b*]pyridin-2-yl]-2-methylpropan-1-one. Using product of Step D Reference Example 1 and product of Step A, along with the procedure described in Step F Reference Example 1, the title compound was obtained. HPLC/MS: 503.1 (M+1), 505.0 (M+3); Rₜ = 4.17 min

### Reference Example 5

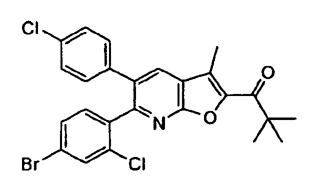

### 1-[6-(4-Bromo-2-chlorophenyl)-5-(4-chlorophenyl)-3-methylfuro[2,3-b]pyridin-2-yl]-2,2-dimethylpropan-1-one

Step A: 3-Acetyl-6-(4-bromo-2-chlorophenyl)-5-(4-chlorophenyl)pyridin-2(1H)-one. The product from Step D Reference Example 1 (0.5 g, 1.18 mmol) was dissolved in THF (4 mL) and methylmagnesium bromide in toluene/THF (1.4 M, 1.5 mL) was added over 5 min. The reaction mixture was heated to 60 °C for 1 h. The reaction was cooled in ice, quenched with HCl (1.0 N), extracted with EtOAc and concentrated. The residue was re-dissolved in hot EtOAc and washed with water, brine, dried (Na₂SO₄), filtered and concentrated affording the product which was used without further purification.

Step B: 1-{[3-Acetyl-6-(4-bromo-2-chlorophenyl)-5-(4-chlorophenyl)pyridin-2-yl]oxy}-3,3-dimethylbutan-2-one. To the product of Step A (presumably 1.18 mmol) in DMF (10 mL) was added cesium carbonate (1.2 g, 3.54 mmol) and 1-bromopinacolone (0.24 mL, 1.77 mmol). The reaction mixture was stirred at rt for 1.5 h, then partitioned between EtOAc and water. The organic layer was washed with 10% aq NaHSO₄, water, brine, dried (Na₂SO₄), filtered and concentrated. The residue was purified on a silica gel flash chromatography column eluted with 0-20% EtOAc in hexane affording the product.

Step C: 1-[6-(4-Bromo-2-chlorophenyl)-5-(4-chlorophenyl)-3-methylfuro[2,3-*b*]pyridin-2-yl]-2,2-dimethylpropan-1-one. To the product of Step B (presumably 1.18 mmol) in DMF (5 mL) was added DBU (0.04 mL, 0.236 mmol). The mixture was heated at 90 °C for 3 hr, cooled to rt and partitioned between EtOAc and saturated aq NaHCO₃. The organic layer was washed with brine, dried (Na₂SO₄), filtered and concentrated. The residue was purified by flash chromatography on silica gel gradient eluted with 0-15% EtOAc in hexane affording the title compound. HPLC/MS: 516.1 (M+1), 518.1 (M+3); Rₜ = 4.75 min.

### Reference Example 6

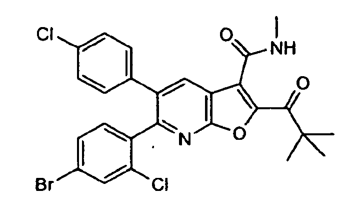

### 6-(4-Bromo-2-chlorophenyl)-5-(4-chlorophenyl)-2-(2,2-dimethylpropanoyl)-N-methylfuro[2,3-b]pyridine-3-carboxamide.

Step A: 1-[6-(4-Bromo-2-chlorophenyl)-3-(bromomethyl)-5-(4-chlorophenyl)furo[2,3-*b*]pyridin-2-yl]-2,2-dimethylpropan-1-one. To the product of Reference Example 5 (5.5 g, 10.6 mmol) in CHCl₃ (50 mL) was added NBS (2.082 g, 11.7 mmol) and AIBN (0.175 g, 1.07 mmol). The reaction was heated at reflux for 3 h and concentrated. The residue was purified by flash chromatography on silica gel gradient eluted with 0-20% EtOAc in hexane affording the product. Alternatively, isopropyl acetate has been used as the solvent for this reaction.

Step B: 6-(4-Bromo-2-chlorophenyl)-5-(4-chlorophenyl)-2-(2,2-dimethylpropanoyl)furo[2,3-b]pyridine-3-carboxylic acid. To the product from Step A (5.70 g, 9.56 mmol) in DMSO (20 mL) was added sodium nitrite (1.979 g, 28.7 mmol) and acetic acid (5.47 mL, 96 mmol). The reaction was heated at 40° C overnight, followed by stirring at room temperature for 2 days. The reaction was quenched by HCl (2 M) and the solution was extracted with EtOAc, dried (Na₂SO₄), filtered and concentrated. The residue was purified by flash chromatography on silica gel gradient eluted with 0-40% EtOAc in hexane affording the product.

Alternatively, the product of Step B (isolated as the diisopropylamine salt) has been formed utilizing the following chemistry: To the product of Step A (10.0 g, 16.95 mmol, 90% purity) in acetone (50 mL) and water (2.5 mL) was added sulfamic acid (1.97 g, 20.3 mmol). The slurry was aged at room temperature for 20 minutes. Sodium chlorite (2.1 g, 18.6 mmol, 80% purity) dissolved in water (3.8 mL) was added dropwise keeping the temperature between 25 and 30 °C. After about 25% of the total amount of sodium chlorite was added (over 5 minutes), and the solution was aged at 25°C for 15 minutes. The addition was resumed, and the remainder of sodium chlorite was added over 10 minutes keeping the temperature between 25 - 30°C. After 30 minutes, the solution was partitioned between EtOAc (65 mL) and 5% aqueous NaHSO₃ (40 mL). The layers were separated, and the organic layer was washed with 5% aqueous NaCl (40 mL). The organic layer was concentrated to remove acetone and azeotropically dried (1 to 2 volumes of EtOAc were used). The solution was adjusted to a final volume of about 85 mL with EtOAc, and diisopropylamine (2.62 mL, 18.7 mmol) was added to crystallize the salt. The resulting slurry was aged at room temperature for 2 hours, filtered and rinsed with EtOAc. The resulting solid was dried in an oven at 45°C under vacuum and a sweep of nitrogen for 24 hours to afford the product as the diisopropylamine salt.

Step C: 6-(4-Bromo-2-chlorophenyl)-5-(4-chlorophenyl)-2-(2,2-dimethylpropanoyl)-*N-*methylfuro[2,3-*b*]pyridine-3-carboxamide. The product from Step B (0.25 g, 0.457 mmol) was dissolved in DMF (2 mL). PyBOP (0.357 g, 0.685 mmol), HOBT (0.105 g, 0.685 mmol) and methylamine (2 M in THF, 1.14 mL, 2.28 mmol) were added and the reaction was stirred at rt for 15 min. The solution was diluted with EtOAc and washed with saturated aq NaHCO₃, dried (Na₂SO₄), filtered and concentrated. The residue was purified by flash chromatography on silica gel gradient eluted with 0-20% EtOAc in hexane affording the title compound. 559.2 (M+1), 561.2 (M+3); Rₜ = 4.22 min.

### Reference Example 7

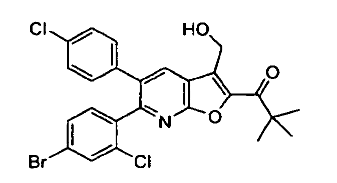

1-[6-(4-bromo-2-chlorophenyl)-5-(4-chlorophenyl)-3-(hydroxymethyl)furo[2,3-*b*]pyridin-2-yl]-2,2-dimethylpropan-1-one. The title compound was isolated as a side product from Step B Reference Example 6. HPLC/MS: 532.0 (M+1), 534.0 (M+3); Rₜ = 4.16 min.

### Reference Example 8

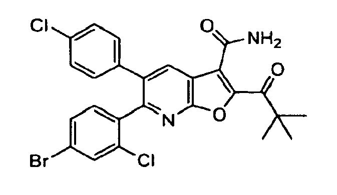

### 6-(4-Bromo-2-chlorophenyl)-5-(4-chlorophenyl)-2-(2,2-dimethylpropanoyl)furo[2,3-b]pyridine-3-carboxamide.

Step A: 6-(4-Bromo-2-chlorophenyl)-5-(4-chlorophenyl)-2-(2,2-dimethylpropanoyl)furo[2,3-*b*]pyridine-3-carbaldehyde. The product from Step A Reference Example 6 (50 mg, 0.084 mmol) was dissolved in CH₂Cl₂ (1 mL) and MeCN (2 mL). NMO (29.5 mg, 0.252 mmol) was added and the reaction was stirred at rt for 3 h. The solution was concentrated and re-dissolved in EtOAc, washed with HCl (2 M) and the aqueous portion was extracted with EtOAc twice. The combined organic layers were washed with brine, dried (Na₂SO₄), filtered and evaporated. The residue was purified by flash chromatography on silica gel gradient eluted with 0-6% EtOAc in hexane affording the product.

Step B: 6-(4-Bromo-2-chlorophenyl)-5-(4-chlorophenyl)-2-(2,2-dimethylpropanoyl)furo[2,3-*b*]pyridine-3-carbaldehyde oxime. To the product of Step A (50 mg, 0.094 mmol) in MeOH (1 mL) was added hydroxylamine hydrochloride (13 mg, 0.188 mmol) and potassium acetate (18.5 mg, 0.188 mmol). The reaction was heated at 40°C for 1 h. The reaction was diluted with EtOAc, washed with brine and saturated aq NaHCO₃, dried (Na₂SO₄), filtered and concentrated to afford the product.

Step C: 6-(4-Bromo-2-chlorophenyl)-5-(4-chlorophenyl)-2-(2,2-dimethylpropanoyl)furo[2,3-*b*]pyridine-3-carboxamide. To the product of Step B (1.0 g, 1.88 mmol) in methanesulfonic acid (5 mL), was added water (0.2 mL) and the reaction was heated at 100 °C for 4 h. The reaction was cooled and diluted with EtOAc, then washed twice with brine and twice with saturated aq NaHCO₃, dried (Na₂SO₄), filtered and concentrated. The residue was purified by flash chromatography on silica gel gradient eluted with 0-20% EtOAc in hexane affording the title compound. HPLC/MS: 545.1 (M+1), 547.1 (M+3); Rₜ = 4.12 min.

Alternatively, the product of Reference Example 8 has been formed utilizing the following chemistry: A slurry of the diisopropylamine salt product of Reference Example 6 Step B (15.0 g, 23.1 mmol), EtOAc (75 mL), and DMF (4 drops, about 85 mg) was treated with oxalyl chloride (3.7 g, 29.1 mmol) dropwise over 5 minutes keeping the temperature ≤ 25°C. After about 30 minutes to 1 hour at room temperature, the reaction mixture was cooled to about -30°C, and NH₄OH (16.2 mL, 28-30%, ~115 mmol) was added in one portion. The resulting slurry was warmed to room temperature. Heptane (75 mL) was added over 30 minutes, then the slurry was aged at room temperature for 2 hours, cooled to 0-5°C for an hour, filtered, and rinsed with water (50 mL), followed by IPA (30 mL). The resulting product was dried in the oven at 65 °C under vacuum and a sweep of nitrogen for 24 hours to afford the title compound.

Using the procedure described in Step B and C, Reference Example 5, and 1-bromo-3-hydroxy-3-methylbutan-2-one, the product of Step E Reference Example 1, the following compound was afforded:

| | Name | HPLC /MS *m*/*z* (M+1) *m*/*z* (M+3) Rₜ (min) | Structure |
|---|---|---|---|
| **Reference Example 9** | 1-[6-(4-Bromo-2-chlorophenyl)-5-(4-chlorophenyl)-3-methylfuro[2,3-*b*]pyridin-2-yl]-2-hydroxy-2-methylpropan-1-one | 517.9 519.9 4.55 | 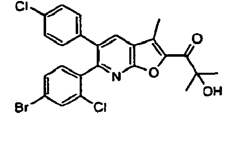 |

### Reference Example 10

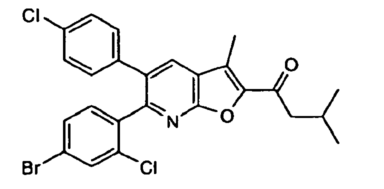

### 1-[6-(4-Bromo-2-chlorophenyl)-5-(4-chlorophenyl)-3-methylfuro[2,3-b]pyridin-2-yl]-3-methylbutan-1-one

Step A: Ethyl 6-(4-bromo-2-chlorophenyl)-5-(4-chlorophenyl)-3-methylfuro[2,3-*b*]pyridine-2-carboxylate. Using ethyl bromoacetate and the procedure described in Reference Example 5, the product was afforded.

Step B: 6-(4-Bromo-2-chlorophenyl)-5-(4-chlorophenyl)-3-methylfuro[2,3-*b*]pyridine-2-carboxylic acid. To the product obtained in Step A (1.54 g, 3 mmol) in THF (10 mL) was added KOH (1 g, 1.78 mmol in 10 mL water). The reaction was heated at 70 °C for 2 h. The reaction was cooled and HCl (2 M) and EtOAc were added. The organic layer was isolated, dried (Na₂SO₄), filtered and concentrated to afford the product, which was used without any further purification.

Step C: 6-(4-Bromo-2-chlorophenyl)-5-(4-chlorophenyl)-*N*-methoxy-*N*,3-dimethylfuro[2,3-*b*]pyridine-2-carboxamide. Product from Step B (0.5 g, 1.05 mmol) was dissolved in CH₂Cl₂ (10 mL). Oxalyl chloride (0.092 mL. 1.05 mmol) was added and reaction was stirred at rt for 1 h. An aliquot work-up with diethylamine suggested the reaction was not done and additional oxalyl chloride (0.4 mL, 4.5 mmol) was added. The reaction was stirred another 1 h. The reaction mixture was concentrated and re-dissolved in CH₂Cl₂ (3 mL). *N*-methoxymethanamine hydrochloride (0.300 g, 3.14 mmol) and NEt₃ (0.73 mL, 5.24 mmol) were added. The reaction was stirred 20 min and applied directly on a silica gel flash chromatography column eluted with 0-30% EtOAc in hexane affording the product.

Step D: 1-[6-(4-Bromo-2-chlorophenyl)-5-(4-chlorophenyl)-3-methylfuro[2,3-*b*]pyridin-2-yl]-3-methylbutan-1-one. The product from Step C (0.35 g, 0.67 mmol) was dissolved in THF (10 mL). Isobutyl magnesium bromide in THF (2 M, 1 mL, 2 mmol) was added and the reaction was stirred for 30 min. The reaction was quenched with HCl (1M) and EtOAc was added. The organic layer was separated, washed with water, brine, dried (Na₂SO₄), filtered and concentrated. The residue was purified by flash chromatography on silica gel gradient eluted with 0-15% EtOAc in hexane affording the title compound. HPLC/MS: 516.1 (M+1), 518.1 (M+3); Rₜ = 4.24 min.

Using the procedure described in Step D Reference Example 10, along with the requisite alkyl Grignard reagent, the following compounds were afforded:

| | Name | HPLC /MS *m*/*z* (M+1) *m*/*z* (M+3) Rₜ (min) | Structure |
|---|---|---|---|
| **Reference Example 11** | 1-[6-(4-Bromo-2-chlorophenyl)-5-(4-chlorophenyl)-3-methylfuro[2,3-*b*]pyridin-2-yl]-2-methylpropan-1-one | 502.1 504.1 4.39 | 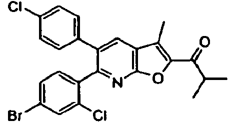 |
| **Reference Example 12** | 1-[6-(4-Bromo-2-chlorophenyl)-5-(4-chlorophenyl)-3-methylfuro[2,3-*b*]pyridin-2-yl]propan-1-one | 488.1 490.1 4.29 | 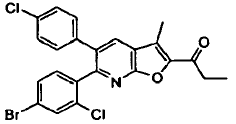 |

### Reference Example 13

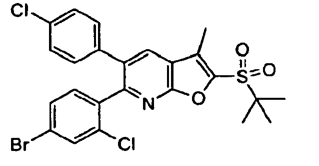

### 6-(4-Bromo-2-chlorophenyl)-2-(tert-butylsulfonyl)-5-(4-chlorophenyl)-3-methylfuro[2,3-b]pyridine

Step A: 1-[6-(4-Bromo-2-chlorophenyl)-2-[(*tert*-butylthio)methoxyl-5-(4-chlorophenyl)pyridin-3-yl]ethanone. To the product from Step A Reference Example 5 (5.1 g, 11.7 mmol) in DMF (35 mL) was added cesium carbonate (4.75 g, 14.5 mmol), KI (0.9 g, 0.58 mmol) and *tert-*butyl(chloromethyl)sulfide (1.9 g, 14 mmol). The reaction was heated to 45 °C for 90 min and additional KI (0.3 g, 1.7 mmol) was added. The reaction was heated an additional 30 min, cooled to rt and 10 % aq NaHSO₄ and EtOAc were added. The organic layer was separated, washed with water, brine, dried (Na₂SO₄), filtered and concentrated. The residue was purified by flash chromatography on silica gel gradient eluted with 0-20% EtOAc in hexane affording the product. Step B: 1-[6-(4-Bromo-2-chlorophenyl)-2-[(*tert*-butylsulfonyl)methoxy]-5-(4-chlorophenyl)pyridin-3-yl]ethanone. The product (2 g, 3.7 mmol) from Step A was dissolved in MeCN (12.6 mL) and CH₂Cl₂ (5.4 mL). The reaction was cooled to -20°C, and 3-chloroperoxybenzoic acid (2.5 g, 11.2 mmol) was added. The reaction was allowed to come to rt and stirred for 1 h. EtOAc and saturated aq NaHCO₃ were added. The organic layer was separated, washed with water, brine, dried (Na₂SO₄), filtered and concentrated affording the product.

Step C: 6-(4-Bromo-2-chlorophenyl)-2-(*tert*-butylsulfonyl)-5-(4-chlorophenyl)-3-methylfuro[2,3-*b*]pyridine. To the product of Step B (2.1 g, 3.68 mmol) in DMF (22 mL) and water (0.77 mL) was added cesium carbonate (4.7 g, 14 mmol) in 3 portions in intervals of 30 min, while the reaction was heated at 138 °C for a total of 90 min. The reaction mixture was cooled and diluted with EtOAc and 10 % aq NaHSO₄. The organic layer was collected, washed with water, brine, dried (Na₂SO₄), filtered and concentrated. The residue was purified by flash chromatography on silica gel gradient eluted with 0-20% EtOAc in hexane affording the title compound. HPLC/MS: 552.1 (M+1), 554.1 (M+3); Rₜ = 4.20 min.

### Reference Example 14

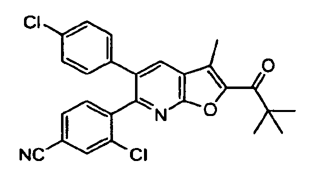

1-[6-(4-Cyano-2-chlorophenyl)-5-(4-chlorophenyl)-3-methylfuro[2,3-*b*]pyridin-2-yl]-2,2-dimethylpropan-1-one. The product of Step C in Reference Example 5 (0.150 g, 0.29 mmol) was dissolved in dioxane (3 mL). NaCN (22 mg, 0.44 mmol), 18-crown-6 (120 mg, 0.44 mmol), and tetrakis(triphenylphosphine)palladium(0) (170 mg, 0.15 mmol) were added and the flask was evacuated and backfilled with nitrogen 3 times. The mixture was heated at 100 °C overnight. The reaction was cooled and partitioned between EtOAc and saturated aq NaHCO₃, washed with water, brine, dried (Na₂SO₄), filtered and concentrated. The residue was purified by flash chromatography on silica gel gradient eluted with 0-20% EtOAc in hexane affording the title compound. HPLC/MS: 463.2 (M+1), 465.2 (M+3); Rₜ = 4.41 min.

### Reference Example 15

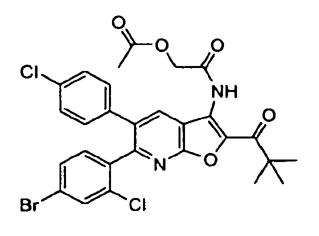

2-{[5-(4-Chlorophenyl)-6-(4-bromo-2-chlorophenyl)-2-(2,2-dimethylpropanoyl)furo[2,3-*b*]pyridin-3-yl]amino}-2-oxoethyl acetate. To a suspension of the product of Reference Example 2 (0.335 g; 0.65 mmol) in MeCN (3 mL) was added acetoxyacetyl chloride (0.2 mL, 1.95 mmol). The reaction mixture was stirred at rt for 30 min, then quenched with saturated aq NaHCO₃ and extracted with EtOAc. The organic layer was washed twice with saturated aq NaHCO₃, brine, dried (Na₂SO₄), filtered, and concentrated affording the title compound. HPLC/MS: 617.0 (M+1), 619.0 (M+3); Rₜ = 4.75 min.

Using the procedure described in Reference Example 15 and the products obtained from Reference Example 1, Reference Example 2 and Reference Example 3, along with the appropriate acid chloride, the following compounds were obtained:

| | Name | HPLC /MS *m*/*z* (M+1) *m*/*z* (M+3) Rₜ (min) | Structure |
|---|---|---|---|
| **Reference Example 16** | 2- {[6-(4-Bromo-2-chlorophenyl)-5-(4-chlorophenyl)-2-(2-hydroxy-2-methylpropanoyl)furo[2,3-*b*]pyridin-3-yl]amino}-2-oxoethyl acetate | 619.1 621.1 4.01 | 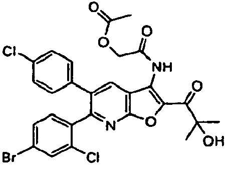 |
| **Reference Example 17** | 2-{[6-(4-Bromo-2-chlorophenyl)-5-(4-chlorophenyl)-2-propionylfuro[2,3-*b*]pyridin-3-yl]amino}-2-oxoethyl acetate | 589.1 591.1 4.20 | 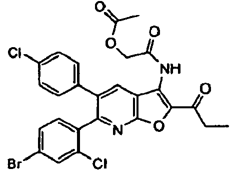 |
| **Reference Example 18** | *N*-[6-(4-Bromo-2-chlorophenyl)-5-(4-chlorophenyl)-2-(2,2-dimethylpropanoyl)furo[2,3-b]pyridin-3-yl]-2-methylpropanamide | 587.2 589.2 4.66 | 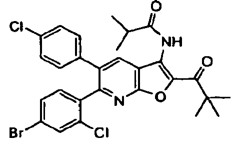 |
| **Reference Example 19** | *N*-[6-(4-Bromo-2-chlorophenyl)-5-(4-chlorophenyl)-2-(2,2-dimethylpropanoyl)furo[2,3-b]pyridin-3-yl]propanamide | 573.2 575.2 4.57 | 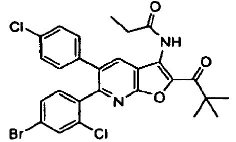 |
| **Reference Example 20** | (1*S*)-2-{[6-(4-Bromo-2-chlorophenyl)-5-(4-chlorophenyl)-2-propionylfuro[2,3-*b*]pyridin-3-yl]amino}-1-methyl-2-oxoethyl acetate | 603.2 605.1 4.35 | 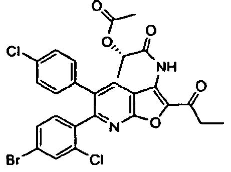 |

### Reference Example 21

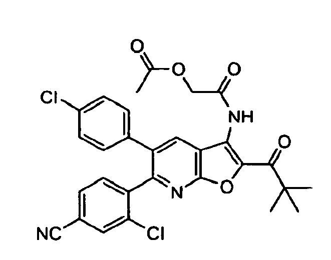

2- {[6-(2-chloro-4-isocyanophenyl)-5-(4-chlorophenyl)-2-(2,2-dimethylpropanoyl)furo[2,3-b]pyridin-3-yl]amino}-2-oxoethyl acetate. Using the product of Reference Example 15 and the procedure described for the preparation of Reference Example 14 the title compound was afforded. HPLC/MS: 564.1 (M+1), 566.0 (M+3); Rₜ = 4.49 min.

### Reference Example 22

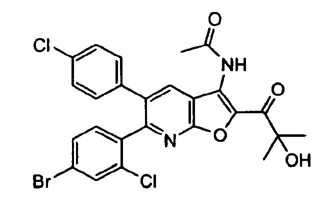

*N*-[6-(4-Bromo-2-chlorophenyl)-5-(4-chlorophenyl)-2-(2-hydroxy-2-methylpronanoyl)furo[2,3-*b*]pyridin-3-yl]acetamide. The product of Reference Example 1 (1.1 g, 2.1 mmol), acetic anhydride (15 mL) and acetic acid (6 mL) were combined. The reaction was heated to 90 °C until all starting material was consumed. The reaction was concentrated, taken up in EtOAc, washed several times with brine and concentrated. The residue was purified by flash chromatography on silica gel gradient eluted with 0-45% EtOAc in hexane affording the title compound. HPLC/MS: 561.1 (M+1), 563.1 (M+3); Rₜ = 3.97 min.

Using procedure described in Reference Example 22 and products of Example 2, 3 and 4 the following compounds were afforded:

| | Name | HPLC /MS *m*/*z* (M+1) *m*/*z* (M+3) Rₜ (min) | Structure |
|---|---|---|---|
| **Reference Example 23** | *N*-[6-(4-Bromo-2-chlorophenyl)-5-(4-chlorophenyl)-2-(2,2-dimethylpropanoyl)-furo[2,3-*b*]pyridin-3-yl]acetamide | 559.1 561.1 4.42 | 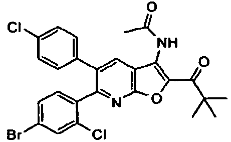 |
| **Reference Example 24** | *N*-[6-(4-Bromo-2-chlorophenyl)-5-(4-chlorophenyl)-2-propionylfuro[2,3-*b*]pyridin-3-yl]acetamide | 531.1 533.1 4.20 | 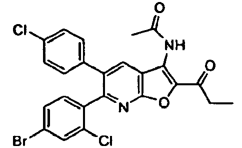 |
| **Reference Example 25** | *N*-[6-(4-Bromo-2-chlorophenyl)-5-(4-chlorophenyl)-2-isobutyrylfuro[2,3-*b*]pyridin-3-yl]acetamide | 545.1 547.1 4.31 | 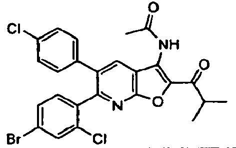 |

### Reference Example 26

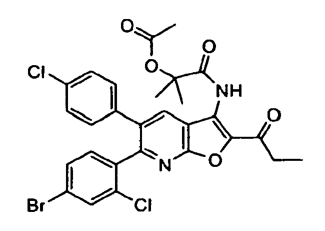

2-{[6-(4-Bromo-2-chlorophenyl)-5-(4-chlorophenyl)-2-propionylfuro[2,3-*b*]pyridin-3-yl]amino}-1,1-dimethyl-2-oxoethyl acetate. To the product of Reference Example 3 (0.275 g; 0.56 mmol) in MeCN (6 mL) was added 2-chloro-1,1-dimethyl-2-oxoethyl acetate (0.45 mL; 3 mmol), NEt₃ (0.16 mL, 1.12 mmol) and DMAP (55 mg, 0.45 mmol). The reaction mixture was stirred at 70 °C for 4 h. The reaction was diluted with EtOAc and washed with saturated aq NaHCO₃ and brine, dried (Na₂SO₄), filtered, and concentrated. The residue was purified by flash chromatography on silica gel gradient eluted with 0-20% EtOAc in hexane affording the title compound. HPLC/MS: 617.2 (M+1), 619.2 (M+3); Rₜ = 4.43 min.

Using procedure described in Reference Example 26, and the product of Reference Example 2, the following compounds were afforded:

| | Name | HPLC /MS *m*/*z* (M+1) *m*/*z* (M+3) Rₜ (min) | Structure |
|---|---|---|---|
| **Reference Example 27** | (1*S*)-2-{[6-(4-Bromo-2-chlorophenyl)-5-(4-chlorophenyl)-2-(2,2-dimethylpropanoyl)-furo[2,3-*b*]pyridin-3-yl]amino}-1-methyl-2-oxoethyl acetate | 631.2 633.2 4.54 | 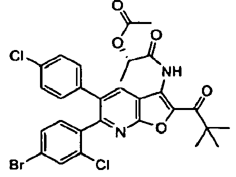 |
| **Reference Example 28** | 2- {[6-(4-Bromo-2-chlorophenyl)-5-(4-chlorophenyl)-2-(2,2-dimethylpropanoyl)-furo[2,3-*b*]pyridin-3-yl]amino}-1,1-dimethyl-2-oxoethyl acetate | 645.2 647.3 4.60 | 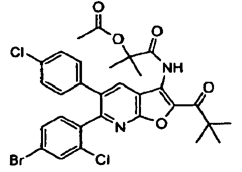 |

### Reference Example 29

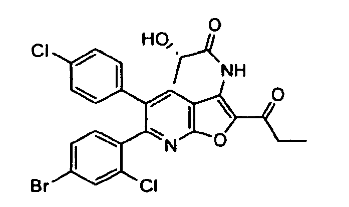

(2*S*)-*N*-[6-(4-Bromo-2-chlorophenyl)-5-(4-chlorophenyl)-2-propionylfuro[2,3-*b*]pyridin-3-yl]-2-hydroxypropanamide. To the product of Reference Example 20 (383 mg, 0.634 mmol) in THF (12.6 mL) was added LiOH (15.2 mg, 0.634 mmol) in MeOH (0.4 mL). The reaction stirred at rt for 25 min and was quenched with a few drops of AcOH and concentrated. The residue was dissolved in EtOAc, washed with brine/saturated aq NaHCO₃ (1:1) and concentrated. The residue was purified by flash chromatography on silica gel gradient eluted with 0-80% EtOAc in hexane affording the title compound. HPLC/MS: 561.1 (M+1), 563.1 (M+3); Rₜ = 4.14 min.

Using the procedure described in Reference Example 29, along with the appropriate starting material, the following compounds were obtained:

| | Name | HPLC /MS *m*/*z* (M+1) *m*/*z* (M+3) Rₜ (min) | Structure |
|---|---|---|---|
| **Reference Example 30** | *N*-[6-(4-Bromo-2-chlorophenyl)-5-(4-chlorophenyl)-2-propionylfuro[2,3-*b*]pyridin-3-yl]-2-hydroxyacetamide | 547.1 549.1 4.01 | 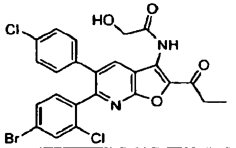 |
| **Reference Example 31** | *N*-[6*N*-[6-(4-Bromo-2-chlorophenyl)-5-(4-chlorophenyl)-2-(2,2-dimethylpropanoyl)furo[2,3-*b*]pyridin-3-yl]-2-hydroxyacetamide | 575.1 577. 4.19 | 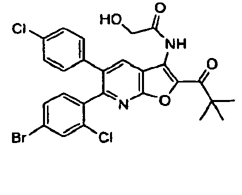 |
| **Reference Example 32** | *N*-[6-(4-Bromo-2-chlorophenyl)-5-(4-chlorophenyl)-2-propionylfuro[2,3-*b*]pyridin-3-yl]-2-hydroxy-2-methylpropanamide | 575.1 577.1 4.22 | 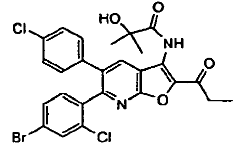 |
| **Reference Example 33** | *N*-[6-(4-Bromo-2-chlorophenyl)-5-(4-chlorophenyl)-2-(2,2-dimethylpropanoyl)furo[2,3-*b*]pyridin-3-yl]-2-hydroxy-2-methylpropanamide | 603.2 605.2 4.40 | 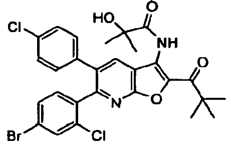 |
| **Reference Example 34** | (2*S*)-*N*-[6-(4-Bromo-2-chlorophenyl)-5-(4-chlorophenyl)-2-(2,2-dimethylpropanoyl)furo[2,3-b]pyridin-3-yl]-2-hydroxypropanamide | 589.1 591.1 4.31 | 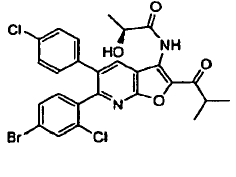 |

### Reference Example 35

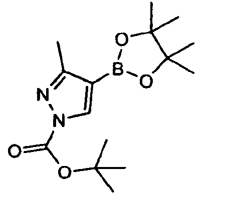

*N*-Boc-3-methyl-4-(4,4,5,5-tetramethyl-1,3,2-dioxaborolan-2-yl)-pyrazole. (Note the product may or may not contain a mixture of Boc regioisomers and the location of the Boc group was not determined.)

Step A: *N*-Boc-4-bromo-3-methyl-pyrazole. To 4-bromo-3-methyl-pyrazole (3.50 g, 21.7 mmol) was added CH₂Cl₂ (30 mL), di-*tert*-butyl dicarbonate (5.22g, 23.9 mmol) and aq Na₂CO₃ (1 M, 43.5 mL). The reaction was stirred for 2.5 h and the aq portion was then separated. The CH₂Cl₂ portion was then washed with 5% citric acid followed by brine/saturated aq NaHCO₃ (1:1). The solution was dried (Na₂SO₄), filtered and concentrated to afford the product.

Step B: *N*-Boc-3-methyl-4-(4,4,5,5-tetramethyl-1,3,2-dioxaborolan-2-yl)-pyrazole. To the product of Step A (3.30 g 12.6 mmol) was added bis(pinacolato)diboron (3.53 g,13.9 mmol), potassium acetate (3.72 g, 37.9 mmol), 1,1'-bis(diphenylphosphino)ferrocenedichloropalladium (II) (0.457 g, 0.632 mmol) and DMSO (15 mL). The reaction vessel was purged with N₂ for 5 min at rt prior to heating to 80 °C for about 5 hours. The reaction was cooled to rt, diluted with EtOAc and filtered through Celite. The solution was then washed with brine 5 times and concentrated. The residue was purified by flash chromatography on silica gel gradient eluted with 0-21 % EtOAc in hexane affording the title compound. HPLC/MS: 309.3 (M+1); Rₜ = 3.48 min.

### Reference Example 36

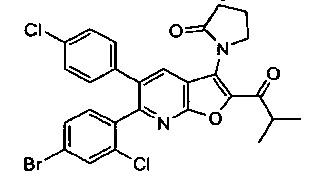

1-[6-(4-Bromo-2-chlorophenyl)-5-(4-chlorophenyl)-2-isobutylfuro[2,3-*b*]pyridin-3-yl]pyrrolidin-2-one. To the product of Reference Example 4 (663 mg, 1.315 mmol) in acetonitrile (12 mL) was added 4-chlorobutanoyl chloride (0.236 mL, 2.10 mmol). The reaction was heated to 65 °C for 25 min. The reaction was diluted with EtOAc and washed with brine/2 M aq Na₂CO₃ (1:1). The solution was dried (Na₂SO₄), filtered and concentrated. To the residue dissolved in dimethylacetamide (6mL) was added NaH (60 mg, 1.5 mmol, 60%). The temperature was increased to 40 °C for 90 min. The reaction was diluted with EtOAc, washed with brine/aq HCl (2M) (5:1) and concentrated. The residue was purified by flash chromatography on silica gel gradient eluted with 0-28% EtOAc in hexane affording the title compound. HPLC/MS: 571.2 (M+1), 573.2 (M+3); Rₜ = 4.11 min.

### Reference Example 37

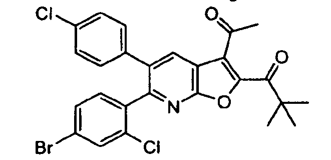

### 1-[3-acetyl-6-(4-Bromo-2-chlorophenyl)-5-(4-chlorophenyl)furo[2,3-b]pyridin-2-yl]-2,2-dimethylpropan-1-one

Step A: 1-[6-(4-Bromo-2-chlorophenyl)-3-(1-bromoethyl)-5-(4-chlorophenyl)furo[2,3-*b*]pyridin-2-yl]-2,2-dimethylpropan-1-one. To 1-[6-(4-bromo-2-chlorophenyl)-5-(4-chlorophenyl)-3-ethylfuro[2,3-*b*]pyridin-2-yl]-2,2-dimethylpropan-1-one (prepared in similar fashion to Reference Example 5 except using ethylmagnesium chloride in step A) (4.54 g, 8.55 mmol) in CCl₄ (50 mL) was added AIBN (0.281 g, 1.71 mmol) and NBS (1.673 g, 9.40 mmol). The reaction was heated to reflux for 3 hr and then an additional charge of AIBN (0.281 g) was added. The reaction was stirred at reflux for an additional hour before adding 1,1'-azobis-1-cyclohexanenitrile (400 mg 1.64 mmol). The reaction stirred at reflux 1 hour and then concentrated. The residue was purified by flash chromatography on silica gel gradient eluted with 0-10% EtOAc in hexane affording the product.

Step B: 1-[3-Acetyl-6-(4-bromo-2-chlorophenyl)-5-(4-chlorophenyl)furo[2,3-*b*]pyridin-2-yl]-2,2-dimethylpropan-1-one. To the product of Step A (700 mg, 1.147 mmol) and NMO (672 mg, 5.74 mmol) was added acetonitrile (20 mL). The reaction stirred at 70 °C for 1.5 h. The reaction was cooled to rt and TPAP (24 mg, 0.068 mmol) was added. The reaction stirred at rt for 40 min and concentrated. The residue was purified by flash chromatography on silica gel gradient eluted with 0-25% EtOAc in hexane affording the title compound. HPLC/MS: 544.0 (M+1), 546.0 (M+3); Rₜ = 4.29 min.

### Reference Example 38

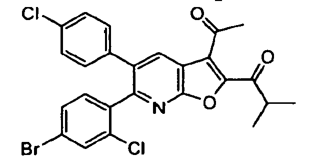

### 1-[3-Acetyl-6-(4-Bromo-2-chlorophenyl)-5-(4-chlorophenyl)furo[2,3-b]pyridin-2-yl]-2-methylpropan-1-one

Step A: 1-[6-(4-bromo-2-chlorophenyl)-3-(1-bromoethyl)-5-(4-chlorophenyl)furo[2,3-*b*]pyridin-2-yl]-2-methylpropan-1-one. To 1-[6-(2-chlorophenyl)-5-(4-chlorophenyl)-3-ethylfuro[2,3-b]pyridin-2-yl]-2-methylpropan-1-one (prepared in similar fashion to Reference Example 5 except using ethyl magnesium chloride in Step A to form the 3-propionyl-2-pyridone and then using 1-bromo-3-methylbutan-2-one for Step B to form the furopyridine) (1.00 g, 1.93 mmol), NBS (0.354 g, 1.99 mmol) and AIBN (79 mg, 0.483 mmol) was added CCl₄ (12 mL). The reaction was heated to reflux for about 1 hr and then concentrated. The residue was purified by flash chromatography on silica gel gradient eluted with 0-10% EtOAc in hexane affording the product.

Step B: 1-[6-(4-Bromo-2-chlorophenyl)-5-(4-chlorophenyl)-3-(1-hydroxyethyl)furo[2,3-b]pyridin-2-yl]-2-methylpropan-1-one. To the product of Step A (500 mg, 0.755 mmol) in 1-methyl-2-pyrrolidinone (NMP) (10 mL) was added KI (160 mg, 0.964 mmol) and saturated aq NaHCO3 (about 0.75 mL). The reaction was heated to 100 °C and then cooled to rt. The reaction was diluted with EtOAc and washed with brine. The concentrated residue was purified by flash chromatography on silica gel gradient eluted with 0-25% EtOAc in hexane affording the product.

Step C: 1-[3-Acetyl-6-(4-bromo-2-chlorophenyl)-5-(4-chlorophenyl)furo[2,3-*b*]pyridin-2-yl]-2-methylpropan-1-one. To the product of Step B (219 mg, 0.411 mmol) in acetonitrile (4 mL) and CH₂Cl₂ (2mL) was added TPAP (11.6 mg, 0.033 mmol) and NMO (72.2 mg, 0.616 mmol). The reaction stirred at rt for 25 min and was concentrated. The residue was purified by flash chromatography on silica gel gradient eluted with 0-20% EtOAc in hexane affording the title compound. HPLC/MS: 530.1 (M+1), 532.2 (M+3); Rₜ = 4.32 min.

### Reference Example 39

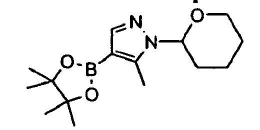

### 5-methyl-1-(tetrahydro-2H-pyran-2-yl)-4-(4,4,5,5-tetramethyl-1,3,2-dioxaborolan-2-yl)-1H-pyrazole. (Note the title compound was isolated as a mixture of tetrahydropyran regioisomers.)

Step A: 4-iodo-5-methyl-1-(tetrahydro-2*H*-pyran-2-yl)-1*H*-pyrazole. To a mixture of 4-iodo-3-methyl-1*H*-pyrazole (20.9 g, 100 mmol, prepared by the method described by Rodrigues-Franco, M.I., Dorronsoro, I., Hemandex-Higueras, A.I., Antequera, G. Tetrahedron Lett. 2001, 42, 863-865.), THF (50 mL), and 3,4-dihydro-2*H*-pyran (10.0 mL, 110 mmol) was added TFA (0.39 mL, 5 mmol). The mixture was heated to 60°C for about 4 hours, allowed to cool to room temperature, and partitioned between a 5% aqueous Na₂CO₃ (150 mL) and MTBE (100 mL). The layers were separated, and the organic layer was washed with water and then concentrated to afford the title product.

Step B: 5-methyl-1-(tetrahydro-2*H*-pyran-2-yl)-4-(4,4,5,5-tetramethyl-1,3,2-dioxaborolan-2-yl)-1*H*-pyrazole. The product of Step A (5.42g g 18.56 mmol) in THF (59 mL) was cooled to -40 °C. Isopropylmagnesium chloride (12.1 mL, 24.2 mmol, 2M in THF) was added keeping the temperature below -40°C. After completion of the addition, the reaction was held at -40°C for 30 minutes. Trimethyl borate (4.1 mL, 37.1 mmol) was then added keeping the temperature below - 37°C. The reaction was warmed to 0°C for 5 minutes. Then acetic acid (4.73 mL, 83.5 mmol) was added in one portion (the temperature increased to 6°C), followed by the addition of pinacol (3.07 g, 26.0 mmol) in one portion. The reaction mixture was allowed to warm to room temperature, and aged for 16 hours. The mixture was then partitioned between 10% aqueous NH₄Cl (40 mL) and MTBE (50 mL). The resulting layers were separated, and the organic layer was washed with saturated aqueous NaHCO₃, and water, and then concentrated to afford the title compound. HPLC/MS: 293.0 (M+1); Rₜ = 2.93 min.

### Example 1

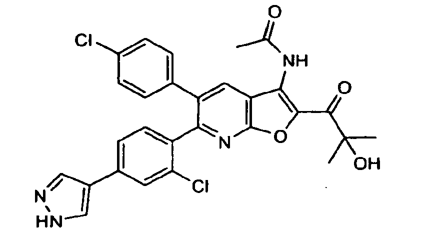

*N*-[5-(4-Chlorophenyl)-6-[2-chloro-4-(1*H*-pyrazol-4-yl)phenyl]-2-(2-hydroxy-2-methylpropanoyl)furo[2,3-*b*]pyridin-3-yl]acetamide. The product of Reference Example 22 (0.100 g, 0.178 mmol) was dissolved in NMP (1.8 mL), water (0.16 mL) and isopropanol (1.35 mL) in a 10 mL reaction tube of a CEM Corporation Discover 300 Watt microwave reactor. An aq solution of Na₂CO₃ (1 M, 0.16 mL, 0.16 mmol), *tert-*butyl 4-(4,4,5,5-tetramethyl-1,3,2-dioxaborolan-2-yl)-1*H*-pyrazole-1-carboxylate (78 mg, 0.267 mmol, Sigma Aldrich) and tetrakis(triphenyl-phosphine)palladium(0) (15 mg, 0.012 mmol) were added and the tube was purged with nitrogen, capped and inserted into the microwave reactor and heated at 105 °C, 50 watts maximum power, for 3 min. The reaction was diluted with EtOAc, washed with brine, dried (Na₂SO₄), filtered and concentrated. The residue was purified by flash chromatography on silica gel gradient eluted with 0-100% EtOAc in hexane affording the title compound. HPLC/MS: 549.1 (M+1), 551.1 (M+3); Rₜ = 3.39 min. ¹H NMR (500 MHz, CHCl₃ -d): δ 10.48 (s, 1H); 9.05 (s, 1H); 7.88 (s, 2 H); 7.47 (s, 1 H); 7.41 (d, J = 8.03 Hz, 1 H); 7.32 (d, J = 7.90 Hz, 1H); 7.22 (d, J = 8.31 Hz, 2H), 7.16 (d, J = 8.32 Hz, 2 H); 2.37 (s, 3 H); 1.72 (s, 6 H).

### Example 2

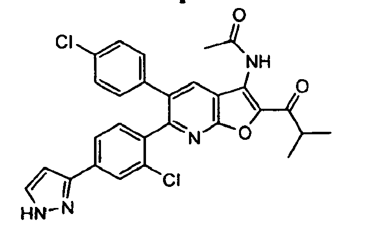

*N*-{5-(4-Chlorophenyl)-6-[2-chloro-4-(1*H*-pyrazol-3-yl)phenyl]-2-isobutyrylfuro[2,3-*b*]pyridin-3-yl}acetamide. To the product of Reference Example 25 (4.50 g, 8.24 mmol) was added 1H-pyrazol-3-ylboronic acid (1.659 g, 14.83 mmol, ChemBridge), tetrakis(triphenylphosphine)-palladium (0) (286 mg, 0.247 mmol), NMP (40 mL), water (3.2 mL) 2-butanol (30 mL) and aq Na₂CO₃ (2 M, 6.18 mL). The reaction vessel was purged with N₂ for 4 min at rt prior to heating to 100 °C for about 3 hours. The reaction was cooled and then concentrated. The residue was dissolved in EtOAc and washed with brine. The concentrated residue was purified by flash chromatography on silica gel gradient eluted with 0-50% EtOAc in hexane. Further purification was achieved by suspension of the product in 2-propanol (heated to 60 °C), followed by filtration of the cooled mixture and concentration to afford the title compound. HPLC/MS: 533.3 (M+1), 535.2 (M+3); Rₜ = 3.83 min. ¹H NMR (500 MHz, CHCl₃ -d): δ 10.44 (s, 1 H); 9.03 (s, 1 H); 7.78 (s, 1 H); 7.69 (d, J = 7.99 Hz, 1 H); 7.63 (d, J = 2.33 Hz, 1 H); 7.35 (d, J = 7.95 Hz, 1 H); 7.23-7.13 (m, 4 H); 6.65 (d, J = 2.38 Hz, 1H); 3.65-3.54 (m, 1H); 2.33 (s, 3 H); 1.30 (d, J = 6.85 Hz, 6 H).

### Example 3

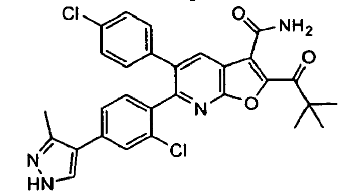

6-[2-chloro-4-(3-methyl-1*H*-pyrazol)phenyl]-5-(4-chlorophenyl)-2-(2,2-dimethylpropanoyl)furo[2,3-*b*]pyridine-3-carboxamide. To the product of Reference Example 8 (4.34 g, 7.95 mmol) was added the product of Reference Example 35 (4.41 g, 14.30 mmol), tetrakis(triphenylphosphine)palladium (0) (413 mg, 0.358 mmol), NMP (40 mL), water (3.2 mL) 2-butanol (30 mL) and aq Na₂CO₃ (2 M, 6.75 mL). The reaction vessel was purged with N₂ for 4 min at rt prior to heating to 100 °C for about 4 hours. The reaction was cooled and then concentrated. The residue was dissolved in EtOAc and washed with saturated aqueous NaHCO₃. Brine has been used instead of NaHCO₃. The concentrated residue was purified by flash chromatography on silica gel gradient eluted with 0-60% EtOAc in hexane. Further purification was achieved by suspension of the product in 2-propanol (heated to 80 °C), followed by filtration of the cooled mixture to afford the title compound. HPLC/MS: 547.0 (M+1), 549.0 (M+3); Rₜ = 3.73 min. ¹H NMR (500 MHz, DMSO-d₆): δ 12.73 (bd, J = 33.27 Hz, 1H); 8.36 (s, 1H); 8.21 (s, 1 H); 8.11 (bs, 0.4 H); 7.92 (s, 1H); 7.81 (bs, 0.6 H); 7.50-7.42 (m, 3 H); 7.36 (d, J = 8.38 Hz, 2 H); 7.26 (d, J = 8.37 Hz, 2 H); 2.38 (s, 3 H); 1.38 (s, 9 H).
Alternatively, the product of Example 3 has been prepared utilizing the following chemistry: A mixture of the product of Reference Example 8 (5.12 g, 9.37 mmol), toluene/water (42 mL, 17 mL), the product of Reference Example 39 (8.87 g, 14.1 mmol, assay 46.35 weight% in toluene), and potassium phosphate tribasic (5.97 g, 28.1 mmol) was sparged with nitrogen for 30 minutes at room temperature. A separate catalyst mixture of Pd(OAc)₂ (84.2 mg, 0.375 mmol), dippf (bis(diisopropylphosphino)ferrocene) (157 mg, 0.375 mmol) and toluene (pre-sparged with nitrogen) was stirred for 30 minutes at room temperature. The mixture of the boronate and bromide was then heated to 60°C and stirred for 10 minutes before adding the catalyst mixture. The resulting mixture was stirred at 60°C for 14 hours, then the mixture was diluted with EtOAc (50 mL) and water (38 mL) and filtered through Solka Flok™. The resulting layers were separated and the organic layer was concentrated, and diluted with EtOH (28 mL) and aqueous HCl (3.7 mL, ~12N). The resulting organic layer was aged 2 hours at room temperature and then heated to 45°C for about 3.5 hours. MP-TMT resin (5.6 g, Argonaut) was added in one portion. The mixture was aged at 47°C for about 16.5 hours, then filtered, and rinsed with EtOAc. The mixture was treated with aqueous 2N NaOH to pH 10. The resulting layers were separated and the organic layer was washed twice with water. The organic layer was then concentrated and crystallized from EtOH and water to afford the crystalline 1:1 ethanol solvate polymorphic form I of the title compound.

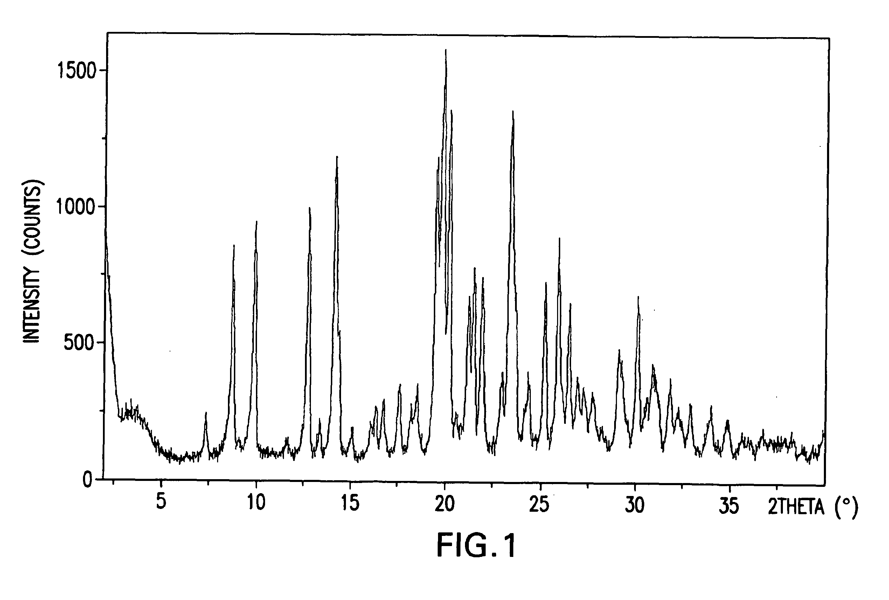

The X-ray powder diffraction (XPRD) pattern for the anhydrous free base crystalline 1:1 ethanol solvate polymorphic form I of Example 3 is shown in Figure 1. The X-ray powder diffraction pattern was recorded at ambient temperature (CuKα radiation, 2° to 40° (2θ), steps of 0.0167°, 5.08 sec per step). Cu K-α of wavelength 1.54187Å was used for d-spacing calculation.

**Table 1. Powder X-ray diffraction of the anhydrous free base crystalline 1:1 ethanol solvate polymorphic form I of Example 3**

| 2θ(2 theta)(degrees) | d-spacing (Å) |
|---|---|
| 7.3 | 12.040 |
| 8.7 | 10.131 |
| 9.9 | 8.960 |
| 12.8 | 6.942 |
| 14.1 | 6.261 |
| 15.1 | 5.876 |
| 16.7 | 5.296 |
| 17.6 | 5.044 |
| 18.5 | 4.794 |
| 19.5 | 4.561 |
| 19.8 | 4.485 |
| 20.1 | 4.408 |
| 21.5 | 4.140 |
| 23.3 | 3.815 |

Although the anhydrous free base crystalline 1:1 ethanol solvate polymorphic form I of Example 3 is characterized by the complete group of angle 2 theta values listed in Table 1, all the values are not required for such identification. The anhydrous free base crystalline 1:1 ethanol solvate polymorphic form I of Example 3 can be identified by the angle theta value in the range of 19.6-20.9°. Further, the anhydrous free base crystalline 1:1 ethanol solvate polymorphic form I of Example 3 can be identified by any one of the following angle theta values, or any one of the following groups of angle theta values:
a) 19.8°;
b) 19.8° and 20.1°;
c) 19.8°, 20.1° and 19.5°;
d) 19.8°, 20.1°, 19.5 and 18.5°;
e) 19.8°, 20.1°, 19.5°, 18.5° and 21.5°;
f) 19.8°, 20.1°, 19.5°, 18.5°, 21.5° and 14.1°;
g) 19.8°, 20.1°, 19.5°, 18.5°, 21.5°, 14.1° and 12.8°;
h) 19.8°, 20.1°, 19.5°, 18.5°, 21.5°, 14.1°, 12.8° and 9.9°;
i) 19.8°, 20.1 °, 19.5°, 18.5°, 21.5°, 14.1 °, 12.8°, 9.9° and 8.7°.

The anhydrous free base crystalline 1:1 ethanol solvate polymorphic form I of Example 3 can also be identified by any one of the following d-spacings, or any one of the following groupings of d-spacings, from an X-ray diffraction pattern obtained using Cu radiation:
a) 4.485, 4.408 and 4.561 angstroms;
b) 4.794, 4.140 and 6.261 angstroms; and
c) 6.942, 8.960 and 10.131 angstroms.

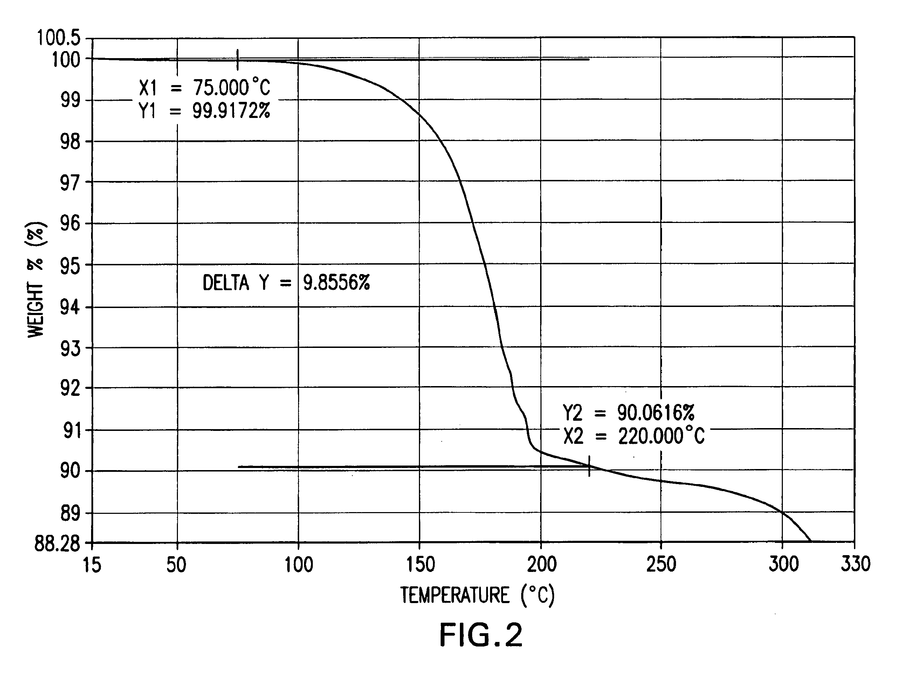

The thermogravimetric analysis (TGA) curve in Figure 2 was obtained for the free base crystalline 1:1 ethanol solvate polymorphic form I of Example 3, under nitrogen flow at a heating rate of 10°C /minute, showed a weight loss of 9.9% from 75°C to 220°C attributed to evolution of ethanol and residual ethyl acetate was observed.

The differential scanning calorimetry (DSC) curve was obtained for the free base crystalline 1:1 ethanol solvate polymorphic form I of Example 3 under a nitrogen flow at a heating rate of 10 °C/minute in a closed aluminum pan. The DSC curve is characterized by an endotherm attributed to desolvation with an extrapolated onset temperature of 173.8 °C, a peak temperature of 178.0 °C and an enthalpy of 138.8 J/g.

Using the general Suzuki coupling procedures described in Example 1 or Example 2, the appropriate starting material and *tert*-butyl 4-(4,4,5,5-tetramethyl-1,3,2-dioxaborolan-2-yl)-1*H-*pyrazole-1-carboxylate (Sigma-Aldrich) the following compounds were obtained:

| | Name | HPLC /MS *m*/*z* (M+1) *m*/*z* (M+3) Rₜ (min) | Structure |
|---|---|---|---|
| **Example 4** | *N*-[5-(4-Chlorophenyl)-6-[2-chloro-4-(1*H*-pyrazol-4-yl)phenyl]-2-(2,2-dimethylpropanoyl)furo[2,3-b]pyridin-3-yl]acetamide | 547.2 549.2 3.88 | 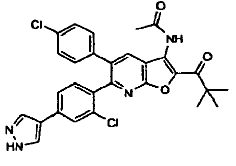 |
| **Example 5** | *N*-{5-(4-Chlorophenyl)-6-[2-chloro-4-(1*H*-pyrazol-4-yl)phenyl]-2-propionylfuro[2,3-*b*]pyridin-3-yl}acetamide | 519.2 521.2 3.63 | 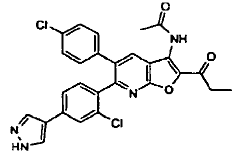 |
| **Example 6** | *N*-[5-(4-Chlorophenyl)-6-[2-chloro-4-(1*H*-pyrazol-4-yl)phenyl]-2-(2,2-dimethylpropanoyl)furo[2,3-*b*]pyridin-3-yl]-2-methylpropanamide | 575.2 577.2 4.2 | 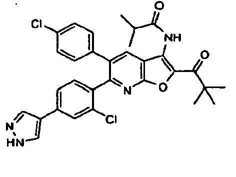 |
| **Example 7** | 1- {3-Amino-5-(4-chlorophenyl)-6-[2-chloro-4-(1*H*-pyrazol-4-yl)phenyl]furo[2,3-*b*]pyridin-2-yl}-2,2-dimethylpropan-1-one | 505.2 507.2 3.70 | 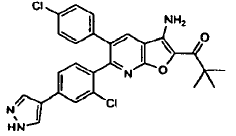 |
| **Example 8** | 1- {3-Amino-5-(4-chlorophenyl)-6-[2-chloro-4-(1*H*-pyrazol-4-yl)phenyl]furo[2,3-*b*]pyridin-2-yl}propan-1-one | 477.2 479.2 3.46 | 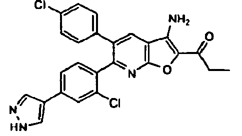 |
| **Example 9** | 1- {5-(4-Chlorophenyl)-6-[2-chloro-4-(1*H*-pyrazol-4-yl)phenyl]-3-methylfuro[2,3-*b*]pyridin-2-yl}-2-hydroxy-2-methylpropan-1-one | 506.2 508.2 3.58 | 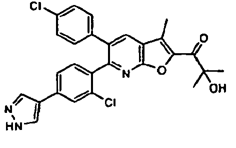 |
| **Example 10** | 1- {5-(4-Chlorophenyl)-6-[2-chloro-4-(1*H*-pyrazol-4-yl)phenyl]-3-methylfuro[2,3-*b*]pyridin-2-yl}-2,2-dimethylpropan-1-one | 504.2 506.2 4.04 | 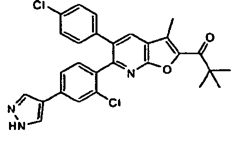 |
| **Example 11** | 2-(*Tert*-butylsulfonyl)-5-(4-chlorophenyl)-6-[2-chloro-4-(1*H*-pyrazol-4-yl)phenyl]-3-methylfuro[2,3-*b*]pyridine | 540.2 542.2 3.7 | 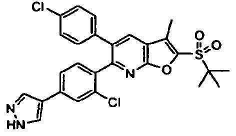 |
| **Example 12** | 1-{5-(4-Chlorophenyl)-6-[2-chloro-4-(1*H*-pyrazol-4-yl)phenyl]-3-methylfuro[2,3-*b*]pyridin-2-yl}-3-methylbutan-1-one | 504.2 506.2 4.01 | 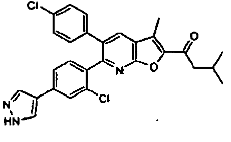 |
| **Example 13** | *N*-{5-(4-Chlorophenyl)-6-[2-chloro-4-(1*H*-pyrazol-4-yl)phenyl]-2-propionylfuro[2,3-*b*]pyridin-3-yl}-2-hydroxy-2-methylpropanamide | 563.2 565.2 3.64 | 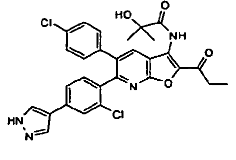 |
| **Example 14** | (2*S*)-*N*-[5-(4-Chlorophenyl)-6-[2-chloro-4-(1*H*-pyrazol-4-yl)phenyl]-2-(2,2-dimethylpropanoyl)furo[2,3-*b*]pyridin-3-yl]-2-hydroxypropanamide | 577.2 579.2 3.71 | 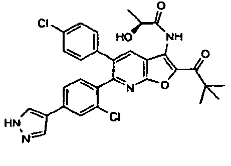 |
| **Example 15** | (2*S*)-*N*-{5-(4-Chlorophenyl)-6-[2-chloro-4-(1*H*-pyrazol-4-yl)phenyl]-2-propionylfuro[2,3-*b*]pyridin-3-yl}-2-hydroxypropanamide | 549.3 551.3 3.54 | 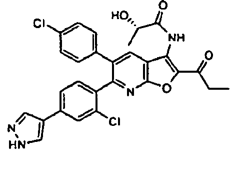 |
| **Example 16** | *N*-[5-(4-Chlorophenyl)-6-[2-chloro-4-(1*H*-pyrazol-4-yl)phenyl]-2-(2,2-dimethylpropanoyl)furo[2,3-*b*]pyridin-3-yl]-2-hydroxy-2-methylpropanamide | 591.2 593.2 3.82 | 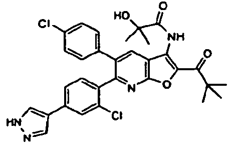 |
| **Example 17** | *N*- {5-(4-Chlorophenyl)-6-[2-chloro-4-(1*H*-pyrazol-4-yl)phenyl]-2-propionylfuro[2,3-*b*]pyridin-3-yl}-2-hydroxyacetamide trifluoroacetate (salt) | 535.2 537.2 3.40 | 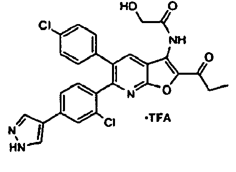 |

Using the general Suzuki coupling procedures described in Example 1 or Example 2, the appropriate starting material and 1*H*-pyrazol-3-ylboronic acid (ChemBridge) the following compounds were obtained:

| | Name | HPLC /MS *m*/*z* (M+1) *m*/*z* (M+3) Rₜ (min) | Structure |
|---|---|---|---|
| **Example 18** | *N*-[5-(4-Chlorophenyl)-6-[2-chloro-4-(1*H*-pyrazol-3-yl)phenyl]-2-(2-hydroxy-2-methylpropanoyl)furo[2,3-*b*]pyridin-3-yl]acetamide | 549.2 551.2 3.46 | 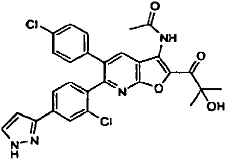 |
| **Example 19** | *N*-[5-(4-Chlorophenyl)-6-[2-chloro-4-(1*H*-pyrazol-3-yl)phenyl]-2-(2,2-dimethylpropanoyl)furo[2,3-*b*]pyridin-3-yl]acetamide | 547.2 549.2 3.94 | 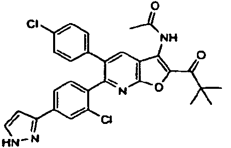 |
| **Example 20** | 5-(4-Chlorophenyl)-6-[2-chloro-4-(1H-pyrazol-3-yl)phenyl]-2-(2,2-dimethylpropanoyl)furo[2,3-b]pyridine-3-carboxamide | 533.3 535.3 3.61 | 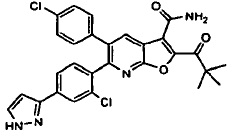 |
| **Example 21** | 1-{3-Amino-5-(4-chlorophenyl)-6-[2-chloro-4-(1*H*-pyrazol-3-yl)phenyl]furo-[2,3-*b*]pyridin-2-yl}-2-hydroxy-2-methylpropan-1-one | 507.2 509.2 3.37 | 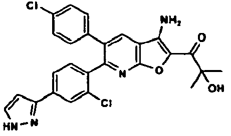 |
| **Example 22** | 1- {3-Amino-5-(4-chlorophenyl)-6-[2-chloro-4-(1*H*-pyrazol-3-yl)phenyl]furo-[2,3-*b*]pyridin-2-yl}-2-methylpropan-1-one | 491.2 493.2 3.63 | 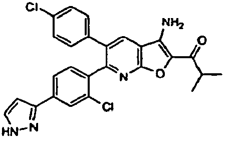 |
| **Example 23** | 1- {5-(4-Chlorophenyl)-6-[2-chloro-4-(1*H*-pyrazol-3-yl)phenyl]-3-methylfuro[2,3-*b*]pyridin-2-yl}-2-hydroxy-2-methylpropan-1-one | 506.2 508.2 3.64 | 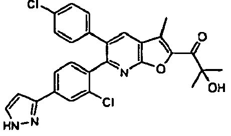 |
| **Example 24** | 2-(T*ert*-butylsulfonyl)-5-(4-chlorophenyl)-6-[2-chloro-4-(1*H*-pyrazol-3-yl)phenyl]-3-methylfuro[2,3-*b*]pyridine | 540.2 542.1 3.70 | 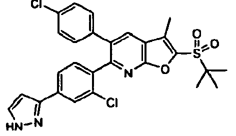 |
| **Example 25** | 1-{5-(4-Chlorophenyl)-6-[2-chloro-4-(1*H*-pyrazol-3-yl)phenyl]-3-methylfuro[2,3-*b*]pyridin-2-yl}-2-methylpropan-1-one | 490.2 492.2 3.95 | 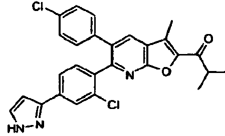 |
| **Example 26** | 1-{5-(4-Chlorophenyl)-6-[2-chloro-4-(1*H*-pyrazol-3-yl)phenyl]-3-methylfuro[2,3-*b*]pyridin-2-yl}propan-1-one | 476.2 478.2 3.83 | 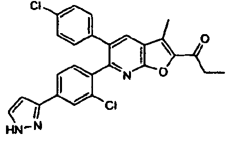 |
| **Example 27** | *N*-[5-(4-Chlorophenyl)-6-[2-chloro-4-(3*H*-pyrazol-5-yl)phenyl]-2-(2,2-dimethyl-propanoyl)furo[2,3-*b*]pyridin-3-yl]-2-hydroxyacetamide | 563.2 565.2 3.67 | 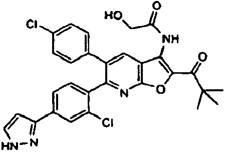 |
| **Example 28** | 1-{5-(4-Chlorophenyl)-6-[2-chloro-4-(1H-pyrazol-3-yl)phenyl]-2-isobutyrylfuro[2,3-*b*]pyridin-3-yl}pyrrolidin-2-one | 559.3 561.2 3.63 | 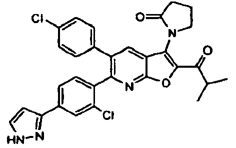 |
| **Example 29** | 5-(4-Chlorophenyl)-6-[2-chloro-4-(1H-pyrazol-3-yl)phenyl]-2-(2,2-dimethylpropanoyl)-N-methylfuro[2,3-b]pyridine-3-carboxamide | 547.2 549.3 3.76 | 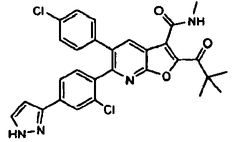 |

### Example 30

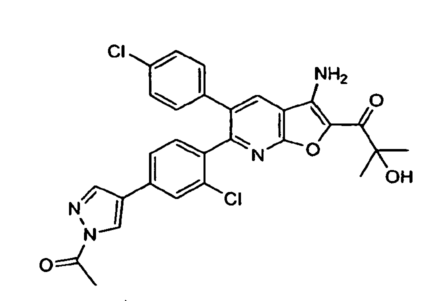

1-[6-[4-(1-Acetyl-1*H*-pyrazol-4-yl)-2-chlorophenyl]-3-amino-5-(4-chlorophenyl)furo[2,3-*b*]pyridin-2-yl]-2-hydroxy-2-methylpropan-1-one. The title compound was isolated as a side product when performing the Suzuki coupling to prepare the product of Example 1 in DME (1 mL), water (0.2 mL) and ethanol (0.4 mL) with the addition of cesium carbonate (120 mg, 0.356 mmol) and running the reaction in similar fashion to Example 1. HPLC/MS: 549.2 (M+1), 551.1, (M+3); Rₜ = 3.47 min. ¹H NMR (500 MHz, CHCl₃ -d): δ 7.97 (s, 1 H); 7.87 (s, 2 H); 7.45 (s, 1 H); 7.42-7.36 (m, 1H); 7.29 (d, J = 7.93 Hz, 1H); 7.22 (d, J = 8.29 Hz, 2 H); 7.12 (d, J = 8.31 Hz, 2 H); 5.83 (s, 2 H); 2.13 (s, 3 H); 1.71 (s, 6 H).

### Example 31

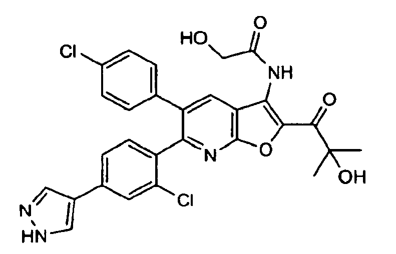

### N-[5-(4-Chlorophenyl)-6-[2-chloro-4-(1H-pyrazol-4-yl)phenyl]-2-(2-hydroxy-2-methylpropanoyl)-furo[2,3-b]pyridin-3-yl]-2-hydroxyacetamide

Step A: 2-{[5-(4-Chlorophenyl)-6-[2-chloro-4-(1*H*-pyrazol-4-yl)phenyl]-2-(2-hydroxy-2-methylpropanoyl)furo[2,3-*b*]pyridin-3-yl]amino}-2-oxoethyl acetate Using the product of Reference Example 16 and procedure for Example 1 the title compound was obtained.

Step B: *N*-[5-(4-Chlorophenyl)-6-[2-chloro-4-(1*H*-pyrazol-4-yl)phenyl]-2-(2-hydroxy-2-methylpropanoyl)furo[2,3-*b*]pyridin-3-yl]-2-hydroxyacetamide To the product of Step A (70 mg, 0.115 mmol) in THF (2.3 mL) was added a solution of LiOH in MeOH (1.7 M, 2.76 mg, 0.115 mmol). The reaction was stirred for 10 min, quenched with acetic acid, and concentrated. The residue was re-dissolved in EtOAc and washed with brine, dried (Na₂SO₄), filtered and concentrated. The residue was purified by flash chromatography on silica gel gradient eluted with 0-100% EtOAc in hexane affording the title compound. HPLC/MS: 565.2 (M+1), 567.2 (M+3); Rₜ = 3.21 min. ¹H NMR (500 MHz, CHCl₃ -d/CH30H-d): δ 11.46 (s, 1H); 9.14 (s, 1 H); 7.85 (s, 2 H); 7.47 (s, 1H); 7.41 (d, J = 7.97 Hz, 1H); 7.31 (d, J = 10.04 Hz, 1H); 7.22 (d, J = 7.93 Hz, 2 H); 7.17 (d, J = 8.10 Hz, 2 H); 4.31 (s, 2 H); 1.72 (s, 6 H).

Using the procedure described in Example 31, and the product of Reference Example 15, the following compound was obtained:

| | Name | HPLC /MS *m*/*z* (M+1) *m*/*z* (M+3) Rₜ (min) | Structure |
|---|---|---|---|
| **Example 32** | *N*-[5-(4-Chlorophenyl)-6-[2-chloro-4-(1*H*-pyrazol-4-yl)phenyl]-2-(2,2-dimethylpropanoyl)furo[2,3-*b*]pyridin-3-yl]-2-hydroxyacetamide | 563.3 565.2 3.61 | 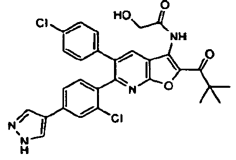 |

Using the general Suzuki coupling procedures described in Example 1 or Example 3, the appropriate starting material and the product of Reference Example 35 the following compounds were obtained:

| | Name | HPLC /MS *m*/*z* (M+1) *m*/*z* (M+3) Rₜ (min) | Structure |
|---|---|---|---|
| **Example 33** | N-[6-[2-Chloro-4-(3-methyl-1H-pyrazol-4-yl)phenyl]-5-(4-chlorophenyl)-2-isobutyrylfuro[2,3-b]pyridin-3-yl]acetamide | 547.2 549.2 3.77 | 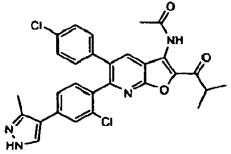 |
| **Example 34** | 1-[6-[2-Chloro-4-(3-methyl-1H-pyrazol-4-yl)phenyl]-5-(4-chlorophenyl)-3-methylfuro[2,3-b]pyridin-2-yl]-2-hydroxy-2-methylpropan-1-one | 520.2 522.2 3.59 | 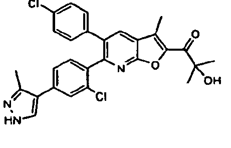 |
| **Example 35** | 1-[6-[2-Chloro-4-(3-methyl-1H-pyrazol-4-yl)phenyl]-5-(4-chlorophenyl)-3-ethylfuro[2,3-b]pyridin-2-yl]-2-hydroxy-2-methylpropan-1-one | 534.2 536.2 3.70 | 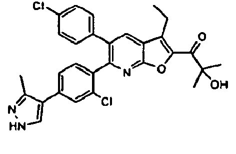 |
| **Example 36** | 1-[6-[2-Chloro-4-(3-methyl-1H-pyrazol-4-yl)phenyl]-5-(4-chlorophenyl)-3-methylfuro[2,3-b]pyridin-2-yl]-2,2-dimethylpropan-1-one | 518.2 520.2 4.01 | 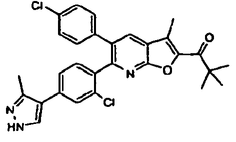 |
| **Example 37** | 1-[6-[2-Chloro-4-(3-methyl-1H-pyrazol-4-yl)phenyl]-5-(4-chlorophenyl)-3-(hydroxymethyl)furo[2,3-b]pyridin-2-yl]-2,2-dimethylpropan-1-one | 534.2 536.2 3.65 | 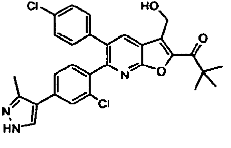 |
| **Example 38** | 1-[3-Acetyl-6-[2-chloro-4-(3-methyl-1H-pyrazol-4-yl)phenyl]-5-(4-chlorophenyl)furo[2,3-b]pyridin-2-yl]-2-methylpropan-1-one | 532.2 534.2 3.82 | 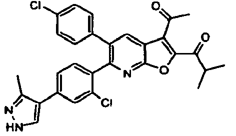 |
| **Example 39** | 1-[6-[2-Chloro-4-(3-methyl-1H-pyrazol-4-yl)phenyl]-5-(4-chlorophenyl)-2-isobutyrylfuro[2,3-b]pyridin-3-yl]pyrrolidin-2-one | 573.3 575.3 3.61 | 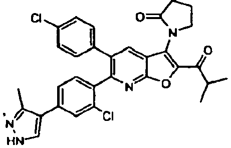 |
| **Example 40** | 1-[3-Acetyl-6-[2-chloro-4-(3-methyl-1 H-pyrazol-4-yl)phenyl]-5-(4-chlorophenyl)furo[2,3-b]pyridin-2-yl]-2,2-dimethylpropan-1-one | 546.2 548.2 3.90 | 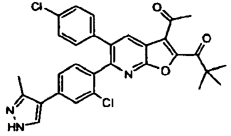 |
| **Example 41** | N-[6-[2-Chloro-4-(3-methyl-1H-pyrazol-4-yl)phenyl]-5-(4-chlorophenyl)-2-(2-hydroxy-2-methylpropanoyl)furo[2,3-b]pyridin-3-yl]acetamide | 563.2 565.2 3.41 | 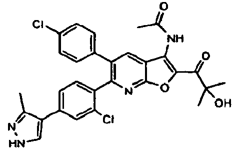 |

### Example 42

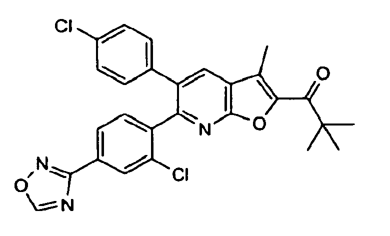

### 1-[6-[2-Chloro-4-(1,2,4-oxadiazol-3-yl)phenyl]-5-(4-chlorophenyl)-3-methylfuro[2,3-b]pyridin-2-yl]-2,2-dimethylpropan-1-one.

Step A: 3-Chloro-4-[5-(4-chlorophenyl)-2-(2,2-dimethylpropanoyl)-3-methylfuro[2,3-*b*]pyridin-6-yl]-*N*'-hydroxybenzenecarboximidamide. The product of Reference Example 14 (55 mg, 0.12 mmol) was dissolved in EtOH (2 mL). Hydroxylamine hydrochloride (10 mg, 0.14 mmol) and NEt₃ (0.35 mL, 0.24 mmol) were added and the reaction was heated to reflux overnight. The reaction mixture was concentrated and re-dissolved in EtOAc and washed with brine, dried (Na₂SO₄), filtered and concentrated. The residue was purified by flash chromatography on silica gel gradient eluted with 0-50% EtOAc in hexane affording the title compound.

Step B: 1-[6-[2-Chloro-4-(1,2,4-oxadiazol-3-yl)phenyl]-5-(4-chlorophenyl)-3-methylfuro[2,3-b]pyridin-2-yl]-2,2-dimethylpropan-1-one To the product from Step A (19 mg, 0.038 mmol) was added trimethyl orthoformate (1 mL) and the reaction was stirred overnight at 100 °C. The reaction was concentrated and purified on a silica gel flash chromatography column eluted with 0-30% EtOAc in hexane affording the title compound. HPLC/MS: 505.8 (M+1), 507.8 (M+3); Rₜ = 4.42 min. ¹H NMR (500 MHz, CHCl₃ -d): δ 8.77 (s, 1H); 8.10 (d, J = 1.57 Hz, 1H); 8.05 (s, 1 H); 8.02 (dd, J = 7.94, 1.60 Hz, 1 H); 7.48 (d, J = 7.97 Hz, 1 H); 7.23-7.20 (m, 2 H); 7.17-7.12 (m, 2 H); 2.66 (s, 3 H); 1.45 (s, 9H).

### Example 43

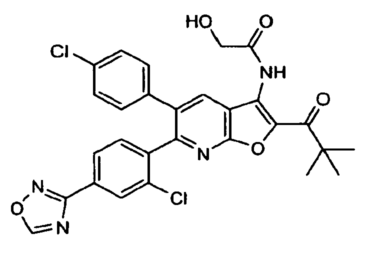

### N-[6-[2-Chloro-4-(1,2,4-oxadiazol-3-yl)phenyl]-5-(4-chlorophenyl)-2-(2,2-dimethylpropanoyl)furo[2,3-b]pyridin-3-yl]-2-hydroxyacetamide

Step A: *N*-[6-[2-Chloro-4-(1,2,4-oxadiazol-3-yl)phenyl]-5-(4-chlorophenyl)-2-(2,2-dimethylpropanoyl)furo[2,3-*b*]pyridin-3-yl]-2-hydroxyacetamide. Using the product of Reference Example 21 and the 2 step procedure described for Example 42 the product was obtained.

Step B: *N*-[6-[2-Chloro-4-(1,2,4-oxadiazol-3-yl)phenyl]-5-(4-chlorophenyl)-2-(2,2-dimethylpropanoyl)furo[2,3-*b*]pyridin-3-yl]-2-hydroxyacetamide. To the product of Step A (20 mg, 0.03 mmol) in CH₂Cl₂ (1 mL) was added a solution of cesium carbonate in MeOH (0.3 M, 6 mg, 0.018 mmol). The reaction was stirred for 30 min, brine was added and the organic layer was extracted, dried (Na₂SO₄), filtered and evaporated. The residue was purified by flash chromatography on silica gel gradient eluted with 0-30% EtOAc in hexane affording the title compound. HPLC/MS: 565.0 (M+1), 567.0 (M+3); Rₜ = 4.23 min. ¹H NMR (500 MHz, CHCl₃ -d): δ 11.41 (s, 1H); 9.12 (s, 1H); 8.80 (s, 1H); 8.13 (s, 1H); 8.04 (d, J = 7.99 Hz, 1 H); 7.49 (d, J = 8.15 Hz, 1 H); 7.23 (d, J = 8.36 Hz, 2 H); 7.17 (d, J = 8.49 Hz, 2 H); 4.43 (s, 2 H); 1.49(s,9H).

### BIOLOGICAL EXAMPLE 1

### Cannabinoid Receptor-1 (CB1) Binding Assay.

Binding affinity determination is based on recombinant human CB1 receptor expressed in Chinese Hamster Ovary (CHO) cells (Felder et al, Mol. Pharmacol. 48: 443-450, 1995). Total assay volume is 250 µl (240 µl CB1 receptor membrane solution plus 5 µl test compound solution plus 5 µl [3H]CP-55940 solution). Final concentration of [3H]CP-55940 is 0.6 nM. Binding buffer contains 50mM Tris-HCl, pH 7.4, 2.5 mM EDTA, 5mM MgCl₂, 0.5mg/mL fatty acid free bovine serum albumin and protease inhibitors (Cat#P8340, from Sigma). To initiate the binding reaction, 5 µl of radioligand solution is added, the mixture is incubated with gentle shaking on a shaker for 1.5 h at 30°C. The binding is terminated by using 96-well harvester and filtering through GF/C filter presoaked in 0.05% polyethylenimine. The bound radiolabel is quantitated using scintillation counter. Apparent binding affinities for various compounds are calculated from IC₅₀ values (DeBlasi et al., Trends Pharmacol Sci 10: 227-229, 1989). Compounds of the present invention have IC50s of less than 5 micromolar in the CB1 binding assay. In particular, compounds of Examples 1 to 43 were assayed in the CB1 Binding assay and found to have IC₅₀ values for the human CB1 receptor less than 1 micromolar.

The binding assay for CB2 receptor is done similarly with recombinant human CB2 receptor expressed in CHO cells. The compounds of the present invention are selective CB I antagonist/inverse agonist compounds having IC50s greater in the CB2 binding assay than in the CB1 assay.

### CB1 Receptor Binding Activity for Selected Compounds

| Example No. | CB1 binding IC₅₀ (nM) |
|---|---|
| 1 | 2 |
| 2 | 6 |
| 3 | 1 |
| 7 | 1 |
| 11 | 1 |
| 37 | 1 |
| 38 | 3 |
| 40 | 1 |
| 41 | 5 |
| 43 | 0.4 |

### BIOLOGICAL EXAMPLE 2

### Cannabinoid Receptor-1 (CB1) Functional Activity Assay.

The functional activation of CB1 receptor is based on recombinant human CB1 receptor expressed in CHO cells (Felder et al, Mol. Pharmacol. 48: 443-450, 1995). To determine the agonist activity or inverse agonist activity of any test compound, 50 ul of CB1-CHO cell suspension are mixed with test compound and 70 ul assay buffer containing 0.34 mM 3-isobutyl-1-methylxanthine and 5.1 uM of forskolin in 96-well plates. The assay buffer is comprised of Earle's Balanced Salt Solution supplemented with 5 mM MgCl₂,1 mM glutamine, 10 mM HEPES, and 1 mg/mL bovine serum albumin. The mixture is incubated at room temperature for 30 minutes, and terminated by adding 30ul/well of 0.5M HCl. The total intracellular cAMP level is quantitated using the New England Nuclear Flashplate and cAMP radioimmunoassay kit.

The compounds of Examples 1, 2, 3, 7, 11, 37, 38, 40, 41, and 43 were all tested in the CB1 functional activity assay and found to have EC50s less than 20 nanomolar.

### BIOLOGICAL EXAMPLE 3

### Cannabinoid Receptor-1 (CB1) Functional Antagonist Assay

To determine the antagonist activity of test compound, the reaction mixture also contains 0.5 nM of the agonist CP55940 (or 50 nM of methanandamide), and the reversal of the CP55940 (or methanandamide) effect is quantitated with increasing concentration of the test compound. Intracellular cAMP is determined as described above. An IC50 value for the test compound is calculated from the titration curve.

Alternatively, a series of dose response curves for the agonist CP55940 (or methanandamide) is performed with increasing concentration of the test compound in each of the dose response curves, and a Schild analysis is carried to calculate the Kb value which is an estimation of test compound binding affinity.

The compounds of Examples 1, 2, 3, 7, 11, 37, 38, 40, 41, and 43 were all tested in the CB1 functional activity assay and were functional inverse agonists.

### BIOLOGICAL EXAMPLE 4

### Cannabinoid Receptor-2 (CB2) Functional Activity Assay.

The functional assay for the CB2 receptor is done similarly with recombinant human CB2 receptor expressed in CHO cells.

### BIOLOGICAL EXAMPLE 5

### GABAa, ³H-muscimol binding assay

Membranes from recombinant Ltk cells expressing human GABA α₁β₃γ₂ receptors were suspended in an ice-cold solution of 100 mM KCl, 10 mM CH₂PO₄, adjusted to pH = 7.4 with 1 M KOH. Tritiated muscimol (PerkinElmer, Wellesley, MA) at a concentration of 2 nM, and serial five-fold dilutions of test compounds at 10 µM, 2.0 µM, 400 nM, 80 nM, 16 nM, 3.2 nM, 0.64 nM and 0.13 nM were mixed in 96-well plates containing 200 µL of membrane suspension. Cold muscimol and compound P18 were used as negative and positive controls respectively. The assay mixture was incubated at 4°C for 2 hours, then harvested on GF/C filter plates and washed with ice-cold 50 mM Tris-Cl buffer. The filter plates were dried and counted in a Wallac MicroBeta® Trilux detector (Model 1450-030; PerkinElmer, Wellesley, MA), and the data were downloaded and analyzed using Prism 3.0. The activity of compound P 18 at 10 µM was used as reference for the test compounds, and it was expressed as increasing ³H-muscimol binding by 100%, whereas cold muscimol at 2 µM antagonized ³H-muscimol binding by 100%. The tested compound's activity at 10 µM was expressed as a percentage of compound P 18's activity at 10 µM.

The following table shows the GABA activity of the Example compounds expressed as a percentage of the activity of control compound P18. As a reference, the parent compound (the compound not substituted with a R⁴ heteroaryl, such as pyrazole or 1,2,4-oxadiazole, as indicated below) is also shown with its Gamma Amino Butyric Acid (GABA) Receptor activity. As is evident from the Examples where comparisons could be made, substitution with a R⁴ heteroaryl, such as a R⁴ pyrazole or 1,2,4-oxadiazole substituent, resulted in an unexpected decrease in the % GABA activity in the compounds of the present invention relative to the % GABA activity of the corresponding parent compounds, which are not substituted with a R⁴ heteroaryl ring. The compounds of the present invention in which R⁴ is a heteroaryl, such as a R⁴ pyrazole or 1,2,4-oxadiazole, are even more selective for the Cannabinoid-1 Receptor and are less active at the GABA receptor than the corresponding parent compounds, which are not substituted with a R⁴ heteroaryl ring. As a result, the compounds of the present invention in which R⁴ is a heteroaryl, such as pyrazole or 1,2,4-oxadiazole, have the unexpected benefits of minimizing off target activity at the GABA receptor.
Note: % GABA is defined as the GABA activity of the indicated compound expressed as a percentage of the activity of control compound P18. NA = data not available.

| Example | Example Structure | % GABA Example | Parent Compound | Parent Compound Structure | % GABA Parent |
|---|---|---|---|---|---|
| Example 1 | 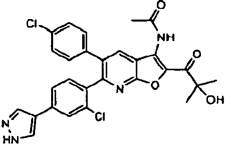 | 38 | P1 | 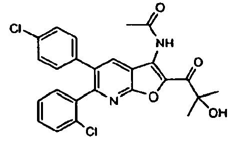 | 99 |
| Example 2 | 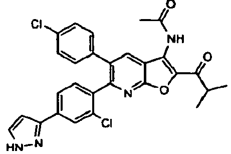 | 17 | P2 | 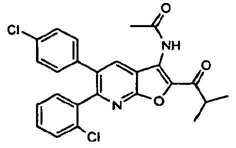 | 107 |
| Example 3 | 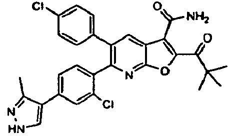 | 9 | P12 | 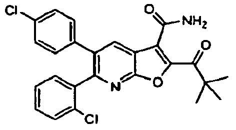 | 40 |
| Example 4 | 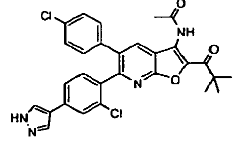 | 12 | P3 | 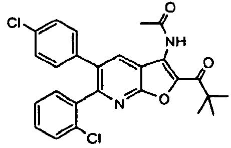 | 67 |
| Example 5 | 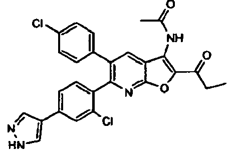 | 11 | P4 | 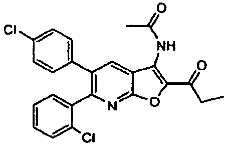 | 76 |
| Example 6 | 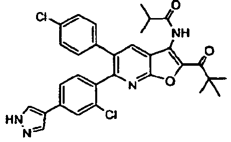 | 3 | NA | NA | NA |
| Example 7 | 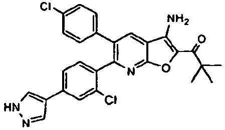 | 17 | P5 | 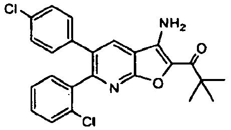 | 33 |
| Example 8 | 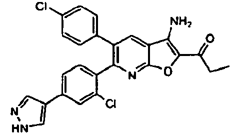 | 26 | P6 | 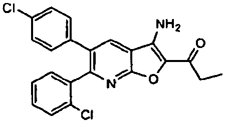 | 141 |
| Example 9 | 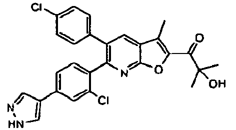 | 30 | P7 | 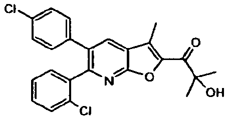 | 75 |
| Example 10 | 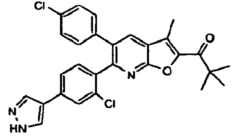 | 9 | P8 | 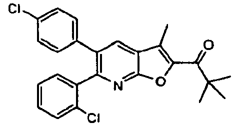 | 25 |
| Example 11 | 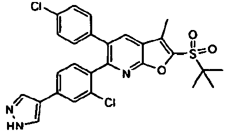 | 7 | P9 | 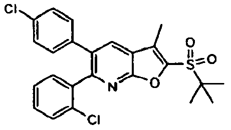 | 29 |
| Example 12 | 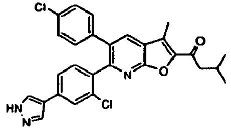 | 51 | P10 | 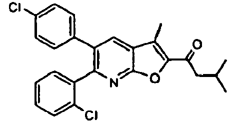 | 59 |
| Example 13 | 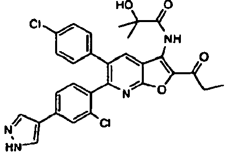 | 15 | NA | NA | NA |
| Example 14 | 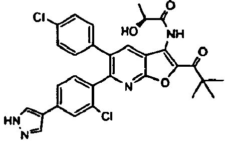 | 13 | P11 | 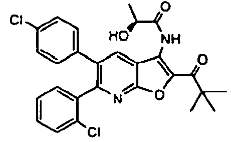 | 94 |
| Example 15 | 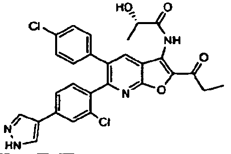 | 13 | NA | NA | NA |
| Example 16 | 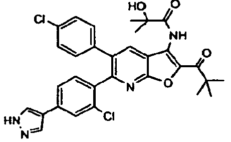 | 11 | NA | NA | NA |
| Example 17 | 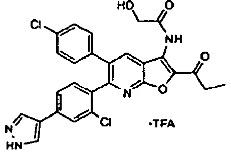 | 36 | NA | NA | NA |
| Example 18 | 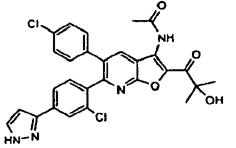 | 26 | P1 | 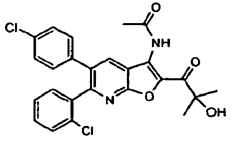 | 99 |
| Example 19 | 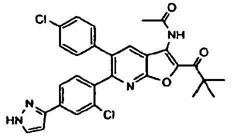 | 16 | P3 | 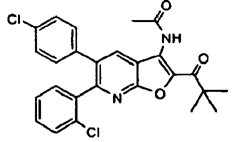 | 67 |
| Example 20 | 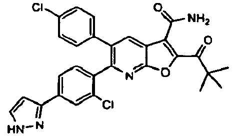 | 13 | P12 | 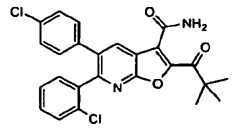 | 40 |
| Example 21 | 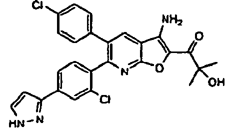 | 27 | P13 | 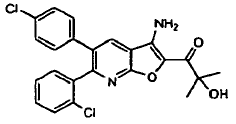 | 91 |
| Example 22 | 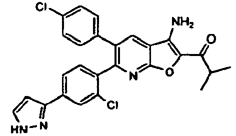 | 18 | P6 | 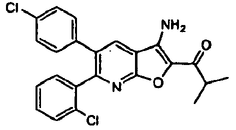 | 141 |
| Example 23 | 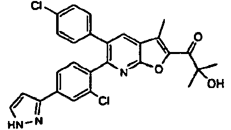 | 24 | P7 | 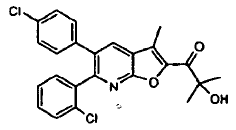 | 75 |
| Example 24 | 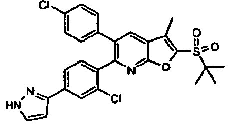 | 13 | P9 | 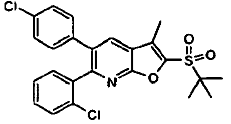 | 29 |
| Example 25 | 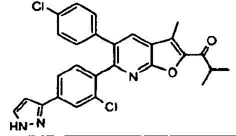 | 35 | P14 | 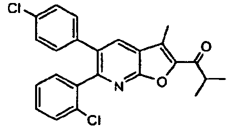 | 51 |
| Example 26 | 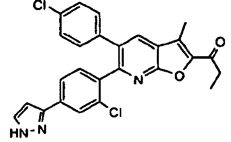 | 28 | P15 | 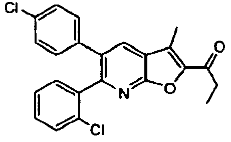 | 50 |
| Example 27 | 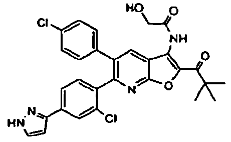 | 29 | P16 | 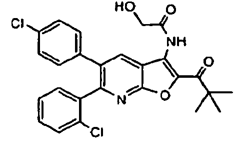 | 90 |
| Example 28 | 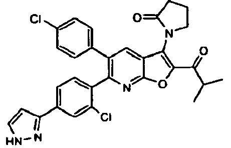 | 16 | P17 | 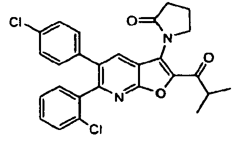 | 100 |
| Example 29 | 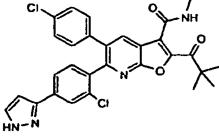 | 20 | NA | NA | NA |
| Example 30 | 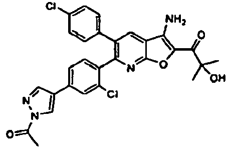 | 19 | P13 | 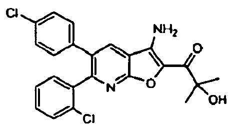 | 91 |
| Example 31 | 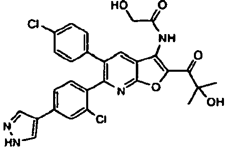 | 30 | P18 | 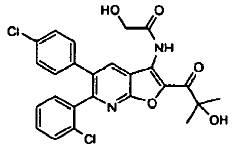 | 100 |
| Example 32 | 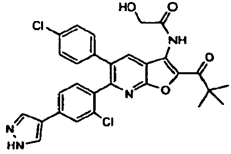 | 29 | P16 | 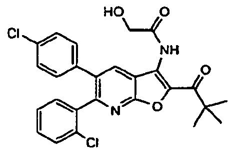 | 90 |
| Example 33 | 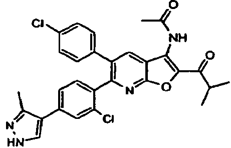 | 34 | P2 | 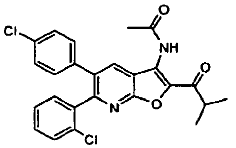 | 107 |
| Example 34 | 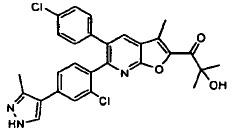 | 29 | P7 | 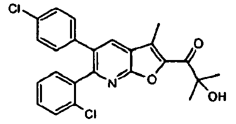 | 75 |
| Example 35 | 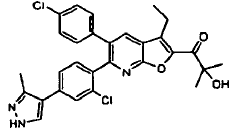 | 59 | P19 | 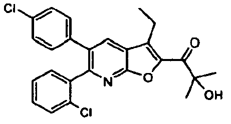 | 96 |
| Example 36 | 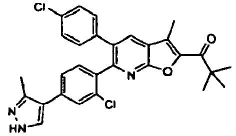 | 16 | P8 | 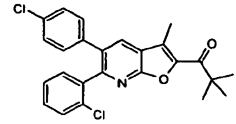 | 25 |
| Example 37 | 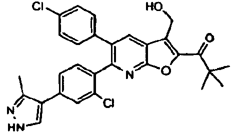 | 14 | P20 | 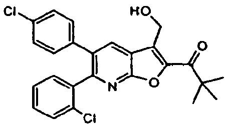 | 50 |
| Example 38 | 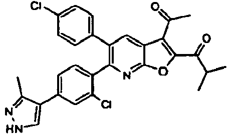 | 39 | NA | NA | NA |
| Example 39 | 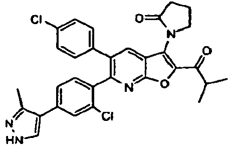 | 29 | P17 | 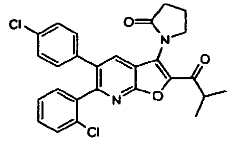 | 100 |
| Example 40 | 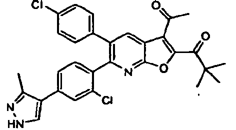 | 12 | P21 | 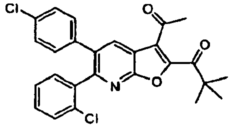 | 62 |
| Example 41 | 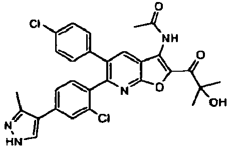 | 35 | P1 | 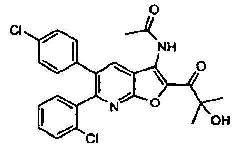 | 99 |
| Example 42 | 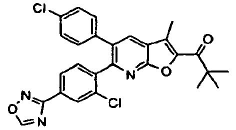 | 5 | P8 | 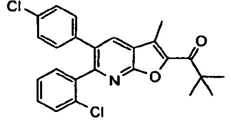 | 25 |
| Example 43 | 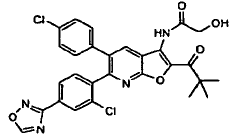 | 41 | P16 | 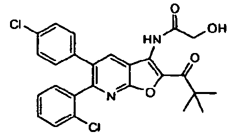 | 90 |

### BIOLOGICAL EXAMPLE 6

### Acute food intake studies in rats or mice: General Procedure

Adult rats or mice are used in these studies. After at least 2 days of acclimation to the vivarium conditions (controlled humidity and temperature, lights on for 12 hours out of 24 hours) food is removed from rodent cages. Experimental compounds or their vehicles are administered orally, intraperitoneally, subcutaneously or intravenously before the return of a known amount of food to cage. The optimal interval between dosing and food presentation is based on the half-life of the compound based on when brain concentrations of the compound is the highest. Food remaining is measured at several intervals. Food intake is calculated as grams of food eaten per gram of body weight within each time interval and the appetite-suppressant effect of the compounds are compared to the effect of vehicle. In these experiments many strains of mouse or rat, and several standard rodent chows can be used.

### BIOLOGICAL EXAMPLE 7

### Chronic weight reduction studies in rats or mice: General Procedure

Adult rats or mice are used in these studies. Upon or soon after weaning, rats or mice are made obese due to exclusive access to diets containing fat and sucrose in higher proportions than in the control diet. The rat strains commonly used include the Sprague Dawley bred through Charles River Laboratories. Although several mouse strains may be used, c57B1/6 mice are more prone to obesity and hyperinsulinemia than other strains. Common diets used to induce obesity include: Research Diets D12266B (32% fat) or D12451 (45% fat) and BioServ S3282 (60% fat). The rodents ingest chow until they are significantly heavier and have a higher proportion of body fat than control diet rats, often 9 weeks. The rodents receive injections (1 to 4 per day) or continuous infusions of experimental compounds or their vehicles either orally, intraperitoneally, subcutaneously or intravenously. Food intake and body weights are measured daily or more frequently. Food intake is calculated as grams of food eaten per gram of body weight within each time interval and the appetite-suppressant and weight loss effects of the compounds are compared to the effects of vehicle.

## Claims

1. A compound of structural formula I: 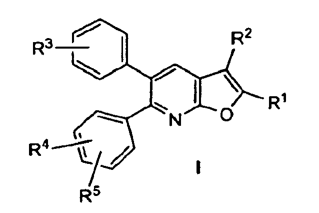 or a pharmaceutically acceptable salt thereof, wherein:
R1 is selected from:
(1) phenyl,
(2) heteroaryl,
(3) -C(O)R^{a},
(4) -C(O)OR^{a},
(5) -C(O)NR^{b}R^{c}, and
(6) -S(O)₂R^{a},
wherein each phenyl and heteroaryl is unsubstituted or substituted with one to four substituents independently selected from -OH, -C₁₋₆alkyl, and halogen, and wherein R^{b} and R^{c} together with the atoms to which they are attached may form a 4-10 membered aromatic or non-aromatic mono- or bicyclic ring, wherein the 4-10 membered ring is unsubstituted or substituted with one to four substituents independently selected from -OH, -C₁₋₆alkyl, and halogen;
R2 is selected from:
(1) C₁₋₁₀alkyl,
(2) C₃₋₁₀cycloalkyl,
(3) cycloheteroalkyl,
(4) phenyl,
(5) heteroaryl,
(6) -C(O)C₁₋₁₀alkyl,
(7) -C(O)OR^{a},
(8) -C(O)N(R^{b})₂,
(9) -N(Rb)₂, and
(10) -NR^{d}C(O)C₁₋₁₀alkyl,
wherein each alkyl, cycloalkyl, cycloheteroalkyl, phenyl and heteroaryl is unsubstituted or substituted with one to four substituents independently selected from -OH, C₁₋₆alkyl, and oxo;
R3 is selected from:
(1) hydrogen,
(2) C₁₋₁₀alkyl,
(3) halogen,
(4) -CN,
(5) -CF₃,
(6) -OCF₃,
(7) -C(O)C₁₋₄alkyl,
(8) -C(O)OC₁₋₄alkyl,
(9) -OC₁₋₄alkyl, and
(10) -SC₁₋₄alkyl;
R4 is selected from:
(1) pyrazole,
(2) oxadiazole,
(3) triazole,
(4) isoxazole,
(5) isothiazole, and
(6) thiadiazole, wherein each pyrazole, oxadiazole, triazole, isoxazole, isothiazole and thiadiazole is unsubstituted or substituted with one or three substituents selected from R⁶ and R⁷;
R⁵ is selected from:
(1) hydrogen,
(2) C₁₋₁₀alkyl,
(3) halogen,
(4) -CN,
(5) -CF₃,
(6) -OCF₃,
(7) -C(O)C₁₋₄alkyl,
(8) -C(O)OC₁₋₄alkyl,
(9) -OC₁₋₄alkyl, and
(10) -SC₁₋₄alkyl;
R6 is selected from:
(1) hydrogen,
(2) C₁₋₁₀alkyl,
(3) halogen,
(4) -CN,
(5) -C(O)C₁₋₆alkyl,
(6) -OC₁₋₆alkyl,
(7) -OCF₃, and
(8) -SC₁₋₆alkyl;
R⁷ is selected from:
(1) hydrogen,
(2) C₁₋₆alkyl, and
(3) C(O)C₁₋₁₀alkyl;
R^{a} is selected from:
(1) C₁₋₆alkyl, and
(2) C₃₋₇cycloalkyl,
wherein each alkyl and cycloalkyl is unsubstituted or substituted with one to four substituents independently selected from -OH, -C₁₋₆alkyl, and halogen;
R^{b} is selected from:
(1) hydrogen,
(2) C₁₋₆alkyl, and
(3) phenyl,
wherein each alkyl and phenyl is unsubstituted or substituted with one to four substituents independently selected from -OH, -C₁₋₆alkyl, and halogen;
R^{c} is selected from:
(1) C₁₋₆alkyl, and
(2) phenyl,
wherein each alkyl and phenyl is unsubstituted or substituted with one to four substituents independently selected from -OH, -C₁₋₆alkyl, and halogen; and
R^{d} is selected from:
(1) hydrogen, and
(2) C₁₋₆alkyl,
wherein each alkyl is unsubstituted or substituted with one to four substituents independently selected from -OH, -C₁₋₆alkyl, and halogen.

2. The compound according to Claim 1, wherein R⁴ is selected from: pyrazole and oxadiazole, wherein each pyrazole and oxadiazole is unsubstituted or substituted with one or two substituents selected from R⁶ and R⁷; or a pharmaceutically acceptable salt thereof.

3. The compound according to Claim 2, wherein R⁴ is pyrazole, wherein pyrazole is unsubstituted or substituted with one or two substituents selected from R⁶ and R⁷; or a pharmaceutically acceptable salt thereof.

4. The compound according to Claim 1, wherein R¹ is selected from: - C(O)Ra, and -S(O)₂R^{a}; or a pharmaceutically acceptable salt thereof.

5. The compound according to Claim 1, wherein R¹ is selected from: - C(O)C(CH₃)₂OH, -C(O)CH(CH₃)₂, and -C(O)C(CH₃)₃; or a pharmaceutically acceptable salt thereof.

6. The compound according to Claim 1, wherein R² is selected from:
(1) C₁₋₁₀alkyl,
(2) cycloheteroalkyl,
(3) -C(O)C₁₋₁₀alkyl,
(4) -C(O)N(R^{b})₂,
(5) -N(R^{b})₂, and
(6) -NR^{d}C(O)C₁₋₁₀alkyl,
wherein each alkyl and cycloheteroalkyl is unsubstituted or substituted with one to four substituents independently selected from -OH and oxo; or a pharmaceutically acceptable salt thereof.

7. The compound according to Claim 1, wherein R² is selected from: - CH₂OH, -C(O)CH₃, -C(O)NH₂, and -NHC(O)CH₃; or a pharmaceutically acceptable salt thereof.

8. The compound according to Claim 1, wherein R³ is halogen; or a pharmaceutically acceptable salt thereof.

9. The compound according to Claim 1, wherein R⁵ is halogen; or a pharmaceutically acceptable salt thereof.

10. The compound according to Claim 1 of structural formula IE: 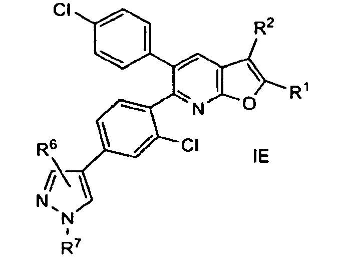 or a pharmaceutically acceptable salt thereof, wherein:
R¹ is selected from: -C(O)C(CH₃)₂OH, -C(O)CH(CH₃)₂, and -C(O)C(CH₃)₃;
R2 is selected from: C₁₋₁₀alkyl, -C(O)C₁₋₁₀alkyl, -C(O)N(R^{b})₂, and -NR^{d}C(O)C₁₋₁₀alkyl,
wherein each alkyl is unsubstituted or substituted with one to four substituents independently selected from -OH;
R6 is hydrogen or methyl; and
R⁷ is hydrogen.

11. The compound according to Claim 1, selected from:
(1) *N*-[5-(4-chlorophenyl)-6-[2-chloro-4-(1*H*-pyrazol-4-yl)phenyl]-2-(2-hydroxy-2-methylpropanoyl)furo[2,3-*b*]pyridin-3-yl]acetamide;
(2) *N*-{5-(4-chlorophenyl)-6-[2-chloro-4-(1*H*-pyrazol-3-yl)phenyl]-2-isobutyrylfuro[2,3-*b*]pyridin-3-yl}acetamide;
(3) *N*-[5-(4-chlorophenyl)-6-[2-chloro-4-(1*H*-pyrazol-4-yl)phenyl]-2-(2,2-dimethylpropanoyl)furo[2,3-*b*]pyridin-3-yl]acetamide;
(4) *N*-{5-(4-chlorophenyl)-6-[2-chloro-4-(1*H*-pyrazol-4-yl)phenyl]-2-propionylfuro[2,3-*b*]pyridin-3-yl}acetamide;
(5) *N*-[5-(4-chlorophenyl)-6-[2-chloro-4-(1*H*-pyrazol-4-yl)phenyl]-2-(2,2-dimethylpropanoyl)furo[2,3-*b*]pyridin-3-yl]-2-methylpropanamide;
(6) 1-{3-amino-5-(4-chlorophenyl)-6-[2-chloro-4-(1*H*-pyrazol-4-yl)phenyl]furo[2,3-*b*]pyridin-2-yl}-2,2-dimethylpropan-1-one;
(7) 1-{3-amino-5-(4-chlorophenyl)-6-[2-chloro-4-(1*H*-pyrazol-4-yl)phenyl]furo[2,3*-b*]pyridin-2-yl}propan-1-one;
(8) 1-{5-(4-chlorophenyl)-6-[2-chloro-4-(1*H*-pyrazol-4-yl)phenyl]-3-methylfuro[2,3*-b*]pyridin-2-yl}-2-hydroxy-2-methylpropan-1-one;
(9) 1-{5-(4-chlorophenyl)-6-[2-chloro-4-(1*H*-pyrazol-4-yl)phenyl]-3-methylfuro[2,3*-b*]pyridin-2-yl}-2,2-dimethylpropan-1-one;
(10) 2-(*tert*-butylsulfonyl)-5-(4-chlorophenyl)-6-[2-chloro-4-(1*H*-pyrazol-4-yl)phenyl]-3-methylfuro[2,3*-b*]pyridine;
(11) 1-{5-(4-chlorophenyl)-6-[2-chloro-4-(1*H*-pyrazol-4-yl)phenyl]-3-methylfuro[2,3-*b*]pyridin-2-yl}-3-methylbutan-1-one;
(12) *N*-{5-(4-chlorophenyl)-6-[2-chloro-4-(1*H*-pyrazol-4-yl)phenyl]-2-propionylfuro[2,3-b]pyridin-3-yl}-2-hydroxy-2-methylpropanamide;
(13) (2S)-*N*-[5-(4-chlorophenyl)-6-[2-chloro-4-(1*H*-pyrazol-4-yl)phenyl]-2-(2,2-dimethylpropanoyl)furo[2,3-*b*]pyridin-3-yl]-2-hydroxypropanamide;
(14) (2*S*)-*N*-{5-(4-chlorophenyl)-6-[2-chloro-4-(1*H*-pyrazol-4-yl)phenyl]-2-propionylfuro[2,3-b]pyridin-3-yl}-2-hydroxypropanamide;
(15) *N*-[5-(4-chlorophenyl)-6-[2-chloro-4-(1*H*-pyrazol-4-yl)phenyl]-2-(2,2-dimethylpropanoyl)furo[2,3-*b*]pyridin-3-yl]-2-hydroxy-2-methylpropanamide;
(16) *N*-{5-(4-chlorophenyl)-6-[2-chloro-4-(1H-pyrazol-4-yl)phenyl]-2-propionylfuro[2,3-b]pyridin-3-yl}-2-hydroxyacetamide;
(17) *N*-[5-(4-chlorophenyl)-6-[2-chloro-4-(1*H*-pyrazol-3-yl)phenyl]-2-(2-hydroxy-2-methylpropanoyl)furo[2,3-*b*]pyridin-3-yl]acetamide;
(18) *N*-[5-(4-chlorophenyl)-6-[2-chloro-4-(1*H*-pyrazol-3-yl)phenyl]-2-(2,2-dimethylpropanoyl)furo[2,3-*b*]pyridin-3-yl]acetamide;
(19) 5-(4-chlorophenyl)-6-[2-chloro-4-(1H-pyrazol-3-yl)phenyl]-2-(2,2-dimethylpropanoyl)furo[2,3-*b*]pyridine-3-carboxamide;
(20) 1-{3-amino-5-(4-chlorophenyl)-6-[2-chloro-4-(1*H*-pyrazol-3-yl)phenyl]furo[2,3-*b*]pyridin-2-yl} -2-hydroxy-2-methylpropan-1-one;
(21) 1-{3-amino-5-(4-chlorophenyl)-6-[2-chloro-4-(1*H*-pyrazol-3-yl)phenyl]furo-[2,3-b]pyridin-2-yl}-2-methylpropan-1-one;
(22) 1-{5-(4-chlorophenyl)-6-[2-chloro-4-(1*H*-pyrazol-3-yl)phenyl]-3-methylfuro[2,3-*b*]pyridin-2-yl}-2-hydroxy-2-methylpropan-1-one;
(23) 2-(*tert*-butylsulfonyl)-5-(4-chlorophenyl)-6-[2-chloro-4-(1*H*-pyrazol-3-yl)phenyl]-3-methylfuro[2,3-*b*]pyridine;
(24) 1-{5-(4-chlorophenyl)-6-[2-chloro-4-(1*H*-pyrazol-3-yl)phenyl]-3-methylfuro[2,3-*b*]pyridin-2-yl} -2-methylpropan-1-one;
(25) 1-{5-(4-chlorophenyl)-6-[2-chloro-4-(1*H*-pyrazol-3-yl)phenyl]-3-methylfuro[2,3-*b*]pyridin-2-yl}propan-1-one;
(26) *N*-[5-(4-chlorophenyl)-6-[2-chloro-4-(3*H*-pyrazol-5-yl)phenyl]-2-(2,2-dimethylpropanoyl)furo[2,3-*b*]pyridin-3-yl]-2-hydroxyacetamide;
(27) 1-{5-(4-chlorophenyl)-6-[2-chloro-4-(1H-pyrazol-3-yl)phenyl]-2-isobutyrylfuro[2,3-b]pyridin-3-yl}pyrrolidin-2-one;
(28) 5-(4-chlorophenyl)-6-[2-chloro-4-(1H-pyrazol-3-yl)phenyl]-2-(2,2-dimethylpropanoyl)-N-methylfuro[2,3-b]pyridine-3-carboxamide;
(29) 1-[6-[4-(1-acetyl-1*H*-pyrazol-4-yl)-2-chlorophenyl]-3-amino-5-(4-chlorophenyl)furo[2,3-*b*]pyridin-2-yl]-2-hydroxy-2-methylpropan-1-one;
(30) *N*-[5-(4-chlorophenyl)-6-[2-chloro-4-(1*H*-pyrazol-4-yl)phenyl]-2-(2-hydroxy-2-methylpropanoyl)-furo[2,3-*b*]pyridin-3-yl]-2-hydroxyacetamide;
(31) *N*-[5-(4-chlorophenyl)-6-[2-chloro-4-(1*H*-pyrazol-4-yl)phenyl]-2-(2,2-dimethylpropanoyl)furo[2,3-*b*]pyridin-3-yl]-2-hydroxyacetamide;
(32) N-[6-[2-chloro-4-(3-methyl-1H-pyrazol-4-yl)phenyl]-5-(4-chlorophenyl)-2-isobutyrylfuro[2,3-b]pyridin-3-yl]acetamide;
(33) 1-[6-[2-chloro-4-(3-methyl-1H-pyrazol-4-yl)phenyl]-5-(4-chlorophenyl)-3-methylfuro[2,3-b]pyridin-2-yl]-2-hydroxy-2-methylpropan-1-one;
(34) 1-[6-[2-chloro-4-(3-methyl-1H-pyrazol-4-yl)phenyl]-5-(4-chlorophenyl)-3-ethylfuro[2,3-b]pyridin-2-yl]-2-hydroxy-2-methylpropan-1-one;
(35) 1-[6-[2-chloro-4-(3-methyl-1H-pyrazol-4-yl)phenyl]-5-(4-chlorophenyl)-3-methylfuro[2,3-b]pyridin-2-yl]-2,2-dimethylpropan-1-one;
(36) 1-[6-[2-chloro-4-(3-methyl-1H-pyrazol-4-yl)phenyl]-5-(4-chlorophenyl)-3-(hydroxymethyl)furo[2,3-b]pyridin-2-yl]-2,2-dimethylpropan-1-one;
(37) 1-[3-acetyl-6-[2-chloro-4-(3-methyl-1H-pyrazol-4-yl)phenyl]-5-(4-chlorophenyl)furo[2,3-b]pyridin-2-yl]-2-methylpropan-1-one;
(38) 1-[6-[2-chloro-4-(3-methyl-1H-pyrazol-4-yl)phenyl]-5-(4-chlorophenyl)-2-isobutyrylfuro[2,3-b]pyridin-3-yl]pyrrolidin-2-one;
(39) 1-[3-acetyl-6-[2-chloro-4-(3-methyl-1H-pyrazol-4-yl)phenyl]-5-(4-chlorophenyl)furo[2,3-b]pyridin-2-yl]-2,2-dimethylpropan-1-one;
(40) *N*-[6-[2-chloro-4-(3-methyl-1H-pyrazol-4-yl)phenyl]-5-(4-chlorophenyl)-2-(2-hydroxy-2-methylpropanoyl)furo[2,3-b]pyridin-3-yl]acetamide;
(41) 1-[6-[2-chloro-4-(1,2,4-oxadiazol-3-yl)phenyl]-5-(4-chlorophenyl)-3-methylfuro[2,3-b]pyridin-2-yl]-2,2-dimethylpropan-1-one; and
(42) *N*-[6-[2-chloro-4-(1,2,4-oxadiazol-3-yl)phenyl]-5-(4-chlorophenyl)-2-(2,2-dimethylpropanoyl)furo[2,3-*b*]pyridin-3-yl]-2-hydroxyacetamide;
or a pharmaceutically acceptable salt thereof.

12. The compound according to Claim 1 which is: 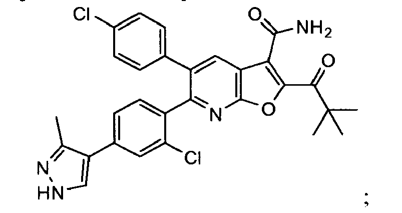 or a pharmaceutically acceptable salt thereof, or 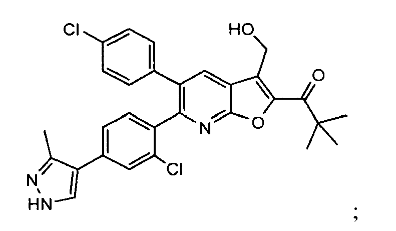 or a pharmaceutically acceptable salt thereof, or 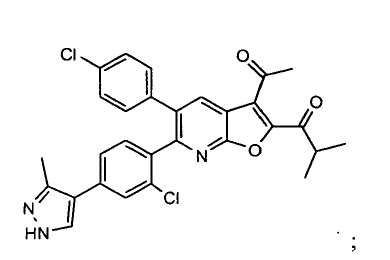 or a pharmaceutically acceptable salt thereof, or 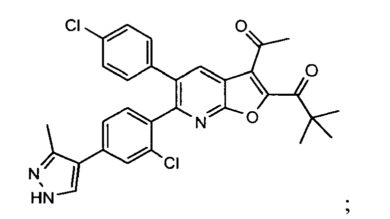 or a pharmaceutically acceptable salt thereof, or 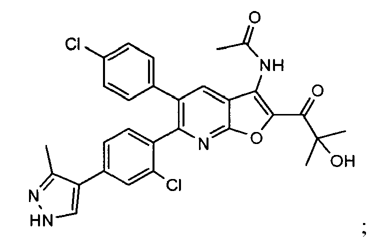 or a pharmaceutically acceptable salt thereof.

13. The compound according to Claim 1 **characterized** as being the crystalline 1:1 ethanol solvate polymorphic form I of 6-[2-chloro-4-(3-methyl-1*H*-pyrazol-4-yl)phenyl]-5-(4-chlorophenyl)-2-(2,2-dimethylpropanoyl)furo[2,3-*b*]pyridine-3-carboxamide.

14. A composition comprising a compound according to any previous claim and optionally a compound selected from simvastatin, ezetimibe and sitagliptin; and a pharmaceutically acceptable carrier.

15. The use of a compound according to any of claims 1-13 for the preparation of a medicament useful for the treatment of a condition selected from: psychosis, memory deficit, cognitive disorders, Alzheimer's disease, migraine, neuropathy, neuro-inflammatory disorders, cerebral vascular accidents, head trauma, anxiety disorders, stress, epilepsy, Parkinson's disease, schizophrenia, substance abuse disorders, constipation, chronic intestinal pseudo-obstruction, cirrhosis of the liver, asthma, obesity, and other eating disorders associated with excessive food intake.

16. The use of a compound according to any of claims 1-13 for the manufacture of a medicament useful for preventing obesity in a person at risk for obesity.

## Patentansprüche

1. Eine Verbindung der Strukturformel I: 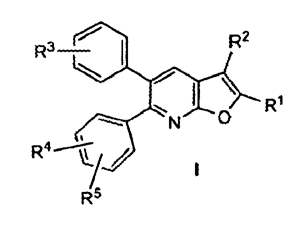 oder ein pharmazeutisch annehmbares Salz davon, wobei:
R¹ ausgewählt ist aus:
(1) Phenyl,
(2) Heteroaryl,
(3) -C(O)R^{a},
(4) -C(O)OR^{a},
(5) -C(O)NR^{b}R^{c} und
(6) -S(O)₂R^{a},
wobei jedes Phenyl und Heteroaryl unsubstituiert oder substituiert ist mit einem bis vier Substituenten, unabhängig ausgewählt aus -OH, -C₁₋₆-Alkyl und Halogen, und wobei R^{b} und R^{c} zusammen mit den Atomen, an die sie gebunden sind, einen 4-10-gliedrigen aromatischen oder nichtaromatischen mono- oder bicyclischen Ring bilden können, wobei der 4-10-gliedrige Ring unsubstituiert oder substituiert ist mit einem bis vier Substituenten, unabhängig ausgewählt aus -OH, -C₁₋₆-Alkyl und Halogen,
R² ausgewählt ist aus:
(1) C₁₋₁₀-Alkyl,
(2) C₃₋₁₀-Cycloalkyl,
(3) Cycloheteroalkyl,
(4) Phenyl,
(5) Heteroaryl,
(6) -C(O)C₁₋₁₀-Alkyl,
(7) -C(O)OR^{a},
(8) -C(O)N(R^{b})₂,
(9) -N(R^{b})₂ und
(10) -NR^{d}C(O)C₁₋₁₀-Alkyl,
wobei jedes Alkyl, Cycloalkyl, Cycloheteroalkyl, Phenyl und Heteroaryl unsubstituiert oder substituiert ist mit einem bis vier Substituenten, unabhängig ausgewählt aus -OH, C₁₋₆-Alkyl und Oxo,
R³ ausgewählt ist aus:
(1) Wasserstoff,
(2) C₁₋₁₀-Alkyl,
(3) Halogen,
(4) -CN,
(5) -CF₃,
(6) -OCF₃,
(7) -C(O)C₁₋₄-Alkyl,
(8) -C(O)OC₁₋₄-Alkyl,
(9) -OC₁₋₄-Alkyl und
(10) -SC₁₋₄-Alkyl,
R⁴ ausgewählt ist aus:
(1) Pyrazol,
(2) Oxadiazol,
(3) Triazol,
(4) Isoxazol,
(5) Isothiazol und
(6) Thiadiazol, wobei jedes Pyrazol, Oxadiazol, Triazol, Isoxazol, Isothiazol und Thiadiazol unsubstituiert oder substituiert ist mit einem bis drei Substituenten, ausgewählt aus R⁶ und R⁷, R⁵ ausgewählt ist aus:
(1) Wasserstoff,
(2) C₁₋₁₀-Alkyl,
(3) Halogen,
(4) -CN,
(5) -CF₃,
(6) -OCF₃,
(7) -C(O)C₁₋₄-Alkyl,
(8) -C(O)OC₁₋₄-Alkyl,
(9) -OC₁₋₄-Alkyl und
(10) -SC₁₋₄-Alkyl,
R⁶ ausgewählt ist aus:
(1) Wasserstoff,
(2) C₁₋₁₀-Alkyl,
(3) Halogen,
(4) -CN,
(5) -C(O)C₁₋₆-Alkyl,
(6) -OC₁₋₆-Alkyl,
(7) -OCF₃ und
(8) -SC₁₋₆-Alkyl,
R⁷ ausgewählt ist aus:
(1) Wasserstoff,
(2) C₁₋₆-Alkyl und
(3) C(O)C₁₋₁₀-Alkyl,
R^{a} ausgewählt ist aus:
(1) C₁₋₆-Alkyl und
(2) C₃₋₇-Cycloalkyl,
wobei jedes Alkyl und Cycloalkyl unsubstituiert oder substituiert ist mit einem bis vier Substituenten, unabhängig ausgewählt aus -OH, -C₁₋₆-Alkyl und Halogen,
R^{b} ausgewählt ist aus:
(1) Wasserstoff,
(2) C₁₋₆-Alkyl und
(3) Phenyl,
wobei jedes Alkyl und Phenyl unsubstituiert oder substituiert ist mit einem bis vier Substituenten, unabhängig ausgewählt aus -OH, -C₁₋₆-Alkyl und Halogen,
R^{c} ausgewählt ist aus:
(1) C₁₋₆-Alkyl und
(2) Phenyl,
wobei jedes Alkyl und Phenyl unsubstituiert oder substituiert ist mit einem bis vier Substituenten, unabhängig ausgewählt aus -OH, -C₁₋₆-Alkyl und Halogen, und
R^{d} ausgewählt ist aus:
(1) Wasserstoff und
(2) C₁₋₆-Alkyl,
wobei jedes Alkyl unsubstituiert oder substituiert ist mit einem bis vier Substituenten, unabhängig ausgewählt aus -OH, -C₁₋₆-Alkyl und Halogen.

2. Die Verbindung gemäß Anspruch 1, wobei R⁴ ausgewählt ist aus: Pyrazol und Oxadiazol, wobei jedes Pyrazol und Oxadiazol unsubstituiert oder substituiert ist mit einem oder zwei Substituenten, ausgewählt aus R⁶ und R⁷, oder ein pharmazeutisch annehmbares Salz davon.

3. Die Verbindung gemäß Anspruch 2, wobei R⁴ Pyrazol ist, wobei Pyrazol unsubstituiert oder substituiert ist mit einem oder zwei Substituenten, ausgewählt aus R⁶ und R⁷, oder ein pharmazeutisch annehmbares Salz davon.

4. Die Verbindung gemäß Anspruch 1, wobei R¹ ausgewählt ist aus: -C(O)R^{a} und -S(O)₂R^{a}, oder ein pharmazeutisch annehmbares Salz davon.

5. Die Verbindung gemäß Anspruch 1, wobei R¹ ausgewählt ist aus: -C(O)C(CH₃)₂OH, -C(O)CH(CH₃)₂ und -C(O)C(CH₃)₃, oder ein pharmazeutisch annehmbares Salz davon.

6. Die Verbindung gemäß Anspruch 1, wobei R² ausgewählt ist aus:
(1) C₁₋₁₀-Alkyl,
(2) Cycloheteroalkyl,
(3) -C(O)C₁₋₁₀-Alkyl,
(4) -C(O)N(R^{b})₂,
(5) -N(R^{b})₂ und
(6) -NR^{d}C(O)C₁₋₁₀-Alkyl,
wobei jedes Alkyl und Cycloheteroalkyl unsubstituiert oder substituiert ist mit einem bis vier Substituenten, unabhängig ausgewählt aus -OH und Oxo, oder ein pharmazeutisch annehmbares Salz davon.

7. Die Verbindung gemäß Anspruch 1, wobei R² ausgewählt ist aus: -CH₂OH, -C(O)CH₃, - C(O)NH₂ und -NHC(O)CH₃, oder ein pharmazeutisch annehmbares Salz davon.

8. Die Verbindung gemäß Anspruch 1, wobei R³ Halogen ist, oder ein pharmazeutisch annehmbares Salz davon.

9. Die Verbindung gemäß Anspruch 1, wobei R⁵ Halogen ist, oder ein pharmazeutisch annehmbares Salz davon.

10. Die Verbindung gemäß Anspruch 1 der Strukturformel IE: 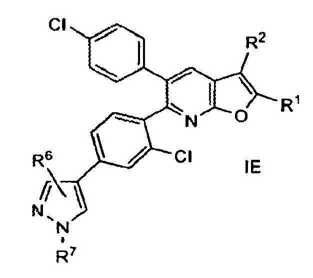 oder ein pharmazeutisch annehmbares Salz davon, wobei:
R¹ ausgewählt ist aus: -C(O)C(CH₃)₂OH, -C(O)CH(CH₃)₂ und -C(O)C(CH₃)₃,
R² ausgewählt ist aus: C₁₋₁₀-Alkyl, -C(O)C₁₋₁₀-Alkyl, -C(O)N(R^{b})₂ und -NR^{d}C(O)C₁₋₁₀-Alkyl, wobei jedes Alkyl unsubstituiert oder substituiert ist mit einem bis vier Substituenten, unabhängig ausgewählt aus -OH,
R⁶ Wasserstoff oder Methyl ist und
R⁷ Wasserstoff ist.

11. Die Verbindung gemäß Anspruch 1, ausgewählt aus:
(1) *N*-[5-(4-Chlorphenyl)-6-[2-chlor-4-(1*H*-pyrazol-4-yl)phenyl]-2-(2-hydroxy-2-methylpropanoyl)furo[2,3-*b*]pyridin-3-yl]acetamid,
(2) *N*-{5-(4-Chlorphenyl)-6-[2-chlor-4-(1*H*-pyrazol-3-yl)phenyl]-2-isobutyrylfuro[2,3-*b*]pyridin-3-yl}acetamid,
(3) *N*-[5-(4-Chlorphenyl)-6-[2-chlor-4-(1*H*-pyrazol-4-yl)phenyl]-2-(2,2-dimethylpropanoyl)furo[2,3-b]pyridin-3-yl]acetamid,
(4) *N*-{5-(4-Chlorphenyl)-6-[2-chlor-4-(1*H*-pyrazol-4-yl)phenyl]-2-propionylfuro[2,3-*b*]pyridin-3-yl}acetamid,
(5) *N*-[5-(4-Chlorphenyl)-6-[2-chlor-4-(1*H*-pyrazol-4-yl)phenyl]-2-(2,2-dimethylpropanoyl)furo[2,3-b]pyridin-3-yl]-2-methylpropanamid,
(6) 1-{3-Amino-5-(4-chlorphenyl)-6-[2-chlor-4-(1*H*-pyrazol-4-yl)phenyl]furo[2,3-*b*]pyridin-2-yl}-2,2-dimethylpropan-1-on,
(7) 1-{3-Amino-5-(4-chlorphenyl)-6-[2-chlor-4-(1*H*-pyrazol-4-yl)phenyl]furo[2,3-*b*]pyridin-2-yl{propan-1-on,
(8) 1-{5-(4-Chlorphenyl)-6-[2-chlor-4-(1*H*-pyrazol-4-yl)phenyl]-3-methylfuro[2,3-*b*]pyridin-2-yl}-2-hydroxy-2-methylpropan-1-on,
(9) 1-{5-(4-Chlorphenyl)-6-[2-chlor-4-(1*H*-pyrazol-4-yl)phenyl]-3-methylfuro[2,3-*b*]pyridin-2-yl}-2,2-dimethylpropan-1-on,
(10) 2-(*tert*-Butylsulfonyl)-5-(4-chlorphenyl)-6-[2-chlor-4-(1*H*-pyrazol-4-yl)phenyl]-3-methylfuro[2,3-b]pyridin,
(11) 1-{5-(4-Chlorphenyl)-6-[2-chlor-4-(1*H*-pyrazol-4-yl)phenyl]-3-methylfuro[2,3-*b*]pyridin-2-yl}-3-methylbutan-1-on,
(12) *N*-{5-(4-Chlorphenyl)-6-[2-chlor-4-(1*H*-pyrazol-4-yl)phenyl]-2-propionylfuro[2,3-*b*]pyridin-3-yl}-2-hydroxy-2-methylpropanamid,
(13) (2*S*)-*N*-[5-(4-Chlorphenyl)-6-[2-chlor-4-(1*H*-pyrazol-4-yl)phenyl]-2-(2,2-dimethylpropanoyl)furo[2,3-*b*]pyridin-3-yl]-2-hydroxypropanamid,
(14) (2*S*)-*N*-{5-(4-Chlorphenyl)-6-[2-chlor-4-(1*H*-pyrazol-4-yl)phenyl]-2-propionylfuro[2,3-*b*]pyridin-3-yl}-2-hydroxypropanamid,
(15) *N*-[5-(4-Chlorphenyl)-6-[2-chlor-4-(1*H*-pyrazol-4-yl)phenyl]-2-(2,2-dimethylpropanoyl)furo[2,3-b]pyridin-3-yl]-2-hydroxy-2-methylopropanamid,
(16) N-{5-(4-Chlorphenyl)-6-[2-chlor-4-(1*H*-pyrazol-4-yl)phenyl]-2-propionylfuro[2,3-b]pyridin-3-yl}-2-hydroxyacetamid,
(17) *N*-[5-(4-Chlorphenyl)-6-[2-chlor-4-(1*H*-pyrazol-3-yl)phenyl]-2-(2-hydroxy-2-methylpropanoyl)furo[2,3-*b*]pyridin-3-yl]acetamid,
(18) *N*-[5-(4-Chlorphenyl)-6-[2-chlor-4-(1*H*-pyrazol-3-yl)phenyl]-2-(2,2-dimethylpropanoyl)furo[2,3-b]pyridin-3-yl]acetamid,
(19) 5-(4-Chlorphenyl)-6-[2-chlor-4-(1H-pyrazol-3-yl)phenyl]-2-(2,2-dimethylpropanoyl)furo[2,3-b]pyridin-3-carboxamid,
(20) 1-{3-Amino-5-(4-chlorphenyl)-6-[2-chlor-4-(1*H*-pyrazol-3-yl)phenyl]furo[2,3-*b*]pyridin-2-yl}-2-hydroxy-2-methylpropan-1 -on,
(21) 1-{3-Amino-5-(4-chlorphenyl)-6-[2-chlor-4-(1*H*-pyrazol-3-yl)phenyl]furo-[2,3-*b*]pyridin-2-yl}-2-methylpropan-1-on,
(22) 1-{5-(4-Chlorphenyl)-6-[2-chlor-4-(1*H*-pyrazol-3-yl)phenyl]-3-methylfuro[2,3-*b*]pyridin-2-yl}-2-hydroxy-2-methylpropan-1-on,
(23) 2-(*tert*-Butylsulfonyl)-5-(4-chlorphenyl)-6-[2-chlor-4-(1*H*-pyrazol-3-yl)phenyl]-3-methylfuro[2,3-*b*]pyridin,
(24) 1-{5-(4-Chlorphenyl)-6-[2-chlor-4-(1*H*-pyrazol-3-yl)phenyl]-3-methylfuro[2,3-*b*]pyridin-2-yl}-2-methyopropan-1-on,
(25) 1-{5-(4-Chlorphenyl)-6-[2-chlor-4-(1*H*-pyrazol-3-yl)phenyl]-3-methylfuro[2,3-*b*]piridin-2-yl}propan-1-on,
(26) *N*-[5-(4-Chlorphenyl)-6-[2-chlor-4-(3H-pyrazol-5-yl)phenyl]-2-(2,2-dimethylpropanoyl)furo[2,3-*b*]pyridin-3-yl]-2-hydroxyacetamid,
(27) 1-{5-(4-Chlorphenyl)-6-[2-chlor-4-(1H-pyrazol-3-yl)phenyl]-2-isobutyrylfuro[2,3-*b*]pyridin-3-yl}pyrrolidin-2-on,
(28) 5-(4-Chlorphenyl)-6-[2-chlor-4-(1H-pyrazol-3-yl)phenyl]-2-(2,2-dimethylpropanoyl)-N-methylfuro[2,3-b]pyridin-3-carboxamid,
(29) 1-[6-[4-(1-Acetyl-1H-pyrazol-4-yl)-2-chlorphenyl]-3-amino-5-(4-chlorphenyl)furo[2,3-*b*]pyridin-2-yl]-2-hydroxy-2-methylpropan-1-on,
(30) *N*-[5-(4-Chlorphenyl)-6-[2-chlor-4-(1*H*-pyrazol-4-yl)phenyl]-2-(2-hydroxy-2-methylpropanoyl)furo[2,3-*b*]pyridin-3-yl]-2-hydroxyacetamid,
(31) *N*-[5-(4-Chlorphenyl)-6-[2-chlor-4-(1*H*-pyrazol-4-yl)phenyl]-2-(2,2-dimethylpropanoyl)furo[2,3-b]pyridin-3-yl]-2-hydroxyacetamid,
(32) N-[6-[2-Chlor-4-(3-methyl-1H-pyrazol-4-yl)phenyl]-5-(4-chlorphenyl)-2-isobutyrylfuro[2,3-b]pyridin-3-yl]acetamid,
(33) 1-[6-[2-Chlor-4-(3-methyl-1H-pyrazol-4-yl)phenyl]-5-(4-chlorphenyl)-3-methylfuro[2,3-b]pyridin-2-yl]-2-hydroxy-2-methylpropan-1-on,
(34) 1-[6-[2-Chlor-4-(3-methyl-1H-pyrazol-4-yl)phenyl]-5-(4-chlorphenyl)-3-ethylfuro[2,3-b]pyridin-2-yl]-2-hydroxy-2-methylpropan-1-on,
(35) 1-[6-[2-Chlor-4-(3-methyl-1H-pyrazol-4-yl)phenyl]-5-(4-chlorphenyl)-3-methylfuro[2,3-b]pyridin-2-yl]-2,2-dimethylpropan-1-on,
(36) 1-[6-[2-Chlor-4-(3-methyl-1H-pyrazol-4-yl)phenyl]-5-(4-chlorphenyl)-3-(hydroxymethyl)furo[2,3-b]pyridin-2-yl]-2,2-dimethylpropan-1-on,
(37) 1-[3-Acetyl-6-[2-chlor-4-(3-methyl-1H-pyrazol-4-yl)phenyl]-5-(4-chlorphenyl)furo[2,3-b]pyridin-2-yl]-2-methylpropan-1-on,
(38) 1-[6-[2-Chlor-4-(3-methyl-1H-pyrazol-4-yl)phenyl]-5-(4-chlorphenyl)-2-isobutyrylfuro[2,3-b]pyridin-3-yl]pyrrolidin-2-on,
(39) 1-[3-Acetyl-6-[2-chlor-4-(3-methyl-1H-pyrazol-4-yl)phenyl]-5-(4-chlorphenyl)furo[2,3-b]pyridin-2-yl]-2,2-dimethylpropan-1-on,
(40) *N*-[6-[2-Chlor-4-(3-methyl-1H-pyrazol-4-yl)phenyl]-5-(4-chlorphenyl)-2-(2-hydroxy-2-methylpropanoyl)furo[2,3-b]pyridin-3-yl]acetamid,
(41) 1-[6-[2-Chlor-4-(1,2,4-oxadiazol-3-yl)phenyl]-5-(4-chlorphenyl)-3-methylfuro[2,3-*b*]pyridin-2-yl]-2,2-dimethylpropan-1-on und
(42) *N*-[6-[2-Chlor-4-(1,2,4-oxadiazol-3-yl)phenyl]-5-(4-chlorphenyl)-2-(2,2-dimethylpropanoyl)furo[2,3-*b*]pyridin-3-yl]-2-hydroxyacetamid,
oder ein pharmazeutisch annehmbares Salz davon.

12. Die Verbindung gemäß Anspruch 1, die ist: 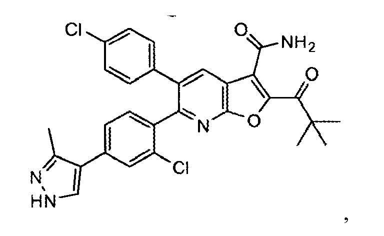 oder ein pharmazeutisch annehmbares Salz davon, oder 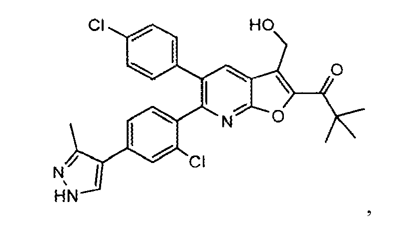 oder ein pharmazeutisch annehmbares Salz davon, oder 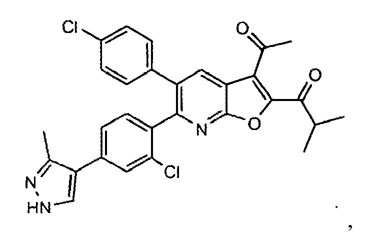 oder ein pharmazeutisch annehmbares Salz davon, oder 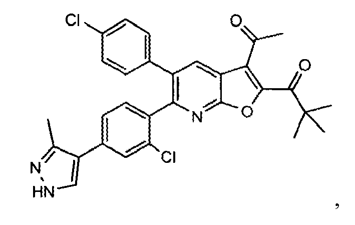 oder ein pharmazeutisch annehmbares Salz davon, oder 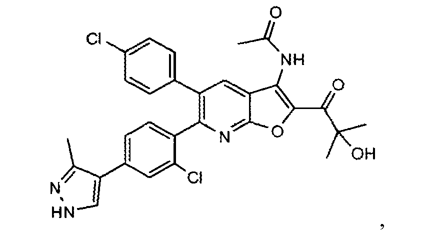 oder ein pharmazeutisch annehmbares Salz davon.

13. Die Verbindung gemäß Anspruch 1, **dadurch gekennzeichnet, dass** sie die kristalline 1:1-Ethanolsolvat-Polymorphieform I von 6-[2-Chlor-4-(3-methyl-1*H*-pyrazol-4-yl)phenyl]-5-(4-chlorphenyl)-2-(2,2-dimethylpropanoyl)furo[2,3-*b*]pyridin-3-carboxamid ist.

14. Eine Zusammensetzung, die eine Verbindung gemäß einem vorhergehenden Anspruch und gegebenenfalls eine Verbindung, ausgewählt aus Simvastatin, Ezetimib und Sitagliptin, und einen pharmazeutisch annehmbaren Träger umfasst.

15. Die Verwendung einer Verbindung gemäß einem der Ansprüche 1-13 zur Herstellung eines Medikaments, geeignet zur Behandlung eines Zustandes , ausgewählt aus: Psychose, Merkfähigkeitsstörung, kognitiven Störungen, Alzheimer-Krankheit, Migräne, Neuropathie, neuroinflammatorischen Störungen, zerebral-vaskulären Störungen, Schädel-Hirn-Trauma, Angststörungen, Stress, Epilepsie, Parkinson-Krankheit, Schizophrenie, Substanzmissbrauchsstörungen, Konstipation, chronischer Darm-Pseudoobstruktion, Leberzirrhose, Asthma, Adipositas und anderen Essstörungen, die mit übermäßiger Nahrungsaufnahme verbunden sind.

16. Die Verwendung einer Verbindung gemäß einem der Ansprüche 1-13 zur Herstellung eines Medikaments, das zur Prävention von Adipositas bei einer Person, die anfällig für Adipositas ist, geeignet ist.

## Revendications

1. Composé de la formule structurale I: 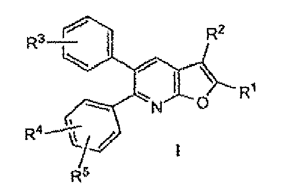 ou un sel pharmaceutiquement acceptable de celui-ci, dans lequel:
R¹ est choisi parmi:
(1) un phényle,
(2) un hétéroaryle,
(3) -C(O)R^{a},
(4) -C(O)OR^{a},
(5) -C(O)NR^{b}R^{c}, et
(6) -S(O)₂R^{a},
dans lequel le phényle et l'hétéroaryle sont chacun non substitués ou substitués avec de un à quatre substituants indépendamment choisis parmi -OH, un -alkyle C₁₋₆ et un halogène, et dans lequel R^{b} et R^{c} accompagnés des atomes auxquels ils sont attachés peuvent former un cycle mono- ou bicyclique aromatique ou non aromatique de 4-10 membres, dans lequel le cycle de 4-10 membres est non substitué ou substitué avec de un à quatre substituants indépendamment choisis parmi -OH, un -alkyle C₁₋₆ et un halogène;
R² est choisi parmi:
(1) un alkyle C₁₋₁₀,
(2) un cycloalkyle C₃₋₁₀,
(3) un cyclohétéroalkyle,
(4) un phényle,
(5) un hétéroaryle,
(6) un -C(O)alkyle C₁₋₁₀,
(7) -C(O)OR^{a},
(8) -C(O)N(R^{b})₂,
(9) -N(R^{b})₂, et
(10) un -NR^{d}C(O)alkyle C₁₋₁₀,
dans lequel l'alkyle, le cycloalkyle, le cyclohétéroalkyle, le phényle et l'hétéroaryle sont chacun non substitués ou substitués avec de un à quatre substituants indépendamment choisis parmi -OH, un -alkyle C₁₋₆ et un oxo;
R³ est choisi parmi:
(1) un hydrogène,
(2) un alkyle C₁₋₁₀,
(3) un halogène,
(4) -CN,
(5) -CF₃,
(6) -OCF₃,
(7) un -C(O)alkyle C₁₋₄,
(8) un -C(O)Oalkyle C₁₋₄,
(9) un -Oalkyle C₁₋₄, et
(10) un -Salkyle C₁₋₄;
R⁴ est choisi parmi:
(1) un pyrazole,
(2) un oxadiazole,
(3) un triazole,
(4) un isoxazole,
(5) un isothiazole, et
(6) un thiadiazole,
dans lequel le pyrazole, l'oxadiazole, le triazole, l'isoxazole, l'isothiazole et le thiadiazole sont chacun non substitués ou substitués avec de un à trois substituants choisis parmi R⁶ et R⁷;
R⁵ est choisi parmi:
(1) un hydrogène,
(2) un alkyle C₁₋₁₀,
(3) un halogène,
(4) -CN,
(5) -CF₃,
(6) -OCF₃,
(7) un -C(O)alkyle C₁₋₄,
(8) un -C(O)Oalkyle C₁₋₄,
(9) un -Oalkyle C₁₋₄, et
(10) un -Salkyle C₁₋₄;
R⁶ est choisi parmi:
(1) un hydrogène,
(2) un alkyle C₁₋₁₀,
(3) un halogène,
(4) -CN,
(5) un -C(O)Oalkyle C₁₋₆,
(6) un -Oalkyle C₁₋₆,
(7) -OCF₃, et
(8) un -Salkyle C₁₋₆;
R⁷ est choisi parmi:
(1) un hydrogène,
(2) un alkyle C₁₋₆, et
(3) un -C(O)alkyle C₁₋₁₀,
R^{a} est choisi parmi:
(1) un alkyle C₁₋₆, et
(2) un cycloalkyle C₃₋₇,
dans lequel l'alkyle et le cycloalkyle sont non substitués ou substitués avec de un à quatre substituants indépendamment choisis parmi -OH, un -alkyle C₁₋₆ et un halogène;
R^{b} est choisi parmi:
(1) un hydrogène,
(2) un alkyle C₁₋₆, et
(3) un phényle,
dans lequel l'alkyle et le phényle sont chacun non substitués ou substitués avec de un à quatre substituants indépendamment choisis parmi -OH, un -alkyle C₁₋₆ et un halogène;
R^{c} est choisi parmi:
(1) un alkyle C₁₋₆, et
(2) un phényle,
dans lequel l'alkyle et le phényle sont chacun non substitués ou substitués avec de un à quatre substituants indépendamment choisis parmi -OH, un -alkyle C₁₋₆ et un halogène; et
R^{d} est choisi parmi:
(1) un hydrogène, et
(2) un alkyle C₁₋₆,
dans lequel chaque alkyle est non substitué ou substitué avec de un à quatre substituants indépendamment choisis parmi -OH, un -alkyle C₁₋₆ et un halogène.

2. Composé selon la revendication 1, dans lequel R⁴ est choisi parmi: un pyrazole et un oxadiazole, dans lequel le pyrazole et l'oxadiazole sont chacun non substitués ou substitués avec un ou deux substituants choisis parmi R⁶ et R⁷, ou un sel pharmaceutiquement acceptable de celui-ci.

3. Composé selon la revendication 2, dans lequel R⁴ est un pyrazole, dans lequel le pyrazole est non substitué ou substitué avec un ou deux substituants choisis parmi R⁶ et R⁷, ou un sel pharmaceutiquement acceptable de celui-ci.

4. Composé selon la revendication 1, dans lequel R¹ est choisi parmi: -C(O)R^{a} et - S(O)₂R^{a}; ou un sel pharmaceutiquement acceptable de celui-ci.

5. Composé selon la revendication 1, dans lequel R¹ est choisi parmi: - C(O)C(CH₃)₂OH, -C(O)CH(CH₃)₂ et -C(O)C(CH₃)₃; ou un sel pharmaceutiquement acceptable de celui-ci.

6. Composé selon la revendication 1, dans lequel R² est choisi parmi:
(1) un alkyle C₁₋₁₀,
(2) un cyclohétéroalkyle,
(3) un -C(O)alkyle C₁₋₁₀,
(4) -C(O)N(R^{b})₂,
(5) -N(R^{b})₂, et
(6) un -NR^{d}C(O)alkyle C₁₋₁₀,
dans lequel l'alkyle et le cyclohétéroalkyle sont chacun non substitués ou substitués avec de un à quatre substituants indépendamment choisis parmi -OH et un oxo; ou un sel pharmaceutiquement acceptable de celui-ci.

7. Composé selon la revendication 1, dans lequel R² est choisi parmi: -CH₂OH, - C(O)CH₃, -C(O)NH₂ et -NHC(O)CH₃; ou un sel pharmaceutiquement acceptable de celui-ci.

8. Composé selon la revendication 1, dans lequel R³ est un halogène; ou un sel pharmaceutiquement acceptable de celui-ci.

9. Composé selon la revendication 1, dans lequel R⁵ est un halogène; ou un sel pharmaceutiquement acceptable de celui-ci.

10. Composé selon la revendication 1 de la formule structurale 1E: 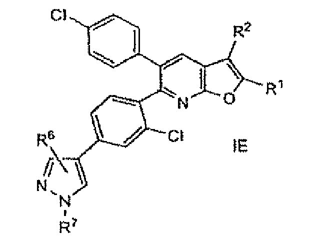 ou un sel pharmaceutiquement acceptable de celui-ci, dans lequel:
R¹ est choisi parmi: -C(O)C(CH₃)₂OH, -C(O)CH(CH₃)₂ et -C(O)C(CH₃)₃;
R² est choisi parmi: un alkyle C₁₋₁₀, un -C(O)alkyle C₁₋₁₀, -C(O)N(R^{b})₂ et un - NR^{d}C(O)alkyle C₁₋₁₀, dans lequel chaque alkyle est non substitué ou substitué avec de un à quatre substituants indépendamment choisis parmi -OH;
R⁶ est un hydrogène ou un méthyle; et
R⁷ est un hydrogène.

11. Composé selon la revendication 1, choisi parmi:
(1) le N-[5-(4-chlorophényl)-6-[2-chloro-4-(1H-pyrazol-4-yl)phényl]-2-(2-hydroxy-2-méthylpropanoyl)furo[2,3-b]pyridin-3-yl]acétamide;
(2) le N- {5-(4-chlorophényl)-6-[2-chloro-4-(1H-pyrazol-3-yl)phényl]-2-isobutyrylfuro [2,3-b]pyridin-3-yl} acétamide;
(3) le N-[5-(4-chlorophényl)-6-[2-chloro-4-(1H-pyrazol-4-yl)phényl]-2-(2,2-diméthyl-propanoyl)furo[2,3-b]pyridin-3-yl]acétamide;
(4) le N- {5-(4-chlorophényl)-6-[2-chloro-4-(1H-pyrazol-4-yl)phényl]-2-propionylfuro[2,3-b]pyridin-3-yl} acétamide;
(5) le N-[5-(4-chlorophényl)-6-[2-chloro-4-(1H-pyrazol-4-yl)phényl]-2-(2,2-diméthyl-propanoyl)furo[2,3-b]pyridin-3-yl]-2-méthylpropanamide;
(6) la 1-{3-amino-5-(4-chlorophényl)-6-[2-chloro-4-(1H-pyrazol-4-yl)phényl]furo[2,3-b]-pyridin-2-yl} -2,2-diméthylpropan-1-one;
(7) la 1-{3-amino-5-(4-chlorophényl)-6-[2-chloro-4-(1H-pyrazol-4-yl)phényl]furo[2,3-b]-pyridin-2-yl}propan-1-one;
(8) la 1-{5-(4-chlorophényl)-6-[2-chloro-4-(1H-pyrazol-4-yl)phényl]-3-méthylfuro[2,3-b]-pyridin-2-yl}-2-hydroxy-2-méthylpropan-1-one;
(9) la 1-{5-(4-chlorophényl)-6-[2-chloro-4-(1H-pyrazol-4-yl)phényl]-3-méthylfuro[2,3-b]-pyridin-2-yl}-2,2-diméthylpropan-1-one;
(10) la 2-(tert-butylsulfonyl)-5-(4-chlorophényl)-6-[2-chloro-4-(1H-pyrazol-4-yl)phényl]-3-méthylfuro[2,3-b]-pyridine;
(11) la 1-{5-(4-chlorophényl)-6-[2-chloro-4-(1H-pyrazol-4-yl)phényl]-3-méthylfuro[2,3-b]-pyridin-2-yl}-3-méthylbutan-1-one;
(12) le N- {5-(4-chlorophényl)-6-[2-chloro-4-(1H-pyrazol-4-yl)phényl]-2-propionylfuro[2,3-b]pyridin-3-yl}-2-hydroxy-2-méthylpropanamide;
(13) le (2S)-N-[5-(4-chlorophényl)-6-[2-chloro-4-(1H-pyrazol-4-yl)phényl]-2-(2,2-diméthyl-propanoyl)furo[2,3-b]pyridin-3-yl]-2-hydroxypropanamide;
(14) le (2S)-N- {5-(4-chlorophényl)-6-[2-chloro-4-(1H-pyrazol-4-yl)phényl]-2-propionylfuro-[2,3-b]pyridin-3-yl}-2-hydroxypropanamide;
(15) le N-[5-(4-chlorophényl)-6-[2-chloro-4-(1H-pyrazol-4-yl)phényl]-2-(2,2-diméthyl-propanoyl)furo[2,3-b]pyridin-3-yl]-2-hydroxy-2-méthylpropanamide;
(16) le N-{5-(4-chlorophényl)-6-[2-chloro-4-(1H-pyrazol-4-yl)phényl]-2-propionylfuro[2,3-b]pyridin-3-yl}-2-hydroxyacétamide;
(17) le N-[5-(4-chlorophényl)-6-[2-chloro-4-(1H-pyrazol-3-yl)phényl]-2-(2-hydroxy-2-méthylpropanoyl)furo[2,3-b]pyridin-3-yl]acétamide;
(18) le N-[5-(4-chlorophényl)-6-[2-chloro-4-(1H-pyrazol-3-yl)phényl]-2-(2,2-diméthyl-propanoyl)furo[2,3-b]pyridin-3-yl]acétamide;
(19) le 5-(4-chlorophényl)-6-[2-chloro-4-(1H-pyrazol-3-yl)phényl]-2-(2,2-diméthyl-propanoyl)furo[2,3-b]pyridin-3-carboxamide;
(20) la 1-{3-amino-5-(4-chlorophényl)-6-[2-chloro-4-(1H-pyrazol-3-yl)phényl]furo[2,3-b]pyridin-2-yl}-2-hydroxy-2-méthylpropan-1-one;
(21) la 1-{3-amino-5-(4-chlorophényl)-6-[2-chloro-4-(1H-pyrazol-3-yl)phényl]furo[2,3-b]pyridin-2-yl}-2-méthylpropan-1-one;
(22) la 1-{5-(4-chlorophényl)-6-[2-chloro-4-(1H-pyrazol-3-yl)phényl]-3-méthylfuro[2,3-b]pyridin-2-yl}-2-hydroxy-2-méthylpropan-1-one;
(23) la 2-(tert-butylsulfonyl)-5-(4-chlorophényl)-6-[2-chloro-4-(1H-pyrazol-3-yl)phényl]-3-méthylfuro[2,3-b]pyridine;
(24) la 1-{5-(4-chlorophényl)-6-[2-chloro-4-(1H-pyrazol-3-yl)phényl]-3-méthylfuro[2,3-b]pyridin-2-yl}-2-méthylpropan-1-one;
(25) la 1-{5-(4-chlorophényl)-6-[2-chloro-4-(1H-pyrazol-3-yl)phényl]-3-méthylfuro[2,3-b]-pyridin-2-yl}propan-1-one;
(26) le N-[5-(4-chlorophényl)-6-[2-chloro-4-(3H-pyrazol-5-yl)phényl]-2-(2,2-diméthyl-propanoyl)furo[2,3-b]pyridin-3-yl]-2-hydroxyacétamide;
(27) la 1-{5-(4-chlorophényl)-6-[2-chloro-4-(1H-pyrazo-3-yl)phényl]-2-isobutyrylfuro[2,3-b]pyridin-3-yl}pyrrolidin-2-one;
(28) le 5-(4-chlorophényl)-6-[2-chloro-4-(1H-pyrazol-3-yl)phényl]-2-(2,2-diméthyl-propanoyl)-N-méthylfuro[2,3-b]pyridine-3-carboxamide;
(29) la 1-[6-[4-(1-acétyl-1H-pyrazol-4-yl)-2-chlorophényl]-3-amino-5-(4-chlorophényl)furo[2,3-b]pyridin-2-yl]-2-hydroxy-2-méthylpropan-1-one;
(30) le N-[5-(4-chlorophényl)-6-[2-chloro-4-(1H-pyrazol-4-yl)phényl]-2-(2-hydroxy-2-méthylpropanoyl)furo[2,3-b]pyridin-3-yl]-2-hydroxyacétamide;
(31) le N-[5-(4-chlorophényl)-6-[2-chloro-4-(1H-pyrazol-4-yl)phényl]-2-(2,2-diméthyl-propanoyl)furo[2,3-b]pyridin-3-yl]-2-hydroxyacétamide;
(32) le N-[6-[2-chloro-4-(3-méthyl-1H-pyrazol-4-yl)phényl]-5-(4-chlorophényl)-2-isobutyryl-furo[2,3-b]pyridin-3-yl]acétamide;
(33) la 1-[6-[2-chloro-4-(3-méthyl-1H-pyrazol-4-yl)phényl]-5-(4-chlorophényl)-3-méthylfuro[2,3-b]pyridin-2-yl]-2-hydroxy-2-méthylpropan-1-one;
(34) la 1-[6-[2-chloro-4-(3-méthyl-1H-pyrazol-4-yl)phényl]-5-(4-chlorophényl)-3-éthylfuro[2,3-b]pyridin-2-yl]-2-hydroxy-2-méthylpropan-1-one;
(35) la 1-[6-[2-chloro-4-(3-méthyl-1H-pyrazol-4-yl)phényl]-5-(4-chlorophényl)-3-méthylfuro[2,3-b]pyridin-2-yl]-2,2-diméthylpropan-1-one;
(36) la 1-[6-[2-chloro-4-(3-méthyl-1H-pyrazol-4-yl)phényl]-5-(4-chlorophényl)-3-(hydroxy-méthyl)furo[2,3-b]pyridin-2-yl]-2,2-diméthylpropan-1-one;
(37) la 1-[3-acétyl-6-[2-chloro-4-(3-méthyl-1H-pyrazol-4-yl)phényl]-5-(4-chlorophényl)furo[2,3-b]pyridin-2-yl]-2-méthylpropan-1-one,
(38) la 1-[6-[2-chloro-4-(3-méthyl-1H-pyrazol-4-yl)phényl]-5-(4-chlorophényl)-2-isobutyrylfuro[2,3-b]pyridin-3-yl]pyrrolidin-2-one;
(39) la 1-[3-acétyl-6-[2-chloro-4-(3-méthyl-1H-pyrazol-4-yl)phényl]-5-(4-chlorophényl)furo[2,3-b]pyridin-2-yl]-2,2-diméthylpropan-1-one;
(40) le N-[6-[2-chloro-4-(3-méthyl-1H-pyrazol-4-yl)phényl]-5-(4-chlorophényl)-2-(2-hydroxy-2-méthylpropanoyl)furo[2,3-b]pyridin-3-yl]acétamide;
(41) la 1-[6-[2-chloro-4-(1,2,4-oxadiazol-3-yl)phényl]-5-(4-chlorophényl)-3-méthylfuro[2,3-b]pyridin-2-yl]-2,2-diméthylpropan-1-one; et
(42) le N-[6-[2-chloro-4-(1,2,4-oxadiazol-3-yl)phényl]-5-(4-chlorophényl)-2-(2,2-diméthyl-propanoyl)furo[2,3-b]pyridin-3-yl]-2-hydroxyacétamide;
ou un sel pharmaceutiquement acceptable de ceux-ci.

12. Composé selon la revendication 1, qui est: 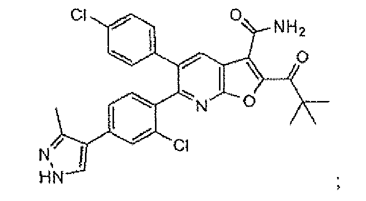 ou un sel pharmaceutiquement acceptable de celui-ci, ou 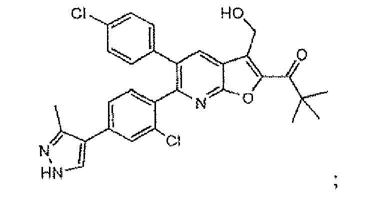 ou un sel pharmaceutiquement acceptable de celui-ci, ou 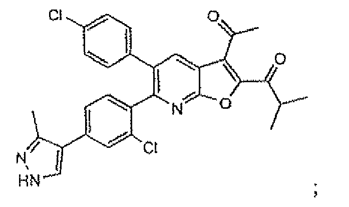 ou un sel pharmaceutiquement acceptable de celui-ci, ou 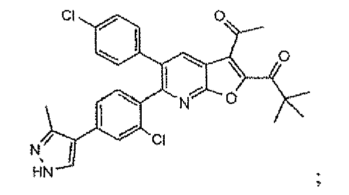 ou un sel pharmaceutiquement acceptable de celui-ci, ou 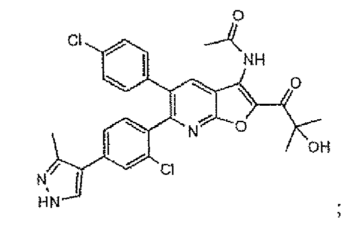 ou un sel pharmaceutiquement acceptable de celui-ci.

13. Composé selon la revendication 1, **caractérisé** comme étant la forme polymorphique cristalline I de solvate d'éthanol 1:1 du 6-[2-chloro-4-(3-méthyl-1H-pyrazol-4-yl)phényl]-5-(4-chlorophényl)-2-(2,2-diméthylpropanoyl)furo[2,3-b]pyridine-3-carboxamide.

14. Composition comprenant un composé selon l'une quelconque des revendications précédentes et optionnellement un composé choisi parmi la simvastatine, l'ézétimibe et la sitagliptine; et un support pharmaceutiquement acceptable.

15. Utilisation d'un composé selon l'une quelconque des revendications 1-13 pour la préparation d'un médicament utile pour le traitement d'un état choisi parmi: une psychose, une déficience de la mémoire, des troubles cognitifs, une maladie d'Alzheimer, une migraine, une neuropathie, des troubles neuro-inflammatoires, des accidents vasculaires cérébraux, un traumatisme crânien, des troubles de l'anxiété, un stress, une épilepsie, une maladie de Parkinson, une schizophrénie, des troubles d'abus de substances psychoactives, une constipation, une pseudo-obstruction intestinale chronique, une cirrhose du foie, un asthme, une obésité et d'autres troubles de l'alimentation associés à un apport alimentaire excessif.

16. Utilisation d'un composé selon l'une quelconque des revendications 1-13 pour la fabrication d'un médicament utile pour la prévention de l'obésité chez une personne menacée d'obésité.
